# EUROPEAN PATENT APPLICATION

(11) **EP 1 500 663 A1**
(43) Date of publication of application: **26.01.2005**
(21) Application number: 04023089.8
(22) Date of filing: 14.09.2001
(51) Int. Cl.: C07K 14/47, C12N 15/12, C12N 5/10, C12N 15/62, C07K 16/18, A61K 38/17, A61K 39/395, A61K 48/00

(54) **Secreted proteins and their uses**

(30) Priority: 28.09.2000 US 236088 P
(62) Divisional of application: 01970558.1
(71) Applicant: ELI LILLY AND COMPANY, Indianapolis, IN 46285 (US)
(72) Inventor: Su, Eric Wen, Carmel, Indiana 46032 (US); Wang, He, Carmel, Indiana 46033 (US)
(74) Representative: Ingham, Stephen H.

(57) **Abstract**

The present invention provides nucleic acid sequences encoding novel human proteins. These novel nucleic acids are useful for constructing the claimed DNA vectors and host cells of the invention and for preparing the claimed recombinant proteins and antibodies that are useful in the claimed methods and medical uses.

SEQUENCE LISTING

<110> Eli Lilly and Company

<120> NOVEL SECRETED PROTEINS AND METHODS OF USING SAME

<130> X13020

<160> 18

<170> PatentIn version 3.0

<210> 1
<211> 3483
<212> DNA
<213> Homo sapiens

<220>
<221> CDS
<222> (117)..(1607)

<220>
<221> sig_peptide
<222> (117)..(182)

<400> 1
cgactcacta tagggcgaat tgggccctct agatgcatgc tcgagcggcc gccagtgtga 60

tggatatctg cagaattcgc ccttgtgagt cccatagttg ccgcaggagt ggagcc atg 119
Met
1

agc tgc gtc ctg ggt ggt gtc atc ccc ttg ggg ctg ctg ttc ctg gtc 167
Ser Cys Val Leu Gly Gly Val Ile Pro Leu Gly Leu Leu Phe Leu Val
            5                   10                  15

tgc gga tcc caa ggc tac ctc ctg ccc aac gtc act ctc tta gag gag 215
Cys Gly Ser Gln Gly Tyr Leu Leu Pro Asn Val Thr Leu Leu Glu Glu

20                  25                  30

ctg ctc agc aaa tac cag cac aac gag tct cac tcc cgg gtc cgc aga        263
Leu Leu Ser Lys Tyr Gln His Asn Glu Ser His Ser Arg Val Arg Arg
    35                  40                  45

gcc atc ccc agg gag gac aag gag gag atc ctc atg ctg cac aac aag        311
Ala Ile Pro Arg Glu Asp Lys Glu Glu Ile Leu Met Leu His Asn Lys
50                  55                  60                  65

ctt cgg ggc cag gtg cag cct cag gcc tcc aac atg gag tac atg acc        359
Leu Arg Gly Gln Val Gln Pro Gln Ala Ser Asn Met Glu Tyr Met Thr
                70                  75                  80

tgg gat gac gaa ctg gag aag tct gct gca gcg tgg gcc agt cag tgc        407
Trp Asp Asp Glu Leu Glu Lys Ser Ala Ala Ala Trp Ala Ser Gln Cys
            85                  90                  95

atc tgg gag cac ggg ccc acc agt ctg ctg gtg tcc atc ggg cag aac        455
Ile Trp Glu His Gly Pro Thr Ser Leu Leu Val Ser Ile Gly Gln Asn
        100                 105                 110

ctg ggc gct cac tgg ggc agg tat cgc tct ccg ggg ttc cat gtg cag        503
Leu Gly Ala His Trp Gly Arg Tyr Arg Ser Pro Gly Phe His Val Gln
    115                 120                 125

tcc tgg tat gac gag gtg aag gac tac acc tac ccc tac ccg agc gag        551
Ser Trp Tyr Asp Glu Val Lys Asp Tyr Thr Tyr Pro Tyr Pro Ser Glu
130                 135                 140                 145

tgc aac ccc tgg tgt cca gag agg tgc tcg ggg ccc atg tgc acg cac        599
Cys Asn Pro Trp Cys Pro Glu Arg Cys Ser Gly Pro Met Cys Thr His
                150                 155                 160

tac aca cag ata gtt tgg gcc acc acc aac aag atc ggt tgt gct gtg        647
Tyr Thr Gln Ile Val Trp Ala Thr Thr Asn Lys Ile Gly Cys Ala Val
            165                 170                 175

aac acc tgc cgg aag atg act gtc tgg gga gaa gtt tgg gag aac gcg        695
Asn Thr Cys Arg Lys Met Thr Val Trp Gly Glu Val Trp Glu Asn Ala
        180                 185                 190

gtc tac ttt gtc tgc aat tat tct cca aag ggg aac tgg att gga gaa        743
Val Tyr Phe Val Cys Asn Tyr Ser Pro Lys Gly Asn Trp Ile Gly Glu
    195                 200                 205

gcc ccc tac aag aat ggc cgg ccc tgc tct gag tgc cca ccc agc tat        791
Ala Pro Tyr Lys Asn Gly Arg Pro Cys Ser Glu Cys Pro Pro Ser Tyr
210                 215                 220                 225

gga ggc agc tgc agg aac aac ttg tgt tac cga gaa gaa acc tac act        839
Gly Gly Ser Cys Arg Asn Asn Leu Cys Tyr Arg Glu Glu Thr Tyr Thr
                230                 235                 240

cca aaa cct gaa acg gac gag atg aat gag gtg gaa acg gct ccc att        887
Pro Lys Pro Glu Thr Asp Glu Met Asn Glu Val Glu Thr Ala Pro Ile
            245                 250                 255

cct gaa gaa aac cat gtt tgg ctc caa ccg agg gtg atg aga ccc acc        935
Pro Glu Glu Asn His Val Trp Leu Gln Pro Arg Val Met Arg Pro Thr
        260                 265                 270

aag ccc aag aaa acc tct gcg gtc aac tac atg acc caa gtc gtc aga        983

Lys Pro Lys Lys Thr Ser Ala Val Asn Tyr Met Thr Gln Val Val Arg
    275                 280                 285

tgt gac acc aag atg aag gac agg tgc aaa ggg tcc acg tgt aac agg 1031
Cys Asp Thr Lys Met Lys Asp Arg Cys Lys Gly Ser Thr Cys Asn Arg
290                 295                 300                 305

tac cag tgc cca gca ggc tgc ctg aac cac aag gcg aag atc ttt gga 1079
Tyr Gln Cys Pro Ala Gly Cys Leu Asn His Lys Ala Lys Ile Phe Gly
                310                 315                 320

act ctg ttc tat gaa agc tcg tct agc ata tgc cgc gcc gcc atc cac 1127
Thr Leu Phe Tyr Glu Ser Ser Ser Ser Ile Cys Arg Ala Ala Ile His
            325                 330                 335

tac ggg atc ctg gat gac aag gga ggc ctg gtg gat atc acc agg aac 1175
Tyr Gly Ile Leu Asp Asp Lys Gly Gly Leu Val Asp Ile Thr Arg Asn
        340                 345                 350

ggg aag gtc ccc ttc ttc gtg aag tct gag aga cac ggc gtg cag tcc 1223
Gly Lys Val Pro Phe Phe Val Lys Ser Glu Arg His Gly Val Gln Ser
    355                 360                 365

ctc agc aaa tac aaa cct tcc agc tca ttc atg gtg tca aaa gtg aaa 1271
Leu Ser Lys Tyr Lys Pro Ser Ser Ser Phe Met Val Ser Lys Val Lys
370                 375                 380                 385

gtg cag gat ttg gac tgc tac acg acc gtt gct cag ctg tgc ccg ttt 1319
Val Gln Asp Leu Asp Cys Tyr Thr Thr Val Ala Gln Leu Cys Pro Phe
                390                 395                 400

gaa aag cca gca act cac tgc cca aga atc cat tgt ccg gca cac tgc 1367
Glu Lys Pro Ala Thr His Cys Pro Arg Ile His Cys Pro Ala His Cys
            405                 410                 415

aaa gac gaa cct tcc tac tgg gct ccg gtg ttt gga acc aac atc tat 1415
Lys Asp Glu Pro Ser Tyr Trp Ala Pro Val Phe Gly Thr Asn Ile Tyr
        420                 425                 430

gca gat acc tca agc atc tgc aag aca gcc gtg cac gcg gga gtc atc 1463
Ala Asp Thr Ser Ser Ile Cys Lys Thr Ala Val His Ala Gly Val Ile
    435                 440                 445

agc aac gag agt ggg ggt gac gtg gac gtg atg ccc gtg gat aaa aag 1511
Ser Asn Glu Ser Gly Gly Asp Val Asp Val Met Pro Val Asp Lys Lys
450                 455                 460                 465

aag acc tac gtg ggc tcg ctc agg aat gga gtt cag tct gaa agc ctg 1559
Lys Thr Tyr Val Gly Ser Leu Arg Asn Gly Val Gln Ser Glu Ser Leu
                470                 475                 480

ggg act cct cgg gat gga aag gcc ttc cgg atc ttt gct gtc agg cag 1607
Gly Thr Pro Arg Asp Gly Lys Ala Phe Arg Ile Phe Ala Val Arg Gln
            485                 490                 495

tgaatttcca gcaccagggg agaaggggcg tcttcaggag ggcttcgggg ttttgctttt 1667

attttatttt tgtcattgcg gggtatatgg agagtcagga aacttccttt gactgatgtt 1727

cagtgtccat cactttgtgg cctgtgggtg aggtgacatc tcatcccctc actgaagcaa 1787

cagcatccca aggtgctcag ccggactccc tggtgcctga tcctgctggg gcccgggggt 1847

ctccatctgg acgtcctctc tcctttagag atctgagctg tctcttaaag gggacagttg 1907
cccaaaatgt tccttgctat gtgttcttct gttggtggag gaagttgatt tcaacctccc 1967
tgccaaaaga acaaaccatt tgaagctcac aattgtgaag cattcacggc gtcggaagag 2027
gccttttgag caagcgccaa tgagtttcag gaatgaagta gaaggtagtt atttaaaaat 2087
aaaaaacaca gtccgtccct accaatagag gaaaatggtt ttaatgtttg ctggtcagac 2147
agacaaatgg gctagagtaa gagggctgcg ggtatgagag accccggctc cgccctggca 2207
cgtgtccttg ctggcggccc gccacaggcc cccttcaatg gccgcattca ggatggctct 2267
atacacagca gtgctggttt atgtagagtt cagcagtcac ttcagagatg tatcttgtct 2327
ttgtcaggcc cttcgtcttc atggcccacc tgttttctgc cgtgaccttt ggtcccattg 2387
aggactaagg atcgggaccc tttctttacc ccctacccgt tgtggctccc accctgcctc 2447
ggactggttt acgtgtcctg gttcacaccc aggacttttc tttgcaagcg aacctgtttg 2507
aagcccaagt cttaactcct ggtctcgtaa ggttccactg agacgagatg tctgagaaca 2567
accaaagaag gcctgctctt tgctgctttt aaaaaatgac aattaaatgt gcagattccc 2627
cacgcacccg atgacctatt ttttcagccg tgggaggaat ggagtctttg gtacattcct 2687
caccgaggtt agcagctcag tttgtggtta tgaaaccgtc tgtggcctca tgacagcgag 2747
agatgggaat acactagaag gatctctttt cctgttttcg tgaaacgact cttgccaaac 2807
gttcccgagg cgccaaggag tgtagtacac cctggctgcc atcactctat aaaagtgctt 2867
catgagccca gaccaaaagc ccacagtgaa atgaagtacc cttttgtaaa tagcattttt 2927
ttgcagaagg tgaaaattcc actctctacc accgggccag ccaatagatc actttggtga 2987
atgctagttt caaatttgat tcaaaatatt tcttaggtga aagaactagc agaaagtcaa 3047
aaactaagat actgtagact ggacaagaaa ttctacctgg gcacctaggt gatgccttct 3107
ttctttgatt gcctttctaa taaatgcaga atctgaaggt aaataggttt aaaacaaaac 3167
aaaaacccac ccctttaagg agttggtaaa aagcagttca actcttagct tgactgagct 3227
aaaattcaca ggactacgtg ctttgtgcat tgtagtctag tcgtaattca taggtactga 3287
ctcctcagcc ccaaatgtcg gagaggaaga attcggtcag cctgtcaggt cgtgagtcca 3347
gttaccacca aacatctggg aaacttctgg gtgctgaagg gcgaattcca gcacactggc 3407
ggccgttact agtggatccg agctcggtac caagcttgat gcatagcttg agtattctat 3467
agtgtcacct aaatag 3483

<210> 2
<211> 497
<212> PRT

<213>  Homo sapiens

<400>  2

Met Ser Cys Val Leu Gly Gly Val Ile Pro Leu Gly Leu Leu Phe Leu
1               5                   10                  15

Val Cys Gly Ser Gln Gly Tyr Leu Leu Pro Asn Val Thr Leu Leu Glu
            20                  25                  30

Glu Leu Leu Ser Lys Tyr Gln His Asn Glu Ser His Ser Arg Val Arg
        35                  40                  45

Arg Ala Ile Pro Arg Glu Asp Lys Glu Glu Ile Leu Met Leu His Asn
    50                  55                  60

Lys Leu Arg Gly Gln Val Gln Pro Gln Ala Ser Asn Met Glu Tyr Met
65                  70                  75                  80

Thr Trp Asp Asp Glu Leu Glu Lys Ser Ala Ala Ala Trp Ala Ser Gln
                85                  90                  95

Cys Ile Trp Glu His Gly Pro Thr Ser Leu Leu Val Ser Ile Gly Gln
            100                 105                 110

Asn Leu Gly Ala His Trp Gly Arg Tyr Arg Ser Pro Gly Phe His Val
        115                 120                 125

Gln Ser Trp Tyr Asp Glu Val Lys Asp Tyr Thr Tyr Pro Tyr Pro Ser
    130                 135                 140

Glu Cys Asn Pro Trp Cys Pro Glu Arg Cys Ser Gly Pro Met Cys Thr
145                 150                 155                 160

His Tyr Thr Gln Ile Val Trp Ala Thr Thr Asn Lys Ile Gly Cys Ala
                165                 170                 175

Val Asn Thr Cys Arg Lys Met Thr Val Trp Gly Glu Val Trp Glu Asn
            180                 185                 190

Ala Val Tyr Phe Val Cys Asn Tyr Ser Pro Lys Gly Asn Trp Ile Gly
        195                 200                 205

Glu Ala Pro Tyr Lys Asn Gly Arg Pro Cys Ser Glu Cys Pro Pro Ser
    210                 215                 220

Tyr Gly Gly Ser Cys Arg Asn Asn Leu Cys Tyr Arg Glu Glu Thr Tyr

225                 230                 235                 240

Thr Pro Lys Pro Glu Thr Asp Glu Met Asn Glu Val Glu Thr Ala Pro
                245                 250                 255

Ile Pro Glu Glu Asn His Val Trp Leu Gln Pro Arg Val Met Arg Pro
            260                 265                 270

Thr Lys Pro Lys Lys Thr Ser Ala Val Asn Tyr Met Thr Gln Val Val
        275                 280                 285

Arg Cys Asp Thr Lys Met Lys Asp Arg Cys Lys Gly Ser Thr Cys Asn
    290                 295                 300

Arg Tyr Gln Cys Pro Ala Gly Cys Leu Asn His Lys Ala Lys Ile Phe
305                 310                 315                 320

Gly Thr Leu Phe Tyr Glu Ser Ser Ser Ser Ile Cys Arg Ala Ala Ile
                325                 330                 335

His Tyr Gly Ile Leu Asp Asp Lys Gly Gly Leu Val Asp Ile Thr Arg
            340                 345                 350

Asn Gly Lys Val Pro Phe Phe Val Lys Ser Glu Arg His Gly Val Gln
        355                 360                 365

Ser Leu Ser Lys Tyr Lys Pro Ser Ser Ser Phe Met Val Ser Lys Val
    370                 375                 380

Lys Val Gln Asp Leu Asp Cys Tyr Thr Thr Val Ala Gln Leu Cys Pro
385                 390                 395                 400

Phe Glu Lys Pro Ala Thr His Cys Pro Arg Ile His Cys Pro Ala His
                405                 410                 415

Cys Lys Asp Glu Pro Ser Tyr Trp Ala Pro Val Phe Gly Thr Asn Ile
            420                 425                 430

Tyr Ala Asp Thr Ser Ser Ile Cys Lys Thr Ala Val His Ala Gly Val
        435                 440                 445

Ile Ser Asn Glu Ser Gly Gly Asp Val Asp Val Met Pro Val Asp Lys
    450                 455                 460

Lys Lys Thr Tyr Val Gly Ser Leu Arg Asn Gly Val Gln Ser Glu Ser
465                 470                 475                 480

Leu Gly Thr Pro Arg Asp Gly Lys Ala Phe Arg Ile Phe Ala Val Arg
                485                 490                 495

Gln

<210> 3

<211> 1370

<212> DNA

<213> Homo sapiens

<220>

<221> CDS

<222> (95)..(406)

<220>

<221> sig_peptide

<222> (95)..(160)

<400> 3
cacgcgtccg cccacgcgtc cggaacaggg tggagtcaca ggggaaagaa aatgaggccc 60

caggacacct gggttcacac ccaggtcccc agcg atg tct cca cca ccg ctg ctg 115
Met Ser Pro Pro Pro Leu Leu
1               5

caa ccc ctg ctg ctg ctg ctg cct ctg ctg aat gtg gag cct tcc ggg 163
Gln Pro Leu Leu Leu Leu Leu Pro Leu Leu Asn Val Glu Pro Ser Gly
        10                  15                  20

gcc aca ctg atc cgc atc cct ctt cat cga gtc caa cct gga cgc agg 211
Ala Thr Leu Ile Arg Ile Pro Leu His Arg Val Gln Pro Gly Arg Arg
    25                  30                  35

atc ctg aac cta ctg agg gga tgg aga gaa cca gca gag ctc ccc aag 259
Ile Leu Asn Leu Leu Arg Gly Trp Arg Glu Pro Ala Glu Leu Pro Lys
40                  45                  50                  55

ttg ggg gcc cca tcc cct ggg gac aag ccc atc ttc gta cct ctc tcg 307
Leu Gly Ala Pro Ser Pro Gly Asp Lys Pro Ile Phe Val Pro Leu Ser
                60                  65                  70

aac tac agg gat ggt tac acc acc gat ttg atc cca aag cct cta gct 355
Asn Tyr Arg Asp Gly Tyr Thr Thr Asp Leu Ile Pro Lys Pro Leu Ala
            75                  80                  85

cct tcc agg cca atg gga cca agt ttg cca ttc aat atg gaa ctg ggc 403
Pro Ser Arg Pro Met Gly Pro Ser Leu Pro Phe Asn Met Glu Leu Gly

90                95                100

ggg tagatggaat cctgagcgag gacaagctga ctattggtgg aatcaagggt          456
Gly

gcatcagtga ttttcgggga ggctctctgg gagcccagcc tggtcttcgc ttttgcccat    516

tttgatggga tattgggcct cggttttccc attctgtctg tggaaggagt tcggcccccg    576

atggatgtac tggtggagca ggggctattg gataagcctg tcttctcctt ttacctcaac    636

agggaccctg aagagcctga tggaggagag ctggtcctgg ggggctcgga cccggcacac    696

tacatcccac ccctcacctt cgtgccagtc acggtccctg cctactggca gatccacatg    756

gagcgtgtga aggtgggccc agggctgact ctctgtgcca agggctgtgc tgccatcctg    816

gatacgggca cgtccctcat cacaggaccc actgaggaga tccgggccct gcatgcagcc    876

attgggggaa tccccttgct ggctggggag tacatcatcc tgtgctcgga aatcccaaag    936

ctccccgcag tctccttcct tcttgggggg gtctggttta acctcacggc ccatgattac    996

gtcatccaga ctactcgaaa tggcgtccgc ctctgcttgt ccggtttcca ggccctggat   1056

gtccctccgc ctgcagggcc cttctggatc ctcggtgacg tcttcttggg gacgtatgtg   1116

gccgtcttcg accgcgggga catgaagagc agcgcccggg tgggcctggc gcgcgctcgc   1176

actcgcggag cggacctcgg atggggagag actgcgcagg cgcagttccc cgggtgacgc   1236

ccaagtgaag cgcatgcgca gcgggtggtc gcggaggtcc tgctacccag taaaaatcca   1296

ctatttccat tgaaaaaaaa aaaaaaaaaa aaaaaaaaaa aaaaaaaaaa aaaaaaaaaa   1356

aaaaaaaaaa aaaa                                                     1370

<210>  4

<211>  104

<212>  PRT

<213>  Homo sapiens

<400>  4

Met Ser Pro Pro Pro Leu Leu Gln Pro Leu Leu Leu Leu Leu Pro Leu
1               5                   10                  15

Leu Asn Val Glu Pro Ser Gly Ala Thr Leu Ile Arg Ile Pro Leu His
            20                  25                  30

Arg Val Gln Pro Gly Arg Arg Ile Leu Asn Leu Leu Arg Gly Trp Arg
        35                  40                  45

Glu Pro Ala Glu Leu Pro Lys Leu Gly Ala Pro Ser Pro Gly Asp Lys
    50                  55                  60

Pro Ile Phe Val Pro Leu Ser Asn Tyr Arg Asp Gly Tyr Thr Thr Asp
65                  70                  75                  80

Leu Ile Pro Lys Pro Leu Ala Pro Ser Arg Pro Met Gly Pro Ser Leu
                85                  90                  95

Pro Phe Asn Met Glu Leu Gly Gly
            100

<210>  5

<211>  6317

<212>  DNA

<213>  Homo sapiens

<220>

<221>  CDS

<222>  (284)..(5155)

<220>

<221>  sig_peptide

<222>  (284)..(328)

<400>  5
tctcttctcg aggcgggcga cacgaggagc ccagggcgca gcgggctcga atcacatagt      60

gcccggcccc ggatttcgtg cgcagagaga accccaggcc ccacaggccc tcccacgctc     120

tgcccagatc cctgggcaag agtccaccgt cagcatcgtc gcttggtctt gctctgtcac     180

ccaggccgga gtgcagtgct gtaatcatgg ctcattacct ctcacgctca agtaatcctc     240

ccacttcagt ctcctgaggc tgagaaaaca ggcacaagcc acc atg cct gga gtg      295
                                                Met Pro Gly Val
                                                1

ctc tcc ctg gtt att cca gaa aca cca gtc gct gag gat ctc tca cct      343
Leu Ser Leu Val Ile Pro Glu Thr Pro Val Ala Glu Asp Leu Ser Pro
5                   10                  15                  20

gca gtt ccc tgc tgg atc ttc att ctg act ggt cca tgg ggc cga tgt      391
Ala Val Pro Cys Trp Ile Phe Ile Leu Thr Gly Pro Trp Gly Arg Cys
                25                  30                  35

atg gga gat gaa tgt ggt ccc gga ggc atc caa acg agg gct gtg tgg 439
Met Gly Asp Glu Cys Gly Pro Gly Gly Ile Gln Thr Arg Ala Val Trp
            40                  45                  50

tgt gct cat gtg gag gga tgg act aca ctg cat act aac tgt aag cag 487
Cys Ala His Val Glu Gly Trp Thr Thr Leu His Thr Asn Cys Lys Gln
        55                  60                  65

gcc gag aga ccc aat aac cag cag aat tgt ttc aaa gtt tgc gat tgg 535
Ala Glu Arg Pro Asn Asn Gln Gln Asn Cys Phe Lys Val Cys Asp Trp
    70                  75                  80

cac aaa gag ttg tac gac tgg aga ctg gga cct tgg aat cag tgt cag 583
His Lys Glu Leu Tyr Asp Trp Arg Leu Gly Pro Trp Asn Gln Cys Gln
85                  90                  95                  100

ccc gtg att tca aaa agc cta gag aaa cct ctt gag tgc att aag ggg 631
Pro Val Ile Ser Lys Ser Leu Glu Lys Pro Leu Glu Cys Ile Lys Gly
                105                 110                 115

gaa gaa ggt att cag gtg agg gag ata gcg tgc atc cag aaa gac aaa 679
Glu Glu Gly Ile Gln Val Arg Glu Ile Ala Cys Ile Gln Lys Asp Lys
            120                 125                 130

gac att cct gcg gag gat atc atc tgt gag tac ttt gag ccc aag cct 727
Asp Ile Pro Ala Glu Asp Ile Ile Cys Glu Tyr Phe Glu Pro Lys Pro
        135                 140                 145

ctc ctg gag cag gct tgc ctc att cct tgc cag caa gat tgc atc gtg 775
Leu Leu Glu Gln Ala Cys Leu Ile Pro Cys Gln Gln Asp Cys Ile Val
    150                 155                 160

tct gaa ttt tct gcc tgg tcc gaa tgc tcc aag acc tgc ggc agc ggg 823
Ser Glu Phe Ser Ala Trp Ser Glu Cys Ser Lys Thr Cys Gly Ser Gly
165                 170                 175                 180

ctc cag cac cgg acg cgt cat gtg gtg gcg ccc ccg cag ttc gga ggc 871
Leu Gln His Arg Thr Arg His Val Val Ala Pro Pro Gln Phe Gly Gly
                185                 190                 195

tct ggc tgt cca aac ctg acg gag ttc cag gtg tgc caa tcc agt cca 919
Ser Gly Cys Pro Asn Leu Thr Glu Phe Gln Val Cys Gln Ser Ser Pro
            200                 205                 210

tgc gag gcc gag gag ctc agg tac agc ctg cat gtg ggg ccc tgg agc 967
Cys Glu Ala Glu Glu Leu Arg Tyr Ser Leu His Val Gly Pro Trp Ser
        215                 220                 225

acc tgc tca atg ccc cac tcc cga caa gta aga caa gca agg aga cgc 1015
Thr Cys Ser Met Pro His Ser Arg Gln Val Arg Gln Ala Arg Arg Arg
    230                 235                 240

ggg aag aat aaa gaa cgg gaa aag gac cgc agc aaa gga gta aag gat 1063
Gly Lys Asn Lys Glu Arg Glu Lys Asp Arg Ser Lys Gly Val Lys Asp
245                 250                 255                 260

cca gaa gcc cgc gag ctt att aag aaa aag aga aac aga aac agg cag 1111
Pro Glu Ala Arg Glu Leu Ile Lys Lys Lys Arg Asn Arg Asn Arg Gln
                265                 270                 275

aac aga caa gag aac aaa tat tgg gac atc cag att gga tat cag acc 1159
Asn Arg Gln Glu Asn Lys Tyr Trp Asp Ile Gln Ile Gly Tyr Gln Thr
            280                 285                 290

aga gag gtt atg tgc att aac aag acg ggg aaa gct gct gat tta agc 1207
Arg Glu Val Met Cys Ile Asn Lys Thr Gly Lys Ala Ala Asp Leu Ser
        295                 300                 305

ttt tgc cag caa gag aag ctt cca atg acc ttc cag tcc tgt gtg atc 1255
Phe Cys Gln Gln Glu Lys Leu Pro Met Thr Phe Gln Ser Cys Val Ile
    310                 315                 320

acc aaa gag tgc cag gtt tcc gag tgg tca gag tgg agc ccc tgc tca 1303
Thr Lys Glu Cys Gln Val Ser Glu Trp Ser Glu Trp Ser Pro Cys Ser
325                 330                 335                 340

aaa aca tgc cat gac atg gtg tcc cct gca ggc act cgt gta agg aca 1351
Lys Thr Cys His Asp Met Val Ser Pro Ala Gly Thr Arg Val Arg Thr
                345                 350                 355

cga acc atc agg cag ttt ccc att ggc agt gaa aag gag tgt cca gaa 1399
Arg Thr Ile Arg Gln Phe Pro Ile Gly Ser Glu Lys Glu Cys Pro Glu
            360                 365                 370

ttt gaa gaa aaa gaa ccc tgt ttg tct caa gga gat gga gtt gtc ccc 1447
Phe Glu Glu Lys Glu Pro Cys Leu Ser Gln Gly Asp Gly Val Val Pro
        375                 380                 385

tgt gcc acg tat ggc tgg aga act aca gag tgg act gag tgc cgt gtg 1495
Cys Ala Thr Tyr Gly Trp Arg Thr Thr Glu Trp Thr Glu Cys Arg Val
    390                 395                 400

gac cct ttg ctc agt cag cag gac aag agg cgc ggc aac cag acg gcc 1543
Asp Pro Leu Leu Ser Gln Gln Asp Lys Arg Arg Gly Asn Gln Thr Ala
405                 410                 415                 420

ctc tgt gga ggg ggc atc cag acc cga gag gtg tac tgc gtg cag gcc 1591
Leu Cys Gly Gly Gly Ile Gln Thr Arg Glu Val Tyr Cys Val Gln Ala
                425                 430                 435

aac gaa aac ctc ctc tca caa tta agt acc cac aag aac aaa gaa gcc 1639
Asn Glu Asn Leu Leu Ser Gln Leu Ser Thr His Lys Asn Lys Glu Ala
            440                 445                 450

tca aag cca atg gac tta aaa tta tgc act gga cct atc cct aat act 1687
Ser Lys Pro Met Asp Leu Lys Leu Cys Thr Gly Pro Ile Pro Asn Thr
        455                 460                 465

aca cag ctg tgc cac att cct tgt cca act gaa tgt gaa gtt tca cct 1735
Thr Gln Leu Cys His Ile Pro Cys Pro Thr Glu Cys Glu Val Ser Pro
    470                 475                 480

tgg tca gct tgg gga cct tgt act tat gaa aac tgt aat gat cag caa 1783
Trp Ser Ala Trp Gly Pro Cys Thr Tyr Glu Asn Cys Asn Asp Gln Gln
485                 490                 495                 500

ggg aaa aaa ggc ttc aaa ctg agg aag cgg cgc att acc aat gag ccc 1831
Gly Lys Lys Gly Phe Lys Leu Arg Lys Arg Arg Ile Thr Asn Glu Pro
                505                 510                 515

act gga ggc tct ggg gta acc gga aac tgc cct cac tta ctg gaa gcc 1879
Thr Gly Gly Ser Gly Val Thr Gly Asn Cys Pro His Leu Leu Glu Ala
            520                 525                 530

att ccc tgt gaa gag cct gcc tgt tat gac tgg aaa gcg gtg aga ctg 1927
Ile Pro Cys Glu Glu Pro Ala Cys Tyr Asp Trp Lys Ala Val Arg Leu

        535                 540                 545

gga gac tgc gag cca gat aac gga aag gag tgt ggt cca ggc acg caa 1975
Gly Asp Cys Glu Pro Asp Asn Gly Lys Glu Cys Gly Pro Gly Thr Gln
    550                 555                 560

gtt caa gag gtt gtg tgc atc aac agt gat gga gaa gaa gtt gac aga 2023
Val Gln Glu Val Val Cys Ile Asn Ser Asp Gly Glu Glu Val Asp Arg
565                 570                 575                 580

cag ctg tgc aga gat gcc atc ttc ccc atc cct gtg gcc tgt gat gcc 2071
Gln Leu Cys Arg Asp Ala Ile Phe Pro Ile Pro Val Ala Cys Asp Ala
                585                 590                 595

cca tgc ccg aaa gac tgt gtg ctc agc aca tgg tct acg tgg tcc tcc 2119
Pro Cys Pro Lys Asp Cys Val Leu Ser Thr Trp Ser Thr Trp Ser Ser
            600                 605                 610

tgc tca cac acc tgc tca ggg aaa acg aca gaa ggg aaa cag ata cga 2167
Cys Ser His Thr Cys Ser Gly Lys Thr Thr Glu Gly Lys Gln Ile Arg
        615                 620                 625

gca cga tcc att ctg gcc tat gcg ggt gaa gaa ggt gga att cgc tgt 2215
Ala Arg Ser Ile Leu Ala Tyr Ala Gly Glu Glu Gly Gly Ile Arg Cys
    630                 635                 640

cca aat agc agt gct ttg caa gaa gta cga agc tgt aat gag cat cct 2263
Pro Asn Ser Ser Ala Leu Gln Glu Val Arg Ser Cys Asn Glu His Pro
645                 650                 655                 660

tgc aca gtg tac cac tgg caa act ggt ccc tgg ggc cag tgc att gag 2311
Cys Thr Val Tyr His Trp Gln Thr Gly Pro Trp Gly Gln Cys Ile Glu
                665                 670                 675

gac acc tca gta tcg tcc ttc aac aca act acg act tgg aat ggg gag 2359
Asp Thr Ser Val Ser Ser Phe Asn Thr Thr Thr Thr Trp Asn Gly Glu
            680                 685                 690

gcc tcc tgc tct gtc ggc atg cag aca aga aaa gtc atc tgt gtg cga 2407
Ala Ser Cys Ser Val Gly Met Gln Thr Arg Lys Val Ile Cys Val Arg
        695                 700                 705

gtc aat gtg ggc caa gtg gga ccc aaa aaa tgt cct gaa agc ctt cga 2455
Val Asn Val Gly Gln Val Gly Pro Lys Lys Cys Pro Glu Ser Leu Arg
    710                 715                 720

cct gaa act gta agg cct tgt ctg ctt cct tgt aag aag gac tgt att 2503
Pro Glu Thr Val Arg Pro Cys Leu Leu Pro Cys Lys Lys Asp Cys Ile
725                 730                 735                 740

gtg acc cca tat agt gac tgg aca tca tgc ccc tct tcg tgt aaa gaa 2551
Val Thr Pro Tyr Ser Asp Trp Thr Ser Cys Pro Ser Ser Cys Lys Glu
                745                 750                 755

ggg gac tcc agt atc agg aag cag tct agg cat cgg gtc atc att cag 2599
Gly Asp Ser Ser Ile Arg Lys Gln Ser Arg His Arg Val Ile Ile Gln
            760                 765                 770

ctg cca gcc aac ggg ggc cga gac tgc aca gat ccc ctc tat gaa gag 2647
Leu Pro Ala Asn Gly Gly Arg Asp Cys Thr Asp Pro Leu Tyr Glu Glu
        775                 780                 785

aag gcc tgt gag gca cct caa gcg tgc caa agc tac agg tgg aag act 2695

Lys Ala Cys Glu Ala Pro Gln Ala Cys Gln Ser Tyr Arg Trp Lys Thr
    790                 795                 800

cac aaa tgg cgc aga tgc caa tta gtc cct tgg agc gtg caa caa gac 2743
His Lys Trp Arg Arg Cys Gln Leu Val Pro Trp Ser Val Gln Gln Asp
805                 810                 815                 820

agc cct gga gca cag gaa ggc tgt ggg cct ggg cga cag gca aga gcc 2791
Ser Pro Gly Ala Gln Glu Gly Cys Gly Pro Gly Arg Gln Ala Arg Ala
                825                 830                 835

att act tgt cgc aag caa gat gga gga cag gct gga atc cat gag tgc 2839
Ile Thr Cys Arg Lys Gln Asp Gly Gly Gln Ala Gly Ile His Glu Cys
            840                 845                 850

cta cag tat gca ggc cct gtg cca gcc ctt acc cag gcc tgc cag atc 2887
Leu Gln Tyr Ala Gly Pro Val Pro Ala Leu Thr Gln Ala Cys Gln Ile
        855                 860                 865

ccc tgc cag gat gac tgt caa ttg acc agc tgg tcc aag ttt tct tca 2935
Pro Cys Gln Asp Asp Cys Gln Leu Thr Ser Trp Ser Lys Phe Ser Ser
    870                 875                 880

tgc aat gga gac tgt ggt gca gtt agg acc aga aag cgc act ctt gtt 2983
Cys Asn Gly Asp Cys Gly Ala Val Arg Thr Arg Lys Arg Thr Leu Val
885                 890                 895                 900

gga aaa agt aaa aag aag gaa aaa tgt aaa aat tcc cat ttg tat ccc 3031
Gly Lys Ser Lys Lys Lys Glu Lys Cys Lys Asn Ser His Leu Tyr Pro
                905                 910                 915

ctg att gag act cag tat tgt cct tgt gac aaa tat aat gca caa cct 3079
Leu Ile Glu Thr Gln Tyr Cys Pro Cys Asp Lys Tyr Asn Ala Gln Pro
            920                 925                 930

gtg ggg aac tgg tca gac tgt att tta cca gag gga aaa gtg gaa gtg 3127
Val Gly Asn Trp Ser Asp Cys Ile Leu Pro Glu Gly Lys Val Glu Val
        935                 940                 945

ttg ctg gga atg aaa gta caa gga gac atc aag gaa tgc gga caa gga 3175
Leu Leu Gly Met Lys Val Gln Gly Asp Ile Lys Glu Cys Gly Gln Gly
    950                 955                 960

tat cgt tac caa gca atg gca tgc tac gat caa aat ggc agg ctt gtg 3223
Tyr Arg Tyr Gln Ala Met Ala Cys Tyr Asp Gln Asn Gly Arg Leu Val
965                 970                 975                 980

gaa aca tct aga tgt aac agc cat ggt tac att gag gag gcc tgc atc 3271
Glu Thr Ser Arg Cys Asn Ser His Gly Tyr Ile Glu Glu Ala Cys Ile
                985                 990                 995

atc ccc tgc ccc tca gac tgc aag ctc agt gag tgg tcc aac tgg 3316
Ile Pro Cys Pro Ser Asp Cys Lys Leu Ser Glu Trp Ser Asn Trp
            1000                1005                1010

tcg cgc tgc agc aag tcc tgt ggg agt ggt gtg aag gtt cgt tct 3361
Ser Arg Cys Ser Lys Ser Cys Gly Ser Gly Val Lys Val Arg Ser
            1015                1020                1025

aaa tgg ctg cgt gaa aaa cca tat aat gga gga agg cct tgc ccc 3406
Lys Trp Leu Arg Glu Lys Pro Tyr Asn Gly Gly Arg Pro Cys Pro
            1030                1035                1040

aaa ctg gac cat gtc aac cag gca cag gtg tat gag gtt gtc cca 3451
Lys Leu Asp His Val Asn Gln Ala Gln Val Tyr Glu Val Val Pro
            1045                1050                1055

tgc cac agt gac tgc aac cag tac cta tgg gtc aca gag ccc tgg 3496
Cys His Ser Asp Cys Asn Gln Tyr Leu Trp Val Thr Glu Pro Trp
            1060                1065                1070

agc atc tgc aag gtg acc ttt gtg aat atg cgg gag aac tgt gga 3541
Ser Ile Cys Lys Val Thr Phe Val Asn Met Arg Glu Asn Cys Gly
            1075                1080                1085

gag ggc gtg caa acc cga aaa gtg aga tgc atg cag aat aca gca 3586
Glu Gly Val Gln Thr Arg Lys Val Arg Cys Met Gln Asn Thr Ala
            1090                1095                1100

gat ggc cct tct gaa cat gta gag gat tac ctc tgt gac cca gaa 3631
Asp Gly Pro Ser Glu His Val Glu Asp Tyr Leu Cys Asp Pro Glu
            1105                1110                1115

gag atg ccc ctg ggc tct aga gtg tgc aaa tta cca tgc cct gag 3676
Glu Met Pro Leu Gly Ser Arg Val Cys Lys Leu Pro Cys Pro Glu
            1120                1125                1130

gac tgt gtg ata tct gaa tgg ggt cca tgg acc caa tgt gtt ttg 3721
Asp Cys Val Ile Ser Glu Trp Gly Pro Trp Thr Gln Cys Val Leu
            1135                1140                1145

cct tgc aat caa agc agt ttc cgg caa agg tca gct gat ccc atc 3766
Pro Cys Asn Gln Ser Ser Phe Arg Gln Arg Ser Ala Asp Pro Ile
            1150                1155                1160

aga caa cca gct gat gaa gga aga tct tgc cct aat gct gtt gag 3811
Arg Gln Pro Ala Asp Glu Gly Arg Ser Cys Pro Asn Ala Val Glu
            1165                1170                1175

aaa gaa ccc tgt aac ctg aac aaa aac tgc tac cac tat gat tat 3856
Lys Glu Pro Cys Asn Leu Asn Lys Asn Cys Tyr His Tyr Asp Tyr
            1180                1185                1190

aat gta aca gac tgg agt aca tgt cag ctg agt gag aag gca gtt 3901
Asn Val Thr Asp Trp Ser Thr Cys Gln Leu Ser Glu Lys Ala Val
            1195                1200                1205

tgt gga aat gga ata aaa aca agg atg ttg gat tgt gtt cga agt 3946
Cys Gly Asn Gly Ile Lys Thr Arg Met Leu Asp Cys Val Arg Ser
            1210                1215                1220

gat ggc aag tca gtt gac ctg aaa tat tgt gaa gcg ctt ggc ttg 3991
Asp Gly Lys Ser Val Asp Leu Lys Tyr Cys Glu Ala Leu Gly Leu
            1225                1230                1235

gag aag aac tgg cag atg aac acg tcc tgc atg gtg gaa tgc cct 4036
Glu Lys Asn Trp Gln Met Asn Thr Ser Cys Met Val Glu Cys Pro
            1240                1245                1250

gtg aac tgt cag ctt tct gat tgg tct cct tgg tca gaa tgt tct 4081
Val Asn Cys Gln Leu Ser Asp Trp Ser Pro Trp Ser Glu Cys Ser
            1255                1260                1265

caa aca tgt ggc ctc aca gga aaa atg atc cga aga cga aca gtg 4126
Gln Thr Cys Gly Leu Thr Gly Lys Met Ile Arg Arg Arg Thr Val
            1270                1275                1280

acc cag ccc ttt caa ggt gat gga aga cca tgc cct tcc ctg atg 4171
Thr Gln Pro Phe Gln Gly Asp Gly Arg Pro Cys Pro Ser Leu Met
            1285                1290                1295

gac cag tcc aaa ccc tgc cca gtg aag cct tgt tat cgg tgg caa 4216
Asp Gln Ser Lys Pro Cys Pro Val Lys Pro Cys Tyr Arg Trp Gln
            1300                1305                1310

tat ggc cag tgg tct cca tgc caa gtg cag gag gcc cag tgt gga 4261
Tyr Gly Gln Trp Ser Pro Cys Gln Val Gln Glu Ala Gln Cys Gly
            1315                1320                1325

gaa ggg acc aga aca agg aac att tct tgt gta gta agt gat ggg 4306
Glu Gly Thr Arg Thr Arg Asn Ile Ser Cys Val Val Ser Asp Gly
            1330                1335                1340

tca gct gat gat ttc agc aaa gtg gtg gat gag gaa ttc tgt gct 4351
Ser Ala Asp Asp Phe Ser Lys Val Val Asp Glu Glu Phe Cys Ala
            1345                1350                1355

gac att gaa ctc att ata gat ggt aat aaa aat atg gtt ctg gag 4396
Asp Ile Glu Leu Ile Ile Asp Gly Asn Lys Asn Met Val Leu Glu
            1360                1365                1370

gaa tcc tgc agc cag cct tgc cca ggt gac tgt tat ttg aag gac 4441
Glu Ser Cys Ser Gln Pro Cys Pro Gly Asp Cys Tyr Leu Lys Asp
            1375                1380                1385

tgg tct tcc tgg agc ctg tgt cag ctg acc tgt gtg aat ggt gag 4486
Trp Ser Ser Trp Ser Leu Cys Gln Leu Thr Cys Val Asn Gly Glu
            1390                1395                1400

gat cta ggc ttt ggt gga ata cag gtc aga tcc aga ccg gtg att 4531
Asp Leu Gly Phe Gly Gly Ile Gln Val Arg Ser Arg Pro Val Ile
            1405                1410                1415

ata caa gaa cta gag aat cag cat ctg tgc cca gag cag atg tta 4576
Ile Gln Glu Leu Glu Asn Gln His Leu Cys Pro Glu Gln Met Leu
            1420                1425                1430

gaa aca aaa tca tgt tat gat gga cag tgc tat gaa tat aaa tgg 4621
Glu Thr Lys Ser Cys Tyr Asp Gly Gln Cys Tyr Glu Tyr Lys Trp
            1435                1440                1445

atg gcc agt gct tgg aag ggc tct tcc cga aca gtg tgg tgt caa 4666
Met Ala Ser Ala Trp Lys Gly Ser Ser Arg Thr Val Trp Cys Gln
            1450                1455                1460

agg tca gat ggt ata aat gta aca ggg ggc tgc ttg gtg atg agc 4711
Arg Ser Asp Gly Ile Asn Val Thr Gly Gly Cys Leu Val Met Ser
            1465                1470                1475

cag cct gat gcc gac agg tct tgt aac cca ccg tgt agt caa ccc 4756
Gln Pro Asp Ala Asp Arg Ser Cys Asn Pro Pro Cys Ser Gln Pro
            1480                1485                1490

cac tcg tac tgt agc gag aca aaa aca tgc cat tgt gaa gaa ggg 4801
His Ser Tyr Cys Ser Glu Thr Lys Thr Cys His Cys Glu Glu Gly
            1495                1500                1505

tac act gaa gtc atg tct tct aac agc acc ctt gag caa tgc aca 4846
Tyr Thr Glu Val Met Ser Ser Asn Ser Thr Leu Glu Gln Cys Thr

1510                1515                1520

ctt atc ccc gtg  gtg gta tta ccc acc  atg gag gac aaa aga  gga      4891
Leu Ile Pro Val  Val Val Leu Pro Thr  Met Glu Asp Lys Arg  Gly
            1525                1530                1535

gat gtg aaa acc  agt cgg gct gta cat  cca acc caa ccc tcc  agt      4936
Asp Val Lys Thr  Ser Arg Ala Val His  Pro Thr Gln Pro Ser  Ser
            1540                1545                1550

aac cca gca gga  cgg gga agg acc tgg  ttt cta cag cca ttt  ggg      4981
Asn Pro Ala Gly  Arg Gly Arg Thr Trp  Phe Leu Gln Pro Phe  Gly
            1555                1560                1565

cca gat ggg aga  cta aag acc tgg gtt  tac ggt gta gca gct  ggg      5026
Pro Asp Gly Arg  Leu Lys Thr Trp Val  Tyr Gly Val Ala Ala  Gly
            1570                1575                1580

gca ttt gtg tta  ctc atc ttt att gtc  tcc atg att tat cta  gct      5071
Ala Phe Val Leu  Leu Ile Phe Ile Val  Ser Met Ile Tyr Leu  Ala
            1585                1590                1595

tgc aaa aag cca  aag aaa ccc caa aga  agg caa aac aac cga  ctg      5116
Cys Lys Lys Pro  Lys Lys Pro Gln Arg  Arg Gln Asn Asn Arg  Leu
            1600                1605                1610

aaa cct tta acc  tta gcc tat gat gga  gat gcc gac atg taacatataa    5165
Lys Pro Leu Thr  Leu Ala Tyr Asp Gly  Asp Ala Asp Met
            1615                1620

cttttcctgg caacaaccag tttcggcttt ctgacttcat agatgtccag aggccacaac    5225

aaatgtatcc aaactgtgtg gattaaaata tattttaatt tttaaaaatg gcatcataaa    5285

gacaagagtg aaaatcatac tgccactgga gatatttaag acagtaccac ttatatacag    5345

accatcaacc gtgagaatta taggagattt agctgaatac atgctgcatt ctgaaagttt    5405

tatgtcatct tttctgaaat ctaccgactg aaaaaccact ttcatctcta aaaaataatg    5465

gtggaattgg ccagttagga tgcctgatac aagaccgtct gcagtgttaa tccataaaac    5525

ttcctagcat gaagagtttc taccaagatc tccacaatac tatggtcaaa ttaacatgtg    5585

tactcagttg aatgacacac attatgtcag attatgtact tgctaataag caattttaac    5645

aatgcataac aaataaactc taagctaagc agaaaatcca ctgaataaat tcagcatctt    5705

ggtggtcgat ggtagatttt attgacctgc atttcagaga caaagcctct tttttaagac    5765

ttcttgtctc tctccaaagt aagaatgctg gacaagtact agtgtcttag aagaacgagt    5825

cctcaagttc agtattttat agtggtaatt gtctggaaaa ctaatttact tgtgttaata    5885

caatacgttt ctactttccc tgattttcaa actggttgcc tgcatctttt ttgctatatg    5945

gaaggcacat ttttgcacta tattagtgca gcacgatagg cgcttaacca gtattgccat    6005

agaaactgcc tcttttcatg tgggatgaag acatctgtgc caagagtggc atgaagacat    6065

ttgcaagttc ttgtatcctg aagagagtaa agttcagttt ggatggcagc aagatgaaat    6125

cagctattac acctgctgta cacacacttc ctcatcactg cagccattgt gaaattgaca    6185

acatggcggt aatttaagtg ttgaagtccc taacccctta accctctaaa aggtggattc 6245

ctctagttgg tttgtaattg ttctttgaag gctgtttatg actagatttt tatatttgtt 6305

atctttgtta ag 6317

<210> 6

<211> 1624

<212> PRT

<213> Homo sapiens

<400> 6

Met Pro Gly Val Leu Ser Leu Val Ile Pro Glu Thr Pro Val Ala Glu
1               5                   10                  15

Asp Leu Ser Pro Ala Val Pro Cys Trp Ile Phe Ile Leu Thr Gly Pro
            20                  25                  30

Trp Gly Arg Cys Met Gly Asp Glu Cys Gly Pro Gly Gly Ile Gln Thr
        35                  40                  45

Arg Ala Val Trp Cys Ala His Val Glu Gly Trp Thr Thr Leu His Thr
    50                  55                  60

Asn Cys Lys Gln Ala Glu Arg Pro Asn Asn Gln Gln Asn Cys Phe Lys
65                  70                  75                  80

Val Cys Asp Trp His Lys Glu Leu Tyr Asp Trp Arg Leu Gly Pro Trp
                85                  90                  95

Asn Gln Cys Gln Pro Val Ile Ser Lys Ser Leu Glu Lys Pro Leu Glu
            100                 105                 110

Cys Ile Lys Gly Glu Glu Gly Ile Gln Val Arg Glu Ile Ala Cys Ile
        115                 120                 125

Gln Lys Asp Lys Asp Ile Pro Ala Glu Asp Ile Ile Cys Glu Tyr Phe
    130                 135                 140

Glu Pro Lys Pro Leu Leu Glu Gln Ala Cys Leu Ile Pro Cys Gln Gln
145                 150                 155                 160

Asp Cys Ile Val Ser Glu Phe Ser Ala Trp Ser Glu Cys Ser Lys Thr
                165                 170                 175

Cys Gly Ser Gly Leu Gln His Arg Thr Arg His Val Val Ala Pro Pro
            180                 185                 190

Gln Phe Gly Gly Ser Gly Cys Pro Asn Leu Thr Glu Phe Gln Val Cys
        195                 200                 205

Gln Ser Ser Pro Cys Glu Ala Glu Glu Leu Arg Tyr Ser Leu His Val
    210                 215                 220

Gly Pro Trp Ser Thr Cys Ser Met Pro His Ser Arg Gln Val Arg Gln
225                 230                 235                 240

Ala Arg Arg Arg Gly Lys Asn Lys Glu Arg Glu Lys Asp Arg Ser Lys
                245                 250                 255

Gly Val Lys Asp Pro Glu Ala Arg Glu Leu Ile Lys Lys Lys Arg Asn
            260                 265                 270

Arg Asn Arg Gln Asn Arg Gln Glu Asn Lys Tyr Trp Asp Ile Gln Ile
        275                 280                 285

Gly Tyr Gln Thr Arg Glu Val Met Cys Ile Asn Lys Thr Gly Lys Ala
    290                 295                 300

Ala Asp Leu Ser Phe Cys Gln Gln Glu Lys Leu Pro Met Thr Phe Gln
305                 310                 315                 320

Ser Cys Val Ile Thr Lys Glu Cys Gln Val Ser Glu Trp Ser Glu Trp
                325                 330                 335

Ser Pro Cys Ser Lys Thr Cys His Asp Met Val Ser Pro Ala Gly Thr
            340                 345                 350

Arg Val Arg Thr Arg Thr Ile Arg Gln Phe Pro Ile Gly Ser Glu Lys
        355                 360                 365

Glu Cys Pro Glu Phe Glu Glu Lys Glu Pro Cys Leu Ser Gln Gly Asp
    370                 375                 380

Gly Val Val Pro Cys Ala Thr Tyr Gly Trp Arg Thr Thr Glu Trp Thr
385                 390                 395                 400

Glu Cys Arg Val Asp Pro Leu Leu Ser Gln Gln Asp Lys Arg Arg Gly
                405                 410                 415

Asn Gln Thr Ala Leu Cys Gly Gly Gly Ile Gln Thr Arg Glu Val Tyr
            420                 425                 430

Cys Val Gln Ala Asn Glu Asn Leu Leu Ser Gln Leu Ser Thr His Lys
        435                 440                 445

Asn Lys Glu Ala Ser Lys Pro Met Asp Leu Lys Leu Cys Thr Gly Pro
    450                 455                 460

Ile Pro Asn Thr Thr Gln Leu Cys His Ile Pro Cys Pro Thr Glu Cys
465                 470                 475                 480

Glu Val Ser Pro Trp Ser Ala Trp Gly Pro Cys Thr Tyr Glu Asn Cys
                485                 490                 495

Asn Asp Gln Gln Gly Lys Lys Gly Phe Lys Leu Arg Lys Arg Arg Ile
            500                 505                 510

Thr Asn Glu Pro Thr Gly Gly Ser Gly Val Thr Gly Asn Cys Pro His
        515                 520                 525

Leu Leu Glu Ala Ile Pro Cys Glu Glu Pro Ala Cys Tyr Asp Trp Lys
    530                 535                 540

Ala Val Arg Leu Gly Asp Cys Glu Pro Asp Asn Gly Lys Glu Cys Gly
545                 550                 555                 560

Pro Gly Thr Gln Val Gln Glu Val Val Cys Ile Asn Ser Asp Gly Glu
                565                 570                 575

Glu Val Asp Arg Gln Leu Cys Arg Asp Ala Ile Phe Pro Ile Pro Val
            580                 585                 590

Ala Cys Asp Ala Pro Cys Pro Lys Asp Cys Val Leu Ser Thr Trp Ser
        595                 600                 605

Thr Trp Ser Ser Cys Ser His Thr Cys Ser Gly Lys Thr Thr Glu Gly
    610                 615                 620

Lys Gln Ile Arg Ala Arg Ser Ile Leu Ala Tyr Ala Gly Glu Glu Gly
625                 630                 635                 640

Gly Ile Arg Cys Pro Asn Ser Ser Ala Leu Gln Glu Val Arg Ser Cys
                645                 650                 655

Asn Glu His Pro Cys Thr Val Tyr His Trp Gln Thr Gly Pro Trp Gly
            660                 665                 670

Gln Cys Ile Glu Asp Thr Ser Val Ser Ser Phe Asn Thr Thr Thr Thr

        675                 680                 685

Trp Asn Gly Glu Ala Ser Cys Ser Val Gly Met Gln Thr Arg Lys Val
    690                 695                 700

Ile Cys Val Arg Val Asn Val Gly Gln Val Gly Pro Lys Lys Cys Pro
705                 710                 715                 720

Glu Ser Leu Arg Pro Glu Thr Val Arg Pro Cys Leu Leu Pro Cys Lys
                725                 730                 735

Lys Asp Cys Ile Val Thr Pro Tyr Ser Asp Trp Thr Ser Cys Pro Ser
            740                 745                 750

Ser Cys Lys Glu Gly Asp Ser Ser Ile Arg Lys Gln Ser Arg His Arg
        755                 760                 765

Val Ile Ile Gln Leu Pro Ala Asn Gly Gly Arg Asp Cys Thr Asp Pro
    770                 775                 780

Leu Tyr Glu Glu Lys Ala Cys Glu Ala Pro Gln Ala Cys Gln Ser Tyr
785                 790                 795                 800

Arg Trp Lys Thr His Lys Trp Arg Arg Cys Gln Leu Val Pro Trp Ser
                805                 810                 815

Val Gln Gln Asp Ser Pro Gly Ala Gln Glu Gly Cys Gly Pro Gly Arg
            820                 825                 830

Gln Ala Arg Ala Ile Thr Cys Arg Lys Gln Asp Gly Gly Gln Ala Gly
        835                 840                 845

Ile His Glu Cys Leu Gln Tyr Ala Gly Pro Val Pro Ala Leu Thr Gln
    850                 855                 860

Ala Cys Gln Ile Pro Cys Gln Asp Asp Cys Gln Leu Thr Ser Trp Ser
865                 870                 875                 880

Lys Phe Ser Ser Cys Asn Gly Asp Cys Gly Ala Val Arg Thr Arg Lys
                885                 890                 895

Arg Thr Leu Val Gly Lys Ser Lys Lys Lys Glu Lys Cys Lys Asn Ser
            900                 905                 910

His Leu Tyr Pro Leu Ile Glu Thr Gln Tyr Cys Pro Cys Asp Lys Tyr
        915                 920                 925

Asn Ala Gln Pro Val Gly Asn Trp Ser Asp Cys Ile Leu Pro Glu Gly
    930                 935                 940

Lys Val Glu Val Leu Leu Gly Met Lys Val Gln Gly Asp Ile Lys Glu
945                 950                 955                 960

Cys Gly Gln Gly Tyr Arg Tyr Gln Ala Met Ala Cys Tyr Asp Gln Asn
                965                 970                 975

Gly Arg Leu Val Glu Thr Ser Arg Cys Asn Ser His Gly Tyr Ile Glu
            980                 985                 990

Glu Ala Cys Ile Ile Pro Cys Pro  Ser Asp Cys Lys Leu  Ser Glu Trp
        995                 1000                 1005

Ser Asn  Trp Ser Arg Cys Ser  Lys Ser Cys Gly Ser  Gly Val Lys
    1010                 1015                 1020

Val Arg  Ser Lys Trp Leu Arg  Glu Lys Pro Tyr Asn  Gly Gly Arg
    1025                 1030                 1035

Pro Cys  Pro Lys Leu Asp His  Val Asn Gln Ala Gln  Val Tyr Glu
    1040                 1045                 1050

Val Val  Pro Cys His Ser Asp  Cys Asn Gln Tyr Leu  Trp Val Thr
    1055                 1060                 1065

Glu Pro  Trp Ser Ile Cys Lys  Val Thr Phe Val Asn  Met Arg Glu
    1070                 1075                 1080

Asn Cys  Gly Glu Gly Val Gln  Thr Arg Lys Val Arg  Cys Met Gln
    1085                 1090                 1095

Asn Thr  Ala Asp Gly Pro Ser  Glu His Val Glu Asp  Tyr Leu Cys
    1100                 1105                 1110

Asp Pro  Glu Glu Met Pro Leu  Gly Ser Arg Val Cys  Lys Leu Pro
    1115                 1120                 1125

Cys Pro  Glu Asp Cys Val Ile  Ser Glu Trp Gly Pro  Trp Thr Gln
    1130                 1135                 1140

Cys Val  Leu Pro Cys Asn Gln  Ser Ser Phe Arg Gln  Arg Ser Ala
    1145                 1150                 1155

Asp Pro  Ile Arg Gln Pro Ala  Asp Glu Gly, Arg Ser  Cys Pro Asn
    1160                 1165                 1170

Ala Val Glu Lys Glu Pro Cys Asn Leu Asn Lys Asn Cys Tyr His
    1175                1180                1185

Tyr Asp Tyr Asn Val Thr Asp Trp Ser Thr Cys Gln Leu Ser Glu
    1190                1195                1200

Lys Ala Val Cys Gly Asn Gly Ile Lys Thr Arg Met Leu Asp Cys
    1205                1210                1215

Val Arg Ser Asp Gly Lys Ser Val Asp Leu Lys Tyr Cys Glu Ala
    1220                1225                1230

Leu Gly Leu Glu Lys Asn Trp Gln Met Asn Thr Ser Cys Met Val
    1235                1240                1245

Glu Cys Pro Val Asn Cys Gln Leu Ser Asp Trp Ser Pro Trp Ser
    1250                1255                1260

Glu Cys Ser Gln Thr Cys Gly Leu Thr Gly Lys Met Ile Arg Arg
    1265                1270                1275

Arg Thr Val Thr Gln Pro Phe Gln Gly Asp Gly Arg Pro Cys Pro
    1280                1285                1290

Ser Leu Met Asp Gln Ser Lys Pro Cys Pro Val Lys Pro Cys Tyr
    1295                1300                1305

Arg Trp Gln Tyr Gly Gln Trp Ser Pro Cys Gln Val Gln Glu Ala
    1310                1315                1320

Gln Cys Gly Glu Gly Thr Arg Thr Arg Asn Ile Ser Cys Val Val
    1325                1330                1335

Ser Asp Gly Ser Ala Asp Asp Phe Ser Lys Val Val Asp Glu Glu
    1340                1345                1350

Phe Cys Ala Asp Ile Glu Leu Ile Ile Asp Gly Asn Lys Asn Met
    1355                1360                1365

Val Leu Glu Glu Ser Cys Ser Gln Pro Cys Pro Gly Asp Cys Tyr
    1370                1375                1380

Leu Lys Asp Trp Ser Ser Trp Ser Leu Cys Gln Leu Thr Cys Val
    1385                1390                1395

Asn Gly Glu Asp Leu Gly Phe Gly Gly Ile Gln Val Arg Ser Arg
    1400                1405                1410

Pro Val Ile Ile Gln Glu Leu Glu Asn Gln His Leu Cys Pro Glu
    1415                1420                1425

Gln Met Leu Glu Thr Lys Ser Cys Tyr Asp Gly Gln Cys Tyr Glu
    1430                1435                1440

Tyr Lys Trp Met Ala Ser Ala Trp Lys Gly Ser Ser Arg Thr Val
    1445                1450                1455

Trp Cys Gln Arg Ser Asp Gly Ile Asn Val Thr Gly Gly Cys Leu
    1460                1465                1470

Val Met Ser Gln Pro Asp Ala Asp Arg Ser Cys Asn Pro Pro Cys
    1475                1480                1485

Ser Gln Pro His Ser Tyr Cys Ser Glu Thr Lys Thr Cys His Cys
    1490                1495                1500

Glu Glu Gly Tyr Thr Glu Val Met Ser Ser Asn Ser Thr Leu Glu
    1505                1510                1515

Gln Cys Thr Leu Ile Pro Val Val Val Leu Pro Thr Met Glu Asp
    1520                1525                1530

Lys Arg Gly Asp Val Lys Thr Ser Arg Ala Val His Pro Thr Gln
    1535                1540                1545

Pro Ser Ser Asn Pro Ala Gly Arg Gly Arg Thr Trp Phe Leu Gln
    1550                1555                1560

Pro Phe Gly Pro Asp Gly Arg Leu Lys Thr Trp Val Tyr Gly Val
    1565                1570                1575

Ala Ala Gly Ala Phe Val Leu Leu Ile Phe Ile Val Ser Met Ile
    1580                1585                1590

Tyr Leu Ala Cys Lys Lys Pro Lys Lys Pro Gln Arg Arg Gln Asn
    1595                1600                1605

Asn Arg Leu Lys Pro Leu Thr Leu Ala Tyr Asp Gly Asp Ala Asp
    1610                1615                1620

Met

<210>  7

<211>  2369

<212>  DNA

<213>  Homo sapiens


<220>

<221>  CDS

<222>  (167)..(2071)


<220>

<221>  sig_peptide

<222>  (167)..(214)


<400>  7
gcccttcctt ccctcatctc tggcggccct cttgggcctc tgacccagcc cctccccggg      60

ccaggctcac agaagctggc ttctgggact gtcctgggcc caagtgggca cctgcgccag     120

ccccacctgt gcctggtctg tggccccttc ctacagggcg ctcacc atg gcc ccg        175
                                                   Met Ala Pro
                                                   1

ccg ctc ctg ctg ctg ctg ctg gcc agt gga gcg gcc gcc tgc ccg ctg       223
Pro Leu Leu Leu Leu Leu Leu Ala Ser Gly Ala Ala Ala Cys Pro Leu
    5                   10                  15

ccc tgc gtc tgc cag aac ctg tcc gag tcg ctc agc acc ctc tgt gcc       271
Pro Cys Val Cys Gln Asn Leu Ser Glu Ser Leu Ser Thr Leu Cys Ala
20                  25                  30                  35

cac cga ggc ctg ctg ttt gtg ccg ccc aac gtg gac cgg cgc aca gtg       319
His Arg Gly Leu Leu Phe Val Pro Pro Asn Val Asp Arg Arg Thr Val
                40                  45                  50

gag ctg cgg ctg gct gac aac ttc atc cag gcc ctg ggg ccc cct gac       367
Glu Leu Arg Leu Ala Asp Asn Phe Ile Gln Ala Leu Gly Pro Pro Asp
            55                  60                  65

ttc cgc aac atg acg gga ctg gtg gac ctg aca ctg tct cgc aat gcc       415
Phe Arg Asn Met Thr Gly Leu Val Asp Leu Thr Leu Ser Arg Asn Ala
        70                  75                  80

atc acc cgc att ggg gcc cgc gcc ttt ggg gac ctc gag agc ctg cgt       463
Ile Thr Arg Ile Gly Ala Arg Ala Phe Gly Asp Leu Glu Ser Leu Arg
    85                  90                  95

tcc ctc cac ctt gac ggc aac agg ctg gtg gag ctg ggc acc ggg agc       511
Ser Leu His Leu Asp Gly Asn Arg Leu Val Glu Leu Gly Thr Gly Ser
100                 105                 110                 115

ctc cgg ggc ccc gtc aat ctg cag cac ctc atc ctc agc ggc aac cag       559

Leu Arg Gly Pro Val Asn Leu Gln His Leu Ile Leu Ser Gly Asn Gln
                120                 125                 130

ctg ggc cgc atc gcg ccg gga gcc ttc gac gac ttc cta gag agc ctg      607
Leu Gly Arg Ile Ala Pro Gly Ala Phe Asp Asp Phe Leu Glu Ser Leu
            135                 140                 145

gag gac ctg gac ctg tcc tac aac aac ctc cgg cag gtg ccc tgg gcc      655
Glu Asp Leu Asp Leu Ser Tyr Asn Asn Leu Arg Gln Val Pro Trp Ala
        150                 155                 160

ggc atc ggc gcc atg cct gcc ctg cac acc ctc aac ctg gac cat aac      703
Gly Ile Gly Ala Met Pro Ala Leu His Thr Leu Asn Leu Asp His Asn
    165                 170                 175

ctt att gac gca ctg ccc cca ggc gcc ttc gcc cag ctc ggt cag ctc      751
Leu Ile Asp Ala Leu Pro Pro Gly Ala Phe Ala Gln Leu Gly Gln Leu
180                 185                 190                 195

tcc cgc ctg gac ctc acc tcc aac cgc ctg gcc acg ctg gct ccg gac      799
Ser Arg Leu Asp Leu Thr Ser Asn Arg Leu Ala Thr Leu Ala Pro Asp
                200                 205                 210

ccg ctt ttc tct cgt ggg cgt gat gca gag gcc tct ccc gcc ccc ctg      847
Pro Leu Phe Ser Arg Gly Arg Asp Ala Glu Ala Ser Pro Ala Pro Leu
            215                 220                 225

gtg ctg agc ttt agc ggg aac ccc ctg cac tgc aac tgt gag ctg ctg      895
Val Leu Ser Phe Ser Gly Asn Pro Leu His Cys Asn Cys Glu Leu Leu
        230                 235                 240

tgg ctg cgg cgg ctg gcg cgg ccg gac gac ctg gaa acg tgc gcc tcc      943
Trp Leu Arg Arg Leu Ala Arg Pro Asp Asp Leu Glu Thr Cys Ala Ser
    245                 250                 255

ccg ccc ggc ctg gcc ggc cgc tac ttc tgg gca gtg ccc gag ggc gag      991
Pro Pro Gly Leu Ala Gly Arg Tyr Phe Trp Ala Val Pro Glu Gly Glu
260                 265                 270                 275

ttc tcc tgt gag ccg ccc ctc att gcc cgc cac acg cag cgc ctc tgg     1039
Phe Ser Cys Glu Pro Pro Leu Ile Ala Arg His Thr Gln Arg Leu Trp
                280                 285                 290

gtg ctg gaa ggc cag cgg gcc acg ctg cgg tgc cgg gcc ctg ggt gac     1087
Val Leu Glu Gly Gln Arg Ala Thr Leu Arg Cys Arg Ala Leu Gly Asp
            295                 300                 305

ccc gcg cct acc atg cac tgg gtc ggt cct gac gac cgg ttg gtt ggc     1135
Pro Ala Pro Thr Met His Trp Val Gly Pro Asp Asp Arg Leu Val Gly
        310                 315                 320

aac tcc tcc cga gcc cgg gct ttc ccc aac ggg acc tta gag att ggg     1183
Asn Ser Ser Arg Ala Arg Ala Phe Pro Asn Gly Thr Leu Glu Ile Gly
    325                 330                 335

gtg acc ggc gct ggg gac gct ggg ggc tac acc tgc atc gcc acc aac     1231
Val Thr Gly Ala Gly Asp Ala Gly Gly Tyr Thr Cys Ile Ala Thr Asn
340                 345                 350                 355

cct gct ggt gag gcc aca gcc cga gta gaa ctg cgg gtg ctg gcc ttg     1279
Pro Ala Gly Glu Ala Thr Ala Arg Val Glu Leu Arg Val Leu Ala Leu
                360                 365                 370

ccc cat ggt ggg aac agc agt gcc gag ggg ggc cgc ccc ggg ccc tcg 1327
Pro His Gly Gly Asn Ser Ser Ala Glu Gly Gly Arg Pro Gly Pro Ser
            375                 380                 385

gac atc gcc gcc tcc gct cgc act gct gcc gag ggt gag ggg acg ctg 1375
Asp Ile Ala Ala Ser Ala Arg Thr Ala Ala Glu Gly Glu Gly Thr Leu
        390                 395                 400

gag tct gag cca gcc gtg cag gtg acg gag gtg acc gcc acc tca ggg 1423
Glu Ser Glu Pro Ala Val Gln Val Thr Glu Val Thr Ala Thr Ser Gly
    405                 410                 415

ctg gtg agc tgg ggt ccc ggg cgg cca gcc gac cca gtg tgg atg ttc 1471
Leu Val Ser Trp Gly Pro Gly Arg Pro Ala Asp Pro Val Trp Met Phe
420                 425                 430                 435

caa atc cag tac aac agc agc gaa gat gag acc ctc atc tac cgg att 1519
Gln Ile Gln Tyr Asn Ser Ser Glu Asp Glu Thr Leu Ile Tyr Arg Ile
                440                 445                 450

gtc cca gcc tcc agc cac cac ttc ctg ctg aag cac ctc gtc ccc ggc 1567
Val Pro Ala Ser Ser His His Phe Leu Leu Lys His Leu Val Pro Gly
            455                 460                 465

gct gac tat gac ctc tgc ctg ctg gcc ttg tca ccg gcc gct ggg ccc 1615
Ala Asp Tyr Asp Leu Cys Leu Leu Ala Leu Ser Pro Ala Ala Gly Pro
        470                 475                 480

tct gac ctc acg gcc acc agg ctg ctg ggc tgt gcc cat ttc tcc acg 1663
Ser Asp Leu Thr Ala Thr Arg Leu Leu Gly Cys Ala His Phe Ser Thr
    485                 490                 495

ctg ccg gcc tcg ccc ctg tgc cac gcc ctg cag gcc cac gtg ctg ggc 1711
Leu Pro Ala Ser Pro Leu Cys His Ala Leu Gln Ala His Val Leu Gly
500                 505                 510                 515

ggg acc ctg acc gtg gcc gtg ggg ggt gtg ctg gtg gct gcc tta ctg 1759
Gly Thr Leu Thr Val Ala Val Gly Gly Val Leu Val Ala Ala Leu Leu
                520                 525                 530

gtc ttc att gtg gcc ttg ctg gtt cgg ggc cgg ggg gcc gga aat ggc 1807
Val Phe Ile Val Ala Leu Leu Val Arg Gly Arg Gly Ala Gly Asn Gly
            535                 540                 545

cgc ctc ccc ctc aag ctc agc cac gtc cag tcc cag acc aat gga ggc 1855
Arg Leu Pro Leu Lys Leu Ser His Val Gln Ser Gln Thr Asn Gly Gly
        550                 555                 560

ccc agc ccc aca ccc aag gcc cac ccg ccg cgg agc ccc ccg ccc cgg 1903
Pro Ser Pro Thr Pro Lys Ala His Pro Pro Arg Ser Pro Pro Pro Arg
    565                 570                 575

ccg cag cgc agc tgc tct ctg gac ctg gga gat gcc ggg tgc tac ggt 1951
Pro Gln Arg Ser Cys Ser Leu Asp Leu Gly Asp Ala Gly Cys Tyr Gly
580                 585                 590                 595

tat gcc agg cgc ctg gga gga gct tgg gcc cga cgg agc cac tct gtg 1999
Tyr Ala Arg Arg Leu Gly Gly Ala Trp Ala Arg Arg Ser His Ser Val
                600                 605                 610

cat ggg ggg ctg ctc ggg gca ggg tgc cgg ggg gta gga ggc agc gcc 2047
His Gly Gly Leu Leu Gly Ala Gly Cys Arg Gly Val Gly Gly Ser Ala
            615                 620                 625

gag cgg ctg gaa gag agt gtg gtg tgatggacgg gcagcttcct gtgtgctcca 2101
Glu Arg Leu Glu Glu Ser Val Val
        630                 635

agggatgagc ctcgtggggc agagggcccg gggccgccgc ctggcctggg agtccctccc 2161

tggtttttat tctcagtacc tcaggctccc ctgtgtactt ggaggggcag ggagcccttt 2221

cctcggttct ggcctccaga ccagggtaag ggcaggcccc tccaacaggt gctcacagcc 2281

accgaggcag gggctgcagc cacccactgg gagtcttgtt tttatttata ataaaattgt 2341

tggggacacc tcaaaaaaaa aaaaaaaa 2369

<210> 8

<211> 635

<212> PRT

<213> Homo sapiens

<400> 8

Met Ala Pro Pro Leu Leu Leu Leu Leu Leu Ala Ser Gly Ala Ala Ala
1               5                   10                  15

Cys Pro Leu Pro Cys Val Cys Gln Asn Leu Ser Glu Ser Leu Ser Thr
            20                  25                  30

Leu Cys Ala His Arg Gly Leu Leu Phe Val Pro Pro Asn Val Asp Arg
        35                  40                  45

Arg Thr Val Glu Leu Arg Leu Ala Asp Asn Phe Ile Gln Ala Leu Gly
    50                  55                  60

Pro Pro Asp Phe Arg Asn Met Thr Gly Leu Val Asp Leu Thr Leu Ser
65                  70                  75                  80

Arg Asn Ala Ile Thr Arg Ile Gly Ala Arg Ala Phe Gly Asp Leu Glu
                85                  90                  95

Ser Leu Arg Ser Leu His Leu Asp Gly Asn Arg Leu Val Glu Leu Gly
            100                 105                 110

Thr Gly Ser Leu Arg Gly Pro Val Asn Leu Gln His Leu Ile Leu Ser
        115                 120                 125

Gly Asn Gln Leu Gly Arg Ile Ala Pro Gly Ala Phe Asp Asp Phe Leu
    130                 135                 140

Glu Ser Leu Glu Asp Leu Asp Leu Ser Tyr Asn Asn Leu Arg Gln Val
145                 150                 155                 160

Pro Trp Ala Gly Ile Gly Ala Met Pro Ala Leu His Thr Leu Asn Leu
                165                 170                 175

Asp His Asn Leu Ile Asp Ala Leu Pro Pro Gly Ala Phe Ala Gln Leu
            180                 185                 190

Gly Gln Leu Ser Arg Leu Asp Leu Thr Ser Asn Arg Leu Ala Thr Leu
        195                 200                 205

Ala Pro Asp Pro Leu Phe Ser Arg Gly Arg Asp Ala Glu Ala Ser Pro
    210                 215                 220

Ala Pro Leu Val Leu Ser Phe Ser Gly Asn Pro Leu His Cys Asn Cys
225                 230                 235                 240

Glu Leu Leu Trp Leu Arg Arg Leu Ala Arg Pro Asp Asp Leu Glu Thr
                245                 250                 255

Cys Ala Ser Pro Pro Gly Leu Ala Gly Arg Tyr Phe Trp Ala Val Pro
            260                 265                 270

Glu Gly Glu Phe Ser Cys Glu Pro Pro Leu Ile Ala Arg His Thr Gln
        275                 280                 285

Arg Leu Trp Val Leu Glu Gly Gln Arg Ala Thr Leu Arg Cys Arg Ala
    290                 295                 300

Leu Gly Asp Pro Ala Pro Thr Met His Trp Val Gly Pro Asp Asp Arg
305                 310                 315                 320

Leu Val Gly Asn Ser Ser Arg Ala Arg Ala Phe Pro Asn Gly Thr Leu
                325                 330                 335

Glu Ile Gly Val Thr Gly Ala Gly Asp Ala Gly Gly Tyr Thr Cys Ile
            340                 345                 350

Ala Thr Asn Pro Ala Gly Glu Ala Thr Ala Arg Val Glu Leu Arg Val
        355                 360                 365

Leu Ala Leu Pro His Gly Gly Asn Ser Ser Ala Glu Gly Gly Arg Pro
    370                 375                 380

Gly Pro Ser Asp Ile Ala Ala Ser Ala Arg Thr Ala Ala Glu Gly Glu
385                 390                 395                 400

Gly Thr Leu Glu Ser Glu Pro Ala Val Gln Val Thr Glu Val Thr Ala
                405                 410                 415

Thr Ser Gly Leu Val Ser Trp Gly Pro Gly Arg Pro Ala Asp Pro Val
            420                 425                 430

Trp Met Phe Gln Ile Gln Tyr Asn Ser Ser Glu Asp Glu Thr Leu Ile
        435                 440                 445

Tyr Arg Ile Val Pro Ala Ser Ser His His Phe Leu Leu Lys His Leu
    450                 455                 460

Val Pro Gly Ala Asp Tyr Asp Leu Cys Leu Leu Ala Leu Ser Pro Ala
465                 470                 475                 480

Ala Gly Pro Ser Asp Leu Thr Ala Thr Arg Leu Leu Gly Cys Ala His
                485                 490                 495

Phe Ser Thr Leu Pro Ala Ser Pro Leu Cys His Ala Leu Gln Ala His
            500                 505                 510

Val Leu Gly Gly Thr Leu Thr Val Ala Val Gly Gly Val Leu Val Ala
        515                 520                 525

Ala Leu Leu Val Phe Ile Val Ala Leu Leu Val Arg Gly Arg Gly Ala
    530                 535                 540

Gly Asn Gly Arg Leu Pro Leu Lys Leu Ser His Val Gln Ser Gln Thr
545                 550                 555                 560

Asn Gly Gly Pro Ser Pro Thr Pro Lys Ala His Pro Pro Arg Ser Pro
                565                 570                 575

Pro Pro Arg Pro Gln Arg Ser Cys Ser Leu Asp Leu Gly Asp Ala Gly
            580                 585                 590

Cys Tyr Gly Tyr Ala Arg Arg Leu Gly Gly Ala Trp Ala Arg Arg Ser
        595                 600                 605

His Ser Val His Gly Gly Leu Leu Gly Ala Gly Cys Arg Gly Val Gly
    610                 615                 620

Gly Ser Ala Glu Arg Leu Glu Glu Ser Val Val
625                 630                 635

<210>  9

<211>  2014

<212>  DNA

<213>  Homo sapiens

<220>

<221>  CDS

<222>  (231)..(1658)

<220>

<221>  sig_peptide

<222>  (231)..(290)

<400>  9
ctgctgctgc tgcaaggggt ggtggcgagt ttcaggattc tatcaaggaa gccaagacct      60

aaggagacag agggatatct atttttacaa tatttttctt tattttcttt cttccttgcg     120

cgtggcaagc tttttttttt ctttttcttt tttcttacca gcctcccccc aatgaggtag     180

ctcagtggca tttctgagca ggggccaccc tgacttcacc ttggcccacc atg agg        236
                                                      Met Arg
                                                      1

gtc ttc ctg ctt tgt gcc tac ata ctg ctg ctg atg gtt tcc cag ttg       284
Val Phe Leu Leu Cys Ala Tyr Ile Leu Leu Leu Met Val Ser Gln Leu
        5                   10                  15

agg gca gtc agc ttt cct gaa gat gat gaa ccc ctt aat act gtc gac       332
Arg Ala Val Ser Phe Pro Glu Asp Asp Glu Pro Leu Asn Thr Val Asp
    20                  25                  30

tat cac tat tca agg caa tat ccg gtt ttt aga gga cgc cct tca ggc       380
Tyr His Tyr Ser Arg Gln Tyr Pro Val Phe Arg Gly Arg Pro Ser Gly
35                  40                  45                  50

aat gaa tcg cag cac agg ctg gac ttt cag ctg atg ttg aaa att cga       428
Asn Glu Ser Gln His Arg Leu Asp Phe Gln Leu Met Leu Lys Ile Arg
                55                  60                  65

gac aca ctt tat att gct ggc agg gat caa gtt tat aca gta aac tta       476
Asp Thr Leu Tyr Ile Ala Gly Arg Asp Gln Val Tyr Thr Val Asn Leu
            70                  75                  80

aat gaa atg ccc aaa aca gaa gta ata ccc aac aag aaa ctg aca tgg       524
Asn Glu Met Pro Lys Thr Glu Val Ile Pro Asn Lys Lys Leu Thr Trp
        85                  90                  95

cga tca aga caa cag gat cga gaa aac tgt gct atg aaa ggc aag cat       572
Arg Ser Arg Gln Gln Asp Arg Glu Asn Cys Ala Met Lys Gly Lys His
    100                 105                 110

aaa gat gaa tgc cac aac ttt atc aaa gta ttt gtt cca aga aac gat 620
Lys Asp Glu Cys His Asn Phe Ile Lys Val Phe Val Pro Arg Asn Asp
115                 120                 125                 130

gag atg gtt ttt gtt tgt ggt acc aat gca ttc aat ccc atg tgt aga 668
Glu Met Val Phe Val Cys Gly Thr Asn Ala Phe Asn Pro Met Cys Arg
                135                 140                 145

tac tac agg ttg agt acc tta gaa tat gat ggg gaa gaa att agt ggc 716
Tyr Tyr Arg Leu Ser Thr Leu Glu Tyr Asp Gly Glu Glu Ile Ser Gly
            150                 155                 160

ctg gca aga tgc cca ttt gat gcc aga caa acc aat gtt gcc ctc ttt 764
Leu Ala Arg Cys Pro Phe Asp Ala Arg Gln Thr Asn Val Ala Leu Phe
        165                 170                 175

gct gat ggg aag ctg tat tct gcc aca gtg gct gac ttc ttg gcc agc 812
Ala Asp Gly Lys Leu Tyr Ser Ala Thr Val Ala Asp Phe Leu Ala Ser
    180                 185                 190

gat gcc gtt att tat cga agc atg ggt gat gga tct gcc ctt cgc aca 860
Asp Ala Val Ile Tyr Arg Ser Met Gly Asp Gly Ser Ala Leu Arg Thr
195                 200                 205                 210

ata aaa tat gat tcc aaa tgg ata aaa gag cca cac ttt ctt cat gcc 908
Ile Lys Tyr Asp Ser Lys Trp Ile Lys Glu Pro His Phe Leu His Ala
                215                 220                 225

ata gaa tat gga aac tat gtc tat ttc ttc ttt cga gaa atc gct gtc 956
Ile Glu Tyr Gly Asn Tyr Val Tyr Phe Phe Phe Arg Glu Ile Ala Val
            230                 235                 240

gaa cat aat aat tta ggc aag gct gtg tat tcc cgc gtg gcc cgc ata 1004
Glu His Asn Asn Leu Gly Lys Ala Val Tyr Ser Arg Val Ala Arg Ile
        245                 250                 255

tgt aaa aac gac atg ggt ggt tcc cag cgg gtc ctg gag aaa cac tgg 1052
Cys Lys Asn Asp Met Gly Gly Ser Gln Arg Val Leu Glu Lys His Trp
    260                 265                 270

act tca ttt cta aag gct cgg ctg aac tgt tct gtc cct gga gat tcg 1100
Thr Ser Phe Leu Lys Ala Arg Leu Asn Cys Ser Val Pro Gly Asp Ser
275                 280                 285                 290

ttt ttc tac ttt gat gtt ctg cag tct att aca gac ata ata caa atc 1148
Phe Phe Tyr Phe Asp Val Leu Gln Ser Ile Thr Asp Ile Ile Gln Ile
                295                 300                 305

aat ggc atc ccc act gtg gtc ggg gtg ttt acc acg cag ctc aat agc 1196
Asn Gly Ile Pro Thr Val Val Gly Val Phe Thr Thr Gln Leu Asn Ser
            310                 315                 320

atc cct ggt tct gct gtc tgt gca ttt agc atg gat gac att gaa aaa 1244
Ile Pro Gly Ser Ala Val Cys Ala Phe Ser Met Asp Asp Ile Glu Lys
        325                 330                 335

gta ttc aaa gga cgg ttt aag gaa cag aaa act cca gat tct gtt tgg 1292
Val Phe Lys Gly Arg Phe Lys Glu Gln Lys Thr Pro Asp Ser Val Trp
    340                 345                 350

aca gca gtt ccc gaa gac aaa gtg cca aag cca agg cct ggc tgt tgt 1340
Thr Ala Val Pro Glu Asp Lys Val Pro Lys Pro Arg Pro Gly Cys Cys

355                 360                 365                 370

gca aaa cac ggc ctt gcc gaa gct tat aaa acc tcc atc gat ttc ccg      1388
Ala Lys His Gly Leu Ala Glu Ala Tyr Lys Thr Ser Ile Asp Phe Pro
                375                 380                 385

gat gaa act ctg tca ttc atc aaa tct cat ccc ctg atg gac tct gcc      1436
Asp Glu Thr Leu Ser Phe Ile Lys Ser His Pro Leu Met Asp Ser Ala
            390                 395                 400

gtt cca ccc att gcc gat gag ccc tgg ttc aca aag act cgg gtc agg      1484
Val Pro Pro Ile Ala Asp Glu Pro Trp Phe Thr Lys Thr Arg Val Arg
        405                 410                 415

tac aga ctg acg gcc atc tca gtg gac cat tca gcc gga ccc tac cag      1532
Tyr Arg Leu Thr Ala Ile Ser Val Asp His Ser Ala Gly Pro Tyr Gln
    420                 425                 430

aac tac aca gtc atc ttt gtt ggc tct gaa gct ggc atg gta ctt aaa      1580
Asn Tyr Thr Val Ile Phe Val Gly Ser Glu Ala Gly Met Val Leu Lys
435                 440                 445                 450

gtt ctg gca aag acc agt cct ttc tct ttg aac gac agc gta tta ctg      1628
Val Leu Ala Lys Thr Ser Pro Phe Ser Leu Asn Asp Ser Val Leu Leu
                455                 460                 465

gaa gag att gaa gcc tac aac cat gca aag taggtatatg ttacgagaac        1678
Glu Glu Ile Glu Ala Tyr Asn His Ala Lys
            470                 475

gcccttcagc actgctcaaa aattttcggc atgtatttca tctagtcatg tccttttggt    1738

cctctaaatt agcagtggtt tggcataata gtgttttgtg ttttttttct cattgaaata    1798

aatcttgggt ttgttttttt cccgagcctg ctagggcgag gggggtgaat ggttgatgag    1858

tttaaaaata atgcagccct tgtttttcac ctgtagaata tgagaacatt ttaacagcac    1918

ctctcttatc ttgcagatat attccaagat gctacatgca gcagacagct gtgagcttgc    1978

atacacacac cacacacact atgttctaga tcgcga                              2014

<210>  10

<211>  476

<212>  PRT

<213>  Homo sapiens


<400>  10

Met Arg Val Phe Leu Leu Cys Ala Tyr Ile Leu Leu Leu Met Val Ser
1               5                   10                  15


Gln Leu Arg Ala Val Ser Phe Pro Glu Asp Asp Glu Pro Leu Asn Thr
            20                  25                  30

Val Asp Tyr His Tyr Ser Arg Gln Tyr Pro Val Phe Arg Gly Arg Pro
        35                  40                  45

Ser Gly Asn Glu Ser Gln His Arg Leu Asp Phe Gln Leu Met Leu Lys
    50                  55                  60

Ile Arg Asp Thr Leu Tyr Ile Ala Gly Arg Asp Gln Val Tyr Thr Val
65                  70                  75                  80

Asn Leu Asn Glu Met Pro Lys Thr Glu Val Ile Pro Asn Lys Lys Leu
                85                  90                  95

Thr Trp Arg Ser Arg Gln Gln Asp Arg Glu Asn Cys Ala Met Lys Gly
            100                 105                 110

Lys His Lys Asp Glu Cys His Asn Phe Ile Lys Val Phe Val Pro Arg
        115                 120                 125

Asn Asp Glu Met Val Phe Val Cys Gly Thr Asn Ala Phe Asn Pro Met
    130                 135                 140

Cys Arg Tyr Tyr Arg Leu Ser Thr Leu Glu Tyr Asp Gly Glu Glu Ile
145                 150                 155                 160

Ser Gly Leu Ala Arg Cys Pro Phe Asp Ala Arg Gln Thr Asn Val Ala
                165                 170                 175

Leu Phe Ala Asp Gly Lys Leu Tyr Ser Ala Thr Val Ala Asp Phe Leu
            180                 185                 190

Ala Ser Asp Ala Val Ile Tyr Arg Ser Met Gly Asp Gly Ser Ala Leu
        195                 200                 205

Arg Thr Ile Lys Tyr Asp Ser Lys Trp Ile Lys Glu Pro His Phe Leu
    210                 215                 220

His Ala Ile Glu Tyr Gly Asn Tyr Val Tyr Phe Phe Phe Arg Glu Ile
225                 230                 235                 240

Ala Val Glu His Asn Asn Leu Gly Lys Ala Val Tyr Ser Arg Val Ala
                245                 250                 255

Arg Ile Cys Lys Asn Asp Met Gly Gly Ser Gln Arg Val Leu Glu Lys
            260                 265                 270

His Trp Thr Ser Phe Leu Lys Ala Arg Leu Asn Cys Ser Val Pro Gly
        275                 280                 285

Asp Ser Phe Phe Tyr Phe Asp Val Leu Gln Ser Ile Thr Asp Ile Ile
    290                 295                 300

Gln Ile Asn Gly Ile Pro Thr Val Val Gly Val Phe Thr Thr Gln Leu
305                 310                 315                 320

Asn Ser Ile Pro Gly Ser Ala Val Cys Ala Phe Ser Met Asp Asp Ile
                325                 330                 335

Glu Lys Val Phe Lys Gly Arg Phe Lys Glu Gln Lys Thr Pro Asp Ser
            340                 345                 350

Val Trp Thr Ala Val Pro Glu Asp Lys Val Pro Lys Pro Arg Pro Gly
        355                 360                 365

Cys Cys Ala Lys His Gly Leu Ala Glu Ala Tyr Lys Thr Ser Ile Asp
    370                 375                 380

Phe Pro Asp Glu Thr Leu Ser Phe Ile Lys Ser His Pro Leu Met Asp
385                 390                 395                 400

Ser Ala Val Pro Pro Ile Ala Asp Glu Pro Trp Phe Thr Lys Thr Arg
                405                 410                 415

Val Arg Tyr Arg Leu Thr Ala Ile Ser Val Asp His Ser Ala Gly Pro
            420                 425                 430

Tyr Gln Asn Tyr Thr Val Ile Phe Val Gly Ser Glu Ala Gly Met Val
        435                 440                 445

Leu Lys Val Leu Ala Lys Thr Ser Pro Phe Ser Leu Asn Asp Ser Val
    450                 455                 460

Leu Leu Glu Glu Ile Glu Ala Tyr Asn His Ala Lys
465                 470                 475

<210>  11

<211>  1054

<212>  DNA

<213>  Homo sapiens


<220>

<221>  CDS

<222>  (119)..(1051)

<220>

<221>  sig_peptide

<222>  (119)..(172)

<400>  11
tgcagattgc aagattgtgc acgagtacat tggcggctac attttcttgt ccacccgctc      60

caaggaccag aatgaaacac tcgatgagga cttgttccac aaattgaccg gcgacgcc       118

atg cgg caa acc cta ccg ctg ctg ctg ctg acg gtg ctg cgc ccc agc       166
Met Arg Gln Thr Leu Pro Leu Leu Leu Leu Thr Val Leu Arg Pro Ser
1               5                   10                  15

tgg gca gac cct ccc cag gag aag gtc ccg ctc ttc cgg gtc act cag       214
Trp Ala Asp Pro Pro Gln Glu Lys Val Pro Leu Phe Arg Val Thr Gln
            20                  25                  30

cag ggc ccc tgg ggg agc agt ggc agc aac gcc acc gac tcg ccc tgc       262
Gln Gly Pro Trp Gly Ser Ser Gly Ser Asn Ala Thr Asp Ser Pro Cys
        35                  40                  45

gag ggg ctg ccc gcc gcg gat gcg acg gcc ttg acc ctg gcg aac cgc       310
Glu Gly Leu Pro Ala Ala Asp Ala Thr Ala Leu Thr Leu Ala Asn Arg
    50                  55                  60

aac ctg gag cgc ctg ccc ggc tgc cta ccg cgc aca ctg cgc agc ctc       358
Asn Leu Glu Arg Leu Pro Gly Cys Leu Pro Arg Thr Leu Arg Ser Leu
65                  70                  75                  80

gac gcc agc cac aac ctg ctg cgc gcc ctg agc act tcc gag ctc ggc       406
Asp Ala Ser His Asn Leu Leu Arg Ala Leu Ser Thr Ser Glu Leu Gly
                85                  90                  95

cac ctg gag cag ctg cag gtg ctg acc ctg cgc cac aac cgc atc gcc       454
His Leu Glu Gln Leu Gln Val Leu Thr Leu Arg His Asn Arg Ile Ala
            100                 105                 110

gcg ctg cgc tgg ggc ccg ggt ggg ccg gcg ggg ctg cac acc ctg gac       502
Ala Leu Arg Trp Gly Pro Gly Gly Pro Ala Gly Leu His Thr Leu Asp
        115                 120                 125

ctc agc tac aac cag ctg gcc gct ctg ccg ccg tgc acc ggg ccc gcg       550
Leu Ser Tyr Asn Gln Leu Ala Ala Leu Pro Pro Cys Thr Gly Pro Ala
    130                 135                 140

ctg agc agc ctc cgc gcc ctg gcg ctc gcc ggg aat ccg ctg cgg gcg       598
Leu Ser Ser Leu Arg Ala Leu Ala Leu Ala Gly Asn Pro Leu Arg Ala
145                 150                 155                 160

ctg cag ccc cgg gcc ttc gcc tgc ttc ccc gcg ctg cag ctc ctc aac       646
Leu Gln Pro Arg Ala Phe Ala Cys Phe Pro Ala Leu Gln Leu Leu Asn
                165                 170                 175

ctc tcc tgc acc gcg ctg ggt cgc gga gcc cag ggg ggc atc gcc gag       694
Leu Ser Cys Thr Ala Leu Gly Arg Gly Ala Gln Gly Gly Ile Ala Glu

            180                 185                 190

gcg gcg ttc gct gga gag gat ggc gcg ccc ctg gtc acg ctc gaa gtc 742
Ala Ala Phe Ala Gly Glu Asp Gly Ala Pro Leu Val Thr Leu Glu Val
        195                 200                 205

ctg gat ctc agc ggc acg ttc ctt gaa cgg gtt gag tca ggg tgg atc 790
Leu Asp Leu Ser Gly Thr Phe Leu Glu Arg Val Glu Ser Gly Trp Ile
    210                 215                 220

aga gac ctg ccg aag ctc aca tcc ctc tac ctg agg aag atg cct cgg 838
Arg Asp Leu Pro Lys Leu Thr Ser Leu Tyr Leu Arg Lys Met Pro Arg
225                 230                 235                 240

ctg acg acc ctg gag ggg gac att ttc aag atg acc ccc aac ctg cag 886
Leu Thr Thr Leu Glu Gly Asp Ile Phe Lys Met Thr Pro Asn Leu Gln
                245                 250                 255

cag ctg gac tgt cag gac tcc cca gca ctt gct tct gtc gcc aca cac 934
Gln Leu Asp Cys Gln Asp Ser Pro Ala Leu Ala Ser Val Ala Thr His
            260                 265                 270

atc ttt caa gat act cca cat cta cag gtc ctt ctg ttc cag aat gaa 982
Ile Phe Gln Asp Thr Pro His Leu Gln Val Leu Leu Phe Gln Asn Glu
        275                 280                 285

gat gat ggt ggt ggt tat aat gga gat gct gat ggt gat gac agt gat 1030
Asp Asp Gly Gly Gly Tyr Asn Gly Asp Ala Asp Gly Asp Asp Ser Asp
    290                 295                 300

ggt gat ggt gtt gat gct gat tga 1054
Gly Asp Gly Val Asp Ala Asp
305                 310

<210> 12

<211> 311

<212> PRT

<213> Homo sapiens

<400> 12

Met Arg Gln Thr Leu Pro Leu Leu Leu Leu Thr Val Leu Arg Pro Ser
1               5                   10                  15

Trp Ala Asp Pro Pro Gln Glu Lys Val Pro Leu Phe Arg Val Thr Gln
            20                  25                  30

Gln Gly Pro Trp Gly Ser Ser Gly Ser Asn Ala Thr Asp Ser Pro Cys
        35                  40                  45

Glu Gly Leu Pro Ala Ala Asp Ala Thr Ala Leu Thr Leu Ala Asn Arg
    50                  55                  60

Asn Leu Glu Arg Leu Pro Gly Cys Leu Pro Arg Thr Leu Arg Ser Leu
65                  70                  75                  80

Asp Ala Ser His Asn Leu Leu Arg Ala Leu Ser Thr Ser Glu Leu Gly
                85                  90                  95

His Leu Glu Gln Leu Gln Val Leu Thr Leu Arg His Asn Arg Ile Ala
            100                 105                 110

Ala Leu Arg Trp Gly Pro Gly Gly Pro Ala Gly Leu His Thr Leu Asp
        115                 120                 125

Leu Ser Tyr Asn Gln Leu Ala Ala Leu Pro Pro Cys Thr Gly Pro Ala
    130                 135                 140

Leu Ser Ser Leu Arg Ala Leu Ala Leu Ala Gly Asn Pro Leu Arg Ala
145                 150                 155                 160

Leu Gln Pro Arg Ala Phe Ala Cys Phe Pro Ala Leu Gln Leu Leu Asn
                165                 170                 175

Leu Ser Cys Thr Ala Leu Gly Arg Gly Ala Gln Gly Gly Ile Ala Glu
            180                 185                 190

Ala Ala Phe Ala Gly Glu Asp Gly Ala Pro Leu Val Thr Leu Glu Val
        195                 200                 205

Leu Asp Leu Ser Gly Thr Phe Leu Glu Arg Val Glu Ser Gly Trp Ile
    210                 215                 220

Arg Asp Leu Pro Lys Leu Thr Ser Leu Tyr Leu Arg Lys Met Pro Arg
225                 230                 235                 240

Leu Thr Thr Leu Glu Gly Asp Ile Phe Lys Met Thr Pro Asn Leu Gln
                245                 250                 255

Gln Leu Asp Cys Gln Asp Ser Pro Ala Leu Ala Ser Val Ala Thr His
            260                 265                 270

Ile Phe Gln Asp Thr Pro His Leu Gln Val Leu Leu Phe Gln Asn Glu
        275                 280                 285

Asp Asp Gly Gly Gly Tyr Asn Gly Asp Ala Asp Gly Asp Asp Ser Asp
    290                 295                 300

Gly Asp Gly Val Asp Ala Asp
305                 310

<210>  13

<211>  1013

<212>  DNA

<213>  Homo sapiens

<220>

<221>  CDS

<222>  (90)..(458)

<220>

<221>  sig_peptide

<222>  (90)..(143)

<400>  13
ctttcctctc ctgactaagt ttctctggct tccctgaggc tgcaggtgtt aatctggggg      60
gccctgggcc ctgagccggc agcagaaat atg agg acc cag agc ctt ctc ctc      113
ctg ggg gcc ctc ctg gct gtg ggg agt cag ctg cct gct gtc ttt ggc      161
agg aag aag gga gag aaa tcg ggg ggc tgc ccg cca gat gat ggg ccc      209
tgc ctc cta tcg gtg cct gac cag tgc gtg gaa gac agc cag tgt ccc      257
ttg acc agg aag tgc tgc tac aga gct tgc ttc cgc cag tgt gtc ccc      305
agg gtc tct gtg aag ctg ggc agc tgc cca gag gac caa ctg cgc tgc      353
ctc agc ccc atg aac cac ctg tgt tac aag gac tca gac tgc tcg ggc      401
aaa aag cga tgc tgc cac agc gcc tgc ggg cgg gat tgc cgg gat cct      449
gcc aga ggc taattctgat ttaggatctg tggctctgca cctaagctgg              498
ggaccaacgg aaagagttca cgatgggagg cctggggccc tgcccgctgg acagcactat 558
ctctaccagc ggtggttcca gccttctgat aatcactggc ctgctgacac ttccctgcaa 618
cccatccacc cctggtttct cctcctggga gtcaaagtcc atagcctgag ctcggaggaa 678
ggcctctgta tcaccccagt actctgcacc actgccatac gagcttccca cccttcctaa 738
cgctttcaca ccaatccgta catgctgctt cctccaccaa aaatgcccaa ttcaggcaga 798
ccctgacctc tccctcaggc agcccaacca tccagaatga atattcttgc agagttttcc 858
aaacatcagt cattcacctc tttcatgatt ttcaccatac ctacaaaata gcaccatgat 918
aggttgcacg ctgcctgtac caccatttac ttaatgtttt ctttaaatgg ctcacttttg 978
tatataaata aattcatttc aaaaaaaaaa aaaaa 1013

<210> 14

<211> 123

<212> PRT

<213> Homo sapiens

<400> 14

<210>  15

<211>  1165

<212>  DNA

<213>  Homo sapiens

<220>

<221>  CDS

<222>  (100)..(651)

<220>

<221>  sig_peptide

<222>  (100)..(180)

<400>  15
gtgaagcttc tccattctgt aagctttccg ggaacatcca aggcaagact ggcacccagc      60

acagcagtga ctgaccacat accccactct ccaggaccc atg gag tcc ttc agc      114
                                           Met Glu Ser Phe Ser
                                           1               5

tca aag agc ctg gca ctg caa gca gag aag aag cta ctg agt aag atg      162
Ser Lys Ser Leu Ala Leu Gln Ala Glu Lys Lys Leu Leu Ser Lys Met
                10                  15                  20

gcg ggt cgc tct gtg gct cat ctc ttc ata gat gag aca agc agt gag      210
Ala Gly Arg Ser Val Ala His Leu Phe Ile Asp Glu Thr Ser Ser Glu
            25                  30                  35

gtg cta gat gag ctc tac cgt gtg tcc aag gag tac acg cac agc cgg      258
Val Leu Asp Glu Leu Tyr Arg Val Ser Lys Glu Tyr Thr His Ser Arg
        40                  45                  50

ccc cag gcc cag cgc gtg atc aag gac ctg atc aaa gtg gcc atc aag      306
Pro Gln Ala Gln Arg Val Ile Lys Asp Leu Ile Lys Val Ala Ile Lys
    55                  60                  65

gtg gct gtg ctg cac cgc aat ggc tcc ttt ggc ccc agt gag ctg gcc      354
Val Ala Val Leu His Arg Asn Gly Ser Phe Gly Pro Ser Glu Leu Ala
70                  75                  80                  85

ctg gct acc cgc ttt cgc cag aag ctg cgg cag ggt gcc atg acg gca      402
Leu Ala Thr Arg Phe Arg Gln Lys Leu Arg Gln Gly Ala Met Thr Ala
                90                  95                  100

ctt agc ttt ggt gag gta gac ttc acc ttc gag gct gct gtt ctg gct      450
Leu Ser Phe Gly Glu Val Asp Phe Thr Phe Glu Ala Ala Val Leu Ala
            105                 110                 115

ggc ctg ctg acc gag tgc cgg gat gtg ctg cta gag ttg gtg gaa cac 498
Gly Leu Leu Thr Glu Cys Arg Asp Val Leu Leu Glu Leu Val Glu His
        120                 125                 130

cac ctc acg ccc aag tca cat ggc cgc atc cgc cac gtg ttt gat cac 546
His Leu Thr Pro Lys Ser His Gly Arg Ile Arg His Val Phe Asp His
    135                 140                 145

ttc tct gac cca ggt ctg ctc acg gcc ctc tat ggg cct gac ttc act 594
Phe Ser Asp Pro Gly Leu Leu Thr Ala Leu Tyr Gly Pro Asp Phe Thr
150                 155                 160                 165

cag cac ctt ggc aag atc tgt gac gga ctc agg aag ctg cta gac gaa 642
Gln His Leu Gly Lys Ile Cys Asp Gly Leu Arg Lys Leu Leu Asp Glu
                170                 175                 180

ggg aag ctc tgagagccct gagcctagca cattccacct tgacaaaatg 691
Gly Lys Leu

gttgactgag aaaacacaga taatgggctt cctaaccctg ctcacctggc actaacactt 751

ttcaatcttc aggcttcatt ccttcccaag agtgcttttg actctgagac cagcccaccc 811

ccaaacagct agtggagaag gagcaatgct gaggggtgag gcctctctcc cactccagcc 871

ccaggacagg aaacagaact gcctgaaaaa ggtgaagtga aacttggatc tctatttctc 931

ccataaggga cttctgaaac agggaagccc cctcccatgt gaaccaagga aaggaggcac 991

agcccagaga accccttggg ggatactaaa gacagaagag gggaaggtgg cccttagaga 1051

cagagcttgg acagatgcca gaggctctgt tccagagtgc aggaagaagg ggctagggca 1111

ggggagattc tcatagggga aataaaacta ctaaaatatg aaaaaaaaaa aaaa 1165

<210> 16

<211> 184

<212> PRT

<213> Homo sapiens

<400> 16

Met Glu Ser Phe Ser Ser Lys Ser Leu Ala Leu Gln Ala Glu Lys Lys
1               5                   10                  15

Leu Leu Ser Lys Met Ala Gly Arg Ser Val Ala His Leu Phe Ile Asp
            20                  25                  30

Glu Thr Ser Ser Glu Val Leu Asp Glu Leu Tyr Arg Val Ser Lys Glu
        35                  40                  45

Tyr Thr His Ser Arg Pro Gln Ala Gln Arg Val Ile Lys Asp Leu Ile

50                  55                  60

Lys Val Ala Ile Lys Val Ala Val Leu His Arg Asn Gly Ser Phe Gly
65                  70                  75                  80

Pro Ser Glu Leu Ala Leu Ala Thr Arg Phe Arg Gln Lys Leu Arg Gln
                85                  90                  95

Gly Ala Met Thr Ala Leu Ser Phe Gly Glu Val Asp Phe Thr Phe Glu
            100                 105                 110

Ala Ala Val Leu Ala Gly Leu Leu Thr Glu Cys Arg Asp Val Leu Leu
        115                 120                 125

Glu Leu Val Glu His His Leu Thr Pro Lys Ser His Gly Arg Ile Arg
    130                 135                 140

His Val Phe Asp His Phe Ser Asp Pro Gly Leu Leu Thr Ala Leu Tyr
145                 150                 155                 160

Gly Pro Asp Phe Thr Gln His Leu Gly Lys Ile Cys Asp Gly Leu Arg
                165                 170                 175

Lys Leu Leu Asp Glu Gly Lys Leu
            180

<210>  17

<211>  901

<212>  DNA

<213>  Homo sapiens


<220>

<221>  CDS

<222>  (28)..(795)


<220>

<221>  sig_peptide

<222>  (28)..(108)


<400>  17
ggaagctgcg gagcccggga cagcgcg atg gcg gag gct cgc ggc gcg ctc ccg      54

Met Ala Glu Ala Arg Gly Ala Leu Pro
1               5

ggc tgg gtc tcg gtg ctg ggc tgg ggc ctg gcg ctg tgc tgt ctg tgc     102
Gly Trp Val Ser Val Leu Gly Trp Gly Leu Ala Leu Cys Cys Leu Cys
10                  15                  20                  25

ggg gcg ggc ccg ctg tgg agt ggc agc cat gag tgg aaa aaa cta att     150
Gly Ala Gly Pro Leu Trp Ser Gly Ser His Glu Trp Lys Lys Leu Ile
                30                  35                  40

ttg acc cag cac tgg ccc ccc aca gta tgc aag gaa gtt aac aga tgt     198
Leu Thr Gln His Trp Pro Pro Thr Val Cys Lys Glu Val Asn Arg Cys
            45                  50                  55

cgg gac tct ctg gat tac tgg aca ata cat gga cta tgg cct gac aga     246
Arg Asp Ser Leu Asp Tyr Trp Thr Ile His Gly Leu Trp Pro Asp Arg
        60                  65                  70

gca gaa gat tgt aac cac tcc tgg cac ttt aac cta aat gag att aag     294
Ala Glu Asp Cys Asn His Ser Trp His Phe Asn Leu Asn Glu Ile Lys
    75                  80                  85

gac ctt ttg cga gac atg aag atc tac tgg cct gat gtg att cac ccg     342
Asp Leu Leu Arg Asp Met Lys Ile Tyr Trp Pro Asp Val Ile His Pro
90                  95                  100                 105

tct tct aat cgc agc caa ttc tgg aaa cat gaa tgg gat aaa cac ggc     390
Ser Ser Asn Arg Ser Gln Phe Trp Lys His Glu Trp Asp Lys His Gly
                110                 115                 120

acc tgt gct gcc cag gtg gat gcc ctc aat tcc gag agg aag tac ttt     438
Thr Cys Ala Ala Gln Val Asp Ala Leu Asn Ser Glu Arg Lys Tyr Phe
            125                 130                 135

ggg aag agc ctg gat cta tac aag cag att gac ctc aac agt gta cta     486
Gly Lys Ser Leu Asp Leu Tyr Lys Gln Ile Asp Leu Asn Ser Val Leu
        140                 145                 150

caa aaa ttt gag atc aag cca tct atc aac tac tac cag ctt gca gat     534
Gln Lys Phe Glu Ile Lys Pro Ser Ile Asn Tyr Tyr Gln Leu Ala Asp
    155                 160                 165

ttc aaa gat gca ctt aca aga atc tat ggt gtg gtg cct aaa atc cag     582
Phe Lys Asp Ala Leu Thr Arg Ile Tyr Gly Val Val Pro Lys Ile Gln
170                 175                 180                 185

tgc ctt atg cca gaa cag gga gag aat gta cag acc att ggg cag ata     630
Cys Leu Met Pro Glu Gln Gly Glu Asn Val Gln Thr Ile Gly Gln Ile
                190                 195                 200

gag ctg tgc ttc acc aag gag gac ttc cat ttg cgg aac tgt act gag     678
Glu Leu Cys Phe Thr Lys Glu Asp Phe His Leu Arg Asn Cys Thr Glu
            205                 210                 215

cca gag gag cag cca tcc tcc ggg cag gaa gcc cgg ctg gcc agg gag     726
Pro Glu Glu Gln Pro Ser Ser Gly Gln Glu Ala Arg Leu Ala Arg Glu
        220                 225                 230

gct tcg acg cat ggg atg atg gtc tgc gaa gac ggc ccg atc ttc tat     774
Ala Ser Thr His Gly Met Met Val Cys Glu Asp Gly Pro Ile Phe Tyr
    235                 240                 245

cct cca ccc aag tcc caa cac tgatgttcac atttggaaa tatttatgt 825
Pro Pro Pro Lys Ser Gln His
250                 255

tccacaaagt aagagaagtt cccaaatcat tgctttctaa aaattcacta aaaagtttct 885

taaaaaaaa aaaaaa 901

<210> 18

<211> 256

<212> PRT

<213> Homo sapiens

<400> 18

Met Ala Glu Ala Arg Gly Ala Leu Pro Gly Trp Val Ser Val Leu Gly
1               5                   10                  15

Trp Gly Leu Ala Leu Cys Cys Leu Cys Gly Ala Gly Pro Leu Trp Ser
            20                  25                  30

Gly Ser His Glu Trp Lys Lys Leu Ile Leu Thr Gln His Trp Pro Pro
        35                  40                  45

Thr Val Cys Lys Glu Val Asn Arg Cys Arg Asp Ser Leu Asp Tyr Trp
    50                  55                  60

Thr Ile His Gly Leu Trp Pro Asp Arg Ala Glu Asp Cys Asn His Ser
65                  70                  75                  80

Trp His Phe Asn Leu Asn Glu Ile Lys Asp Leu Leu Arg Asp Met Lys
                85                  90                  95

Ile Tyr Trp Pro Asp Val Ile His Pro Ser Ser Asn Arg Ser Gln Phe
            100                 105                 110

Trp Lys His Glu Trp Asp Lys His Gly Thr Cys Ala Ala Gln Val Asp
        115                 120                 125

Ala Leu Asn Ser Glu Arg Lys Tyr Phe Gly Lys Ser Leu Asp Leu Tyr
    130                 135                 140

Lys Gln Ile Asp Leu Asn Ser Val Leu Gln Lys Phe Glu Ile Lys Pro
145                 150                 155                 160

Ser Ile Asn Tyr Tyr Gln Leu Ala Asp Phe Lys Asp Ala Leu Thr Arg
                165                 170                 175

Ile Tyr Gly Val Val Pro Lys Ile Gln Cys Leu Met Pro Glu Gln Gly
            180                 185                 190

Glu Asn Val Gln Thr Ile Gly Gln Ile Glu Leu Cys Phe Thr Lys Glu
        195                 200                 205

Asp Phe His Leu Arg Asn Cys Thr Glu Pro Glu Glu Gln Pro Ser Ser
    210                 215                 220

Gly Gln Glu Ala Arg Leu Ala Arg Glu Ala Ser Thr His Gly Met Met
225                 230                 235                 240

Val Cys Glu Asp Gly Pro Ile Phe Tyr Pro Pro Pro Lys Ser Gln His
                245                 250                 255

## Description

### FIELD OF THE INVENTION

The present invention relates to the identification and isolation of novel DNA, therapeutic and drug discovery uses, and the recombinant production of novel secreted polypeptides, designated herein as LP095, LP191, LP217, LP220, LP221, LP222, LP222a, LP229, LP237, and LP238. The present invention also relates to vectors, host cells, and antibodies directed to these polypeptides.

### BACKGROUND OF THE INVENTION

Extracellular proteins play an important role in the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including pharmaceuticals, diagnostics, biosensors and bioreactors. Most protein drugs available at present, such as thrombolytic agents, interferons, interleukins, colony stimulating factors, erythropoietins, and various other cytokines, are secretory proteins. Their receptors, which are membrane proteins, also have potential as therapeutic or diagnostic agents.

### SUMMARY OF THE INVENTION

The present invention provides isolated LP095, LP191, LP217, LP220, LP221, LP222, LP222a, LP229, LP237, or LP238 polypeptide-encoding nucleic acids and the polypeptides encoded thereby, including fragments or specified variants thereof. Contemplated by the present invention are LP probes, primers, recombinant vectors, host cells, transgenic animals, chimeric antibodies and constructs, LP polypeptide antibodies, as well as methods of making and using them diagnostically and therapeutically as described and enabled herein.

The present invention includes isolated nucleic acid molecules comprising polynucleotides that encode LP095, LP191, LP217, LP220, LP221, LP222, LP222a, LP229, LP237, or LP238 polypeptides as defined herein, as well as fragments or specified variants thereof, or isolated nucleic acid molecules that are complementary to polynucleotides that encode such LP polypeptides, fragments or specified variants thereof as defined herein.

A polypeptide of the present invention includes an isolated LP polypeptide comprising at least one fragment, domain, or specified variant of at least 90 to 100% of the contiguous amino acids of at least one portion of SEQ ID NO:2, 4, 6, 8, 10, 12, 14, 16, 18 or 20.

The present invention also provides an isolated LP polypeptide as described herein, wherein the polypeptide further comprises at least one specified substitution, insertion, or deletion, corresponding to portions or specific residues of SEQ ID NO:2, 4, 6, 8, 10, 12, 14, 16, 18 or 20.

The present invention also provides an isolated nucleic acid probe, primer, or fragment, as described herein, wherein the nucleic acid comprises a polynucleotide of at least 10 nucleotides, corresponding or complementary to at least 10 nucleotides of SEQ ID NO:1, 3, 5, 7, 9, 11, 13, 15, 17 or 19.

The present invention also provides compositions, including pharmaceutical compositions, comprising an LP polypeptide, an LP polypeptide-encoding polynucleotide, an LP polynucleotide, or an LP polypeptide antibody, wherein the composition has a measurable effect on an activity associated with a particular LP polypeptide as disclosed herein. A method of treatment or prophylaxis based on an LP polypeptide associated activity, as disclosed herein, can be effected by administration of one or more of the polypeptides, nucleic acids, antibodies, vectors, host cells, transgenic cells, or compositions described herein to a mammal in need of such treatment or prophylactic. Accordingly, the present invention also includes methods for the prophylaxis or treatment of a patho-physiological condition in which at least one cell type involved in said condition is sensitive or responsive to an LP polypeptide, LP polypeptide-encoding polynucleotide, LP nucleic acid, LP polypeptide antibody, host cell, transgenic cell, or composition of the present invention.

The present invention also provides a method for identifying compounds that bind an LP polypeptide, comprising:
(a) admixing at least one isolated LP polypeptide as described herein with a test compound or composition; and
(b) detecting at least one binding interaction between the polypeptide and the compound or composition, optionally further comprising detecting a change in biological activity, such as a reduction or increase.

### DETAILED DESCRIPTION OF THE PREFERRED EMBODIMENTS

Applicants have identified cDNA clones comprising polynucleotides that encode novel polypeptides or novel variants of known polypeptides:

### 1) FEATURES OF POLYPEPTIDES ENCODED BY LP095 POLYNUCLEOTIDES

LP095 polypeptides comprising the amino acid sequence of the open reading frame encoded by the polynucleotide sequence as shown in SEQ ID NO:1 are contemplated by the present invention. Specifically, polypeptides of the present invention comprise the amino acid sequence as shown in SEQ ID NO:2, as well as fragments, variants, and derivatives thereof. Accordingly, LP095 polynucleotides encoding the LP095 polypeptides are also contemplated by the present invention.

LP095 polypeptide shares sequence similarity with 25 kDa trypsin inhibitor (P25TI), a secreted protein which has been purified from human glioblastoma cells [Yamakawa, *et al*., Biochim. Biophys. Acta 1395(2): 202-8 (1998)]. Accordingly, compositions comprising LP095 polypeptides, polynucleotides, and/or antibodies are useful for the diagnosis, treatment, and intervention of cancers, including but not limited to, glioblastoma, neuroblastoma (Yamakawa, *supra*), mucinous ovarian cancer, and non-malignant diseases such as pancreatitis, severe infections, and tissue destruction [Stenman, *et al*., Scand. J. Clin. Lab. Invest. Suppl. 207: 5-9 (1991)].

### 2) FEATURES OF POLYPEPTIDES ENCODED BY LP191 POLYNUCLEOTIDES

In another embodiment, LP191 polypeptides comprising the amino acid sequence of the open reading frame encoded by the polynucleotide sequence as shown in SEQ ID NO:3 are contemplated by the present invention. Specifically, polypeptides of the present invention comprise the amino acid sequence as shown in SEQ ID NO:4, as well as fragments, variants, and derivatives thereof. Accordingly, LP191 polynucleotides encoding the LP191 polypeptides are also contemplated by the present invention.

LP191 polypeptide is an aspartic proteinase. It shares sequence similarity with beta-secretase BACE, a target for Alzheimer's disease [Haniu, *et al*., J. Biol. Chem. 275(28): 21099-106 (2000)]. Accordingly, compositions comprising LP191 polypeptides, polynucleotides, and/or antibodies are useful for the diagnosis, treatment, and intervention of neurological disorders, especially Alzheimer's disease.

### 3) FEATURES OF POLYPEPTIDES ENCODED BY LP217 POLYNUCLEOTIDES

In another embodiment, LP217 polypeptides comprising the amino acid sequence of the open reading frame encoded by the polynucleotide sequence as shown in SEQ ID NO:5 are contemplated by the present invention. Specifically, polypeptides of the present invention comprise the amino acid sequence as shown in SEQ ID NO:6, as well as fragments, variants, and derivatives thereof. Accordingly, LP217 polynucleotides encoding the LP217 polypeptides are also contemplated by the present invention.

The gene encoding the LP217 polypeptide has been localized to the chromosomal location 7p21-p22 (GenBank g3080662). Alteration of this chromosomal region is associated with 7p duplication syndrome [Cai, *et al*., Am. J. Med. Genet. 86(4): 305-11 (1999)], Saethre-Chotzen syndrome [Johnson, *et al*., Am. J. Hum. Genet. 63(5): 1282-93 (1998); Wilkie, *et al*., J. Med. Genet. 32(3): 174-80 (1995)], and craniosynostosis [Motegi, *et al*., Hum. Genet. 71(2): 160-2 (1985)]. LP217 mRNA is expressed in the digestive system, the hemic and immune system, the nervous system, the respiratory system, and the urinary tract. Furthermore, the LP217 polypeptide, as shown in SEQ ID NO:6, contains a thrombospondin type 1 domain and shares sequence similarity with vascular smooth muscle cell growth factor.
Accordingly, compositions comprising LP217 polypeptides, polynucleotides, and/or antibodies are useful for diagnosis, treatment, and intervention of abnormal blood vessel formation, wound healing failure, intractable skin ulcers, diabetic retinopathy, psoriasis, rheumatoid arthritis, angiomatosis, arteriosclerosis, or solid tumors (EP 905 235), 7p duplication syndrome, Saethre-Chotzen syndrome, and craniosynostosis.

### 4) FEATURES OF POLYPEPTIDES ENCODED BY LP220 POLYNUCLEOTIDES

In another embodiment, LP220 polypeptides comprising the amino acid sequence of the open reading frame encoded by the polynucleotide sequence as shown in SEQ ID NO:7 are contemplated by the present invention. Specifically, polypeptides of the present invention comprise the amino acid sequence as shown in SEQ ID NO:8, as well as fragments, variants, and derivatives thereof. Accordingly, LP220 polynucleotides encoding the LP220 polypeptides are also contemplated by the present invention.

Originally, an EST with a partial coding sequence was located at 11q13. The partial sequence was extended to full-length using 5'-RACE, yielding the cDNA encoding LP220 polypeptide. Many human diseases are mapped to chromosome 11q13, including oral squamous cell carcinoma [Shuster, *et al*., Genes Chromosomes Cancer 28(2): 153-63 (2000)], familial somatotropinomas [Gadelha, *et al*., J. Clin. Endocrinol. Metab. 85(2): 707-14 (1999)], Bardet-Beidl syndrome 1 (BBS1) [Katsanis, *et al.,* Am. J. Hum. Gen. 65(6): 1672-9 (1999)], diaphragmatic spinal muscular atrophy [Grohmann, *et al*., Am. J. Hum. Gen. 65(5):1459-62 (1999)], hematological malignancies [Wong, Cancer Genet. Cytogenet. 113(1): 93-5 (1999)], asthma [Adra, *et al*., Clin. Genet. 55(6): 431-7 (1999)], sporadic follicular thyroid tumors [Nord, *et al*., Genes Chromosomes Cancer 26(1): 35-9 (1999)], head and neck cancer [Alavi, *et al*., Laryngoscope 109(6): 874-9 (1999)], pituitary adenomas [Metzger, *et al*., J. Neurosurg. 90(2): 306-14 (1999)], myeloma [Ronchetti, *et al*., Blood 93(4): 1330-7 (1999)], acromegaly/ gigantism (excessive production of growth hormone) [Gadelha, *et al*., J. Clin. Endocrinol. Metab. 84(1): 249-56 (1999)], renal oncocytomas [Fuzesi, *et al*., Cancer Genet. Cytogenet. 107(1): 1-6 (1998)], osteopetrosis [Heaney, *et al*., Hum. Mol. Genet. 7(9): 1407-10 (1998)], congenital fibrosis of the extraocular muscles type 2 [Wang, *et al*., Am. J. Hum. Genet. 63(2): 517-25 (1998)], and diabetes [Nakagawa, *et al*., Am. J. Hum. Genet. 63(2): 547-56 (1998)]. Accordingly, compositions comprising LP220 polypeptides, polynucleotides, or antibodies are useful for diagnosis, treatment, and intervention of the aforementioned diseases associated with this chromosomal location.

### 5) FEATURES OF POLYPEPTIDES ENCODED BY LP221 POLYNUCLEOTIDES

In another embodiment, LP221 polypeptides comprising the amino acid sequence of the open reading frame encoded by the polynucleotide sequence as shown in SEQ ID NO:9 are contemplated by the present invention. Specifically, polypeptides of the present invention comprise the amino acid sequence as shown in SEQ ID NO:10, as well as fragments, variants, and derivatives thereof. Accordingly, LP221 polynucleotides encoding the LP221 polypeptides are also contemplated by the present invention.

The gene encoding LP221 polypeptide has been localized to chromosome 15q21 (GenBank g9802727). Human diseases that have been mapped to 15q21 include, but are not limited to, colorectal adenomas and carcinomas [Park, *et al*., Cancer Res. 60(1): 70-3 (2000)], non-syndromal autosomal recessive sensorineural hearing loss [Campbell, *et al*., J. Med. Genet. 34(12): 1015-7 (1997)], and late infantile neuronal ceroid lipofuscinosis [Sharp, *et al*., Hum. Mol. Genet. 6(4): 591-5 (1997)]. A library, including cDNA that encodes LP221 polypeptide, was constructed using poly A RNA isolated from diseased prostate tissue. Accordingly, compositions comprising LP221 polypeptides, polynucleotides, and/or antibodies are useful for diagnosis, treatment, and intervention of colorectal adenomas and carcinomas, non-syndromal autosomal recessive sensorineural hearing loss, late infantile neuronal ceroid lipofuscinosis, and prostate cancer.

### 6) FEATURES OF POLYPEPTIDES ENCODED BY LP222/222a POLYNUCLEOTIDES

In another embodiment, LP222 and LP222a polypeptides comprising the amino acid sequence of the open reading frame encoded by the polynucleotide sequence as shown in SEQ ID NO:11 are contemplated by the present invention. Specifically, polypeptides of the present invention comprise the amino acid sequence as shown in SEQ ID NO:12, as well as fragments, variants, and derivatives thereof. Accordingly, LP222 and LP222a polynucleotides encoding the LP222 and LP222a polypeptides are also contemplated by the present invention.

The genes encoding LP222 and LP222a polypeptides have been localized to chromosome 20 (GenBank g8218098). LP222 polypeptide, as shown in SEQ ID NO:12, has sequence similarity to insulin-like growth factor binding protein and fibromodulin, both of which are secreted proteins. Accordingly, compositions comprising LP222 and LP222a polypeptides, polynucleotides, and/or antibodies are useful for diagnosis, treatment, and intervention of growth control and carcinogenesis [Grimberg and Cohen, J. Cell. Physiol 183(1): 1-9 (2000)], including, but not limited to, breast cancer, prostate cancer, colorectal cancer, and lung cancer [Giovannucci, Horm Res. 51 Suppl. 3: 34-41 (1999)]. LP222 and LP222a nucleic acid sequence has been localized to the 20p11-13 region of human chromosome number 20. Moreover, the following diseases, conditions, syndromes, disorders, or pathological states have also been mapped to this region of the human genome: Thrombophilia, susceptibility to myocardialinfarction (Maglott, et al. 1996 "Radiation hybrid mapping of SNAP, PCSK2, and THBD (human chromosome 20p)." Mammalian Genome 7: 400-401); posterior polymorphous corneal dystrophy (Heon, et al. 1995 "Linkage of posterior polymorphous corneal dystrophy to 20q11." Hum. Molec. Genet. 4: 485-488); Huntington-like neurodegenerative disorder 1 (Xiang, et al. 1998 "A Huntington disease-like neurodegenerative disorder maps to chromosome 20p." Am. J. Hum. Genet. 63: 1431-1438), atopic dermatitis, (Cookson, et al. 2001 "Genetic linkage of childhood atopic dermatitis to psoriasis susceptibility loci." Nature Genet. 27: 372-373); Hunter-Thompson type acromesomelic dysplasia (Thomas, et al 1996 "A human chondrodysplasia due to a mutation in a TGF-beta superfamily member." Nature Genet. 12: 315-317); Hemolytic anemia (Webb, et al. 1995 "The gene encoding human glutathione synthetase (GSS) maps to the long arm of chromosome 20 at band 11.2." Genomics 30: 617-619); noninsulin-dependent diabetes mellitus (Altshuler, et al. 2000 "The common PPAR-gamma pro12ala polymorphism is associated with decreased risk of type 2 diabetes. Nature Genet. 76-80); susceptibility to obesity/hyperinsulinism (Lembertas, et al. 1997 "Identification of an obesity quantitative trait locus on mouse chromosome 2 and evidence of linkage to body fat and insulin on the human homologous region 20q." J. Clin. Invest. 100: 1240-1247); susceptibility to Graves disease (Tomer, et al. 1998 "A new Graves disease-susceptibility locus maps to chromosome 20q11.2." Am. J. Hum. Genet. 63: 1749-1756; Pearce, 1999 "Further evidence for a susceptibility locus on chromosome 20q13.11 in families with dominant transmission of Graves disease." Am. J. Hum. Genet. 65: 1462-1465); Galactosialidosis (Halal, et al. 1992 "Ring chromosome 20 and possible assignment of the structural gene encoding human carboxypeptidase-L to the distal segment of the long arm of chromosome 20." Am. J. Med. Genet. 43: 576-579); severe combined immunodeficiency due to ADA deficiency or hemolytic anemia due to ADA excess (Hershfield & Mitchell, "Immunodeficiency diseases caused by adenosine deaminase deficiency and purine nucleoside phosphorylase deficiency." In: Scriver, C. R.; Beaudet, A. L.; Sly, W. S.; Valle, D. (eds.) : The Metabolic and Molecular Bases of Inherited Disease. Vol. 2. New York: McGraw-Hill (7th ed.) 1995. Pp. 1725-1768). Accordingly, an isolated and/or recombinant molecule comprising LP369 nucleic acid sequence meets the statutory utility requirement of 35 U.S.C. §101 since such a molecule can be used, for example, to hybridize near a nucleic acid sequence associated with one or more of the above stated diseases, conditions, syndromes, disorders, or pathological states and thus serve as a marker for such a disease gene.

### 7) FEATURES OF POLYPEPTIDES ENCODED BY LP229 POLYNUCLEOTIDES

In another embodiment, LP229 polypeptides comprising the amino acid sequence of the open reading frame encoded by the polynucleotide sequence as shown in SEQ ID NO:13 are contemplated by the present invention. Specifically, polypeptides of the present invention comprise the amino acid sequence as shown in SEQ ID NO:14, as well as fragments, variants, and derivatives thereof. Accordingly, LP229 polynucleotides encoding the LP229 polypeptides are also contemplated by the present invention.

The gene encoding LP229 polypeptide has been localized to chromosome 20q12-13.2 (GenBank g2695870). LP229 polypeptide, as shown in SEQ ID NO:14, has sequence similarity to secretory leukocyte protease inhibitor (SLPI), also located at chromosome 20q12-13.2. SLPI exhibits activity against the human immunodeficiency virus type 1 (HIV-1) in vitro at physiological concentrations [Baqui, *et al*., Clin. Diagn. Lab. Immunol. 6(6): 808-11 (1999)], anti-inflammatory and antibacterial effects [Mulligan, *et al*., Am. J. Pathol. 156(3): 1033-9 (2000), King, *et al*., Mol. Hum. Reprod. 6(2): 191-6 (2000)], protective effects against hepatic ischemia and reperfusion injury [Lentsch, *et al*., Gastroenterology 117(4): 953-61 (1999)], and involvement in the pathogenesis of endometriosis [Suzumori, *et al*., Fertil. Steril. 72(5): 857-67 (1999)]. Accordingly, compositions comprising LP229 polypeptides, polynucleotides, and/or antibodies are useful for diagnosis, treatment, and intervention of the aforementioned disorders.

### 8) FEATURES OF POLYPEPTIDES ENCODED BY LP237 POLYNUCLEOTIDES

In another embodiment, LP237 polypeptides comprising the amino acid sequence of the open reading frame encoded by the polynucleotide sequence as shown in SEQ ID NO:15 are contemplated by the present invention. Specifically, polypeptides of the present invention comprise the amino acid sequence as shown in SEQ ID NO:16, as well as fragments, variants, and derivatives thereof. Accordingly, LP237 polynucleotides encoding the LP237 polypeptides are also contemplated by the present invention.

LP237 polypeptide shares sequence similarity with "human secreted protein encoded by gene 98" (WO 98/39446). The gene encoding LP237 polypeptide is found mainly in the hemic and immune system. Accordingly, compositions comprising LP237 polypeptides, polynucleotides, and/or antibodies are useful for the diagnosis, treatment, and intervention of hemic and immune system diseases and disorders including, but not limited to, atherosclerosis [Horrevoets, *et al*., Blood 93(10): 3418-31 (1999)].

### 9) FEATURES OF POLYPEPTIDES ENCODED BY LP238 POLYNUCLEOTIDES

In another embodiment, LP238 polypeptides comprising the amino acid sequence of the open reading frame encoded by the polynucleotide sequence as shown in SEQ ID NO:17 are contemplated by the present invention. Specifically, polypeptides of the present invention comprise the amino acid sequence as shown in SEQ ID NO:18, as well as fragments, variants, and derivatives thereof. Accordingly, LP238 polynucleotides encoding the LP238 polypeptides are also contemplated by the present invention.

The gene encoding LP238 polypeptide has been localized to chromosome 6. LP238 polypeptide shares sequence similarity with "human secreted protein encoded by gene 47" (WO 99/31117) and secreted Rnase6, a tumor suppressor candidate [Trubia, *et al*., Genomics 42(2): 342-4 (1997)]. The gene encoding LP238 polypeptide is found as a singleton in the liver. Accordingly, compositions comprising LP238 polypeptides, polynucleotides, and/or antibodies are useful for the diagnosis, treatment, and intervention of cell proliferative disorders such as neoplasias and cancers, especially liver cancer.

The polynucleotides of the present invention are designated herein as "LP polynucleotides" or "LP polypeptide-encoding polynucleotides." The polypeptides of the present invention are designated herein as "LP polypeptides." When immediately followed by a numerical designation (e.g., LP095), the term LP refers to a specific group of molecules as defined herein. A complete designation wherein the term "LP" is immediately followed by a numerical designation and a molecule type (e.g., LP095 polypeptide) refers to a specific type of molecule within the designated group of molecules as defined herein.

The terms "LP polypeptide-encoding polynucleotides," "LP polynucleotides," "LP polypeptides" wherein the term is followed by an actual numerical designation as used herein encompass novel polynucleotides and polypeptides, respectively, which are further defined herein. The LP molecules described herein may be isolated from a variety of sources including, but not limited to, human tissue types, or prepared by recombinant or synthetic methods.

One aspect of the present invention provides an isolated nucleic acid molecule comprising a polynucleotide which encodes an LP095, LP191, LP217, LP220, LP221, LP222, LP222a, LP229, LP237, or LP238 polypeptide as defined herein. In a preferred embodiment of the present invention, the isolated nucleic acid comprises 1) a polynucleotide encoding an LP095, LP191, LP217, LP220, LP221, LP222, LP222a, LP229, LP237, or LP238 polypeptide having an amino acid sequence as shown in 2, 4, 6, 8, 10, 12, 14, 16, 18 or 20, respectively, 2) a polynucleotide complementary to such encoding nucleic acid sequences, and which remain stably bound to them under at least moderate, and optionally, high stringency conditions, or 3) any fragment and/or variant of 1) or 2).

The term "LP polypeptide" specifically encompasses truncated or secreted forms of an LP polypeptide (e.g., soluble forms containing, for instance, an extracellular domain sequence), variant forms (e.g., alternatively spliced forms), and allelic variants of an LP polypeptide.

In one embodiment of the invention, the native sequence LP polypeptide is a full-length or mature LP polypeptide comprising amino acids shown in SEQ ID NO:2, 4, 6, 8, 10, 12, 14, 16, 18 and 20. The predicted signal peptides are indicated in the sequence listing of the present application. Also, while the LP polypeptides disclosed herein are shown to begin with a methionine residue designated as amino acid position 1, it is conceivable and possible that another methionine residue located either upstream or downstream from amino acid position 1 may be employed as the starting amino acid residue.

"LP polypeptide variant" is intended to refer to an "active" LP polypeptide, wherein activity is as defined herein, having at least about 90% amino acid sequence identity with an LP polypeptide having the deduced amino acid sequences as shown above. Such LP polypeptide variants include, for instance, LP polypeptides wherein one or more amino acid residues are added, substituted or deleted, at the N- or C-terminus or within the sequence of SEQ ID NO:2, 4, 6, 8, 10, 12, 14, 16, 18 or 20. Ordinarily, an LP polypeptide variant will have at least about 90% amino acid sequence identity, preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity, yet more preferably at least about 99% amino acid sequence identity with the amino acid sequence described, with or without the signal peptide.

"Percent (%) amino acid sequence identity" with respect to the LP amino acid sequences identified herein is defined as the percentage of amino acid residues in a candidate sequence that are identical with the amino acid residues in an LP polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as ALIGN, ALIGN-2, Megalign (DNASTAR) or BLAST (e.g., Blast, Blast-2, WU-Blast-2) software. Those skilled in the art can determine appropriate parameters for measuring alignment, including any algorithms needed to achieve maximal alignment over the full length of the sequences being compared. For example, the % identity values used herein are generated using WU-BLAST-2 [Altschul, *et al*., Methods in Enzymology 266: 460-80 (1996)]. Most of the WU-BLAST-2 search parameters are set to the default values. Those not set to default values, i.e., the adjustable parameters, are set with the following values: overlap span = 1; overlap fraction = 0.125; word threshold (T) = 11; and scoring matrix = BLOSUM 62. For purposes herein, a % amino acid sequence identity value is determined by dividing (a) the number of matching identical amino acid residues between the amino acid sequence of the LP polypeptide of interest and the comparison amino acid sequence of interest (i.e., the sequence against which the LP polypeptide of interest is being compared) as determined by WU-BLAST-2, by (b) the total number of amino acid residues of the LP polypeptide of interest.

"LP variant polynucleotide," "LP127 polynucleotide variant," or "LP variant nucleic acid sequence" is intended to refer to a nucleic acid molecule as defined below having at least about 75% nucleic acid sequence identity with the polynucleotide sequence shown in SEQ ID NO:1, 3, 5, 7, 9, 11, 13, 15, 17 or 19. Ordinarily, an LP polynucleotide variant will have at least about 75% nucleic acid sequence identity, more preferably at least about 80% nucleic acid sequence identity, yet more preferably at least about 81% nucleic acid sequence identity, yet more preferably at least about 82% nucleic acid sequence identity, yet more preferably at least about 83% nucleic acid sequence identity, yet more preferably at least about 84% nucleic acid sequence identity, yet more preferably at least about 85% nucleic acid sequence identity, yet more preferably at least about 86% nucleic acid sequence identity, yet more preferably at least about 87% nucleic acid sequence identity, yet more preferably at least about 88% nucleic acid sequence identity, yet more preferably at least about 89% nucleic acid sequence identity, yet more preferably at least about 90% nucleic acid sequence identity, yet more preferably at least about 91% nucleic acid sequence identity, yet more preferably at least about 92% nucleic acid sequence identity, yet more preferably at least about 93% nucleic acid sequence identity, yet more preferably at least about 94% nucleic acid sequence identity, yet more preferably at least about 95% nucleic acid sequence identity, yet more preferably at least about 96% nucleic acid sequence identity, yet more preferably at least about 97% nucleic acid sequence identity, yet more preferably at least about 98% nucleic acid sequence identity, yet more preferably at least about 99% nucleic acid sequence identity with the nucleic acid sequences shown above. Variants specifically exclude or do not encompass the native nucleotide sequence, as well as those prior art sequences that share 100% identity with the nucleotide sequences of the invention.

"Percent (%) nucleic acid sequence identity" with respect to the LP polynucleotide sequences identified herein is defined as the percentage of nucleotides in a candidate sequence that are identical with the nucleotides in the LP sequence after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as ALIGN, Align-2, Megalign (DNASTAR), or BLAST (e.g., Blast, Blast-2) software. Those skilled in the art can determine appropriate parameters for measuring alignment, including any algorithms needed to achieve maximal alignment over the full length of the sequences being compared. For purposes herein, however, percent nucleic acid identity values are generated using the WU-BLAST-2 (BlastN module) computer program [Altschul, *et al*., Methods in Enzymology 266: 460-80 (1996)]. Most of the WU-BLAST-2 search parameters are set to the default values. Those not set default values, i.e., the adjustable parameters, are set with the following values: overlap span = 1; overlap fraction = 0.125; word threshold (T) = 11; and scoring matrix = BLOSUM62. For purposes herein, a % nucleic acid sequence identity value is determined by dividing (a) the number of matching identical nucleotides between the nucleic acid sequence of the LP polypeptide-encoding nucleic acid molecule of interest and the comparison nucleic acid molecule of interest (i.e., the sequence against which the LP polypeptide-encoding nucleic acid molecule of interest is being compared) as determined by WU-BLAST-2, by (b) the total number of nucleotides of the LP polypeptide-encoding nucleic acid molecule of interest.

In other embodiments, the LP variant polypeptides are nucleic acid molecules which are capable of hybridizing, preferably under stringent hybridization and wash conditions, to nucleotide sequences encoding the full-length LP polypeptide shown in SEQ ID NO:2, 4, 6, 8, 10, 12, 14, 16, 18 or 20. This scope of variant polynucleotides specifically excludes those sequences that are known as of the filing and/or priority dates of the present application.

The term "mature protein" or "mature polypeptide" as used herein refers to the form(s) of the protein produced by expression in a mammalian cell. It is generally hypothesized that once export of a growing protein chain across the rough endoplasmic reticulum has been initiated, proteins secreted by mammalian cells have a signal peptide (SP) sequence which is cleaved from the complete polypeptide to produce a "mature" form of the protein. Oftentimes, cleavage of a secreted protein is not uniform and may result in more than one species of mature protein. The cleavage site of a secreted protein is determined by the primary amino acid sequence of the complete protein and generally cannot be predicted with complete accuracy. Methods for predicting whether a protein has an SP sequence, as well as the cleavage point for that sequence, are available. A cleavage point may exist within the N-terminal domain between amino acid 10 and amino acid 35. More specifically the cleavage point is likely to exist after amino acid 15 but before amino acid 30, more likely after amino acid 27. As one of ordinary skill would appreciate, cleavage sites sometimes vary from organism to organism and cannot be predicted with absolute certainty. Optimally, cleavage sites for a secreted protein are determined experimentally by amino-terminal sequencing of the one or more species of mature proteins found within a purified preparation of the protein.

The term "positives", in the context of sequence comparison performed as described above, includes residues in the sequences compared that are not identical but have similar properties (e.g., as a result of conservative substitutions). The % identity value of positives is determined by the fraction of residues scoring a positive value in the BLOSUM 62 matrix. This value is determined by dividing (a) the number of amino acid residues scoring a positive value in the BLOSUM62 matrix of WU-BLAST-2 between the LP polypeptide amino acid sequence of interest and the comparison amino acid sequence (i.e., the amino acid sequence against which the LP polypeptide sequence is being compared) as determined by WU-BLAST-2, by (b) the total number of amino acid residues of the LP polypeptide of interest.

The term "isolated," when used to describe the various polypeptides disclosed herein, means a polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the LP polypeptide natural environment will not be present. Ordinarily, however, isolated polypeptide will be prepared by at least one purification step.

An "isolated LP polypeptide-encoding nucleic acid" or "isolated LP nucleic acid" is a nucleic acid molecule that is identified and separated from at least one contaminant nucleic acid molecule with which it is ordinarily associated in the natural source of the nucleic acid. Such an isolated nucleic acid molecule is other than in the form or setting in which it is found in nature. Isolated nucleic acid molecules therefore are distinguished from nucleic acid molecule as it exists in natural cells. However, an isolated LP polypeptide-encoding nucleic acid molecule includes LP polypeptide-encoding nucleic acid molecules contained in cells that ordinarily express LP polypeptide where, for example, the nucleic acid molecule is in a chromosomal location different from that of natural cells.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "amino acid" is used herein in its broadest sense, and includes naturally-occurring amino acids as well as non-naturally-occurring amino acids, including amino acid analogs and derivatives. The latter includes molecules containing an amino acid moiety. One skilled in the art will recognize, in view of this broad definition, that reference herein to an amino acid includes, for example, naturally-occurring proteogenic L-amino acids; D-amino acids; chemically modified amino acids such as amino acid analogs and derivatives; naturally-occurring non-proteogenic amino acids such as norleucine, beta-alanine, ornithine, etc.; and chemically synthesized compounds having properties known in the art to be characteristic of amino acids. As used herein, the term "proteogenic" indicates that the amino acid can be incorporated into a peptide, polypeptide, or protein in a cell through a metabolic pathway.

The incorporation of non-natural amino acids, including synthetic non-native amino acids, substituted amino acids, or one or more D-amino acids into the LP peptides, polypeptides, or proteins of the present invention ("D-LP polypeptides") is advantageous in a number of different ways. D-amino acid-containing peptides, etc., exhibit increased stability *in vitro* or *in vivo* compared to L-amino acid-containing counterparts. Thus, the construction of peptides, etc., incorporating D-amino acids can be particularly useful when greater intracellular stability is desired or required. More specifically, D-peptides, etc., are resistant to endogenous peptidases and proteases, thereby providing improved bioavailability of the molecule, and prolonged lifetimes *in vivo* when such properties are desirable. When it is desirable to allow the peptide, etc., to remain active for only a short period of time, the use of L-amino acids therein will permit endogenous peptidases, proteases, etc., in a cell to digest the molecule *in vivo,* thereby limiting the cell's exposure to the molecule. Additionally, D-peptides, etc., cannot be processed efficiently for major histocompatibility complex class II-restricted presentation to T helper cells, and are therefore less likely to induce humoral immune responses in the whole organism.

In addition to using D-amino acids, those of ordinary skill in the art are aware that modifications in the amino acid sequence of a peptide, polypeptide, or protein can result in equivalent, or possibly improved, second generation peptides, etc., that display equivalent or superior functional characteristics when compared to the original amino acid sequences. Alterations in the LP peptides, polypeptides, or proteins of the present invention can include one or more amino acid insertions, deletions, substitutions, truncations, fusions, shuffling of subunit sequences, and the like, either from natural mutations or human manipulation, provided that the sequences produced by such modifications have substantially the same (or improved or reduced, as may be desirable) activity(ies) as the naturally-occurring counterpart sequences disclosed herein.

One factor that can be considered in making such changes is the hydropathic index of amino acids. The importance of the hydropathic amino acid index in conferring interactive biological function on a protein has been discussed by Kyte and Doolittle [J. Mol. Biol. 157: 105-32 (1982)]. It is accepted that the relative hydropathic character of amino acids contributes to the secondary structure of the resultant protein. This, in turn, affects the interaction of the protein with molecules such as enzymes, substrates, receptors, ligands, DNA, antibodies, antigens, etc. Based on its hydrophobicity and charge characteristics, each amino acid has been assigned a hydropathic index as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate/glutamine/aspartate/asparagine (-3.5); lysine (-3.9); and arginine (-4.5).

As is known in the art, certain amino acids in a peptide, polypeptide, or protein can be substituted for other amino acids having a similar hydropathic index or score and produce a resultant peptide, etc., having similar biological activity, i.e., which still retains biological functionality. In making such changes, it is preferable that amino acids having hydropathic indices within ±2 are substituted for one another. More preferred substitutions are those wherein the amino acids have hydropathic indices within ±1. Most preferred substitutions are those wherein the amino acids have hydropathic indices within ±0.5.

Like amino acids can also be substituted on the basis of hydrophilicity. U.S. Patent No. 4,554,101 discloses that the greatest local average hydrophilicity of a protein, as governed by the hydrophilicity of its adjacent amino acids, correlates with a biological property of the protein. The following hydrophilicity values have been assigned to amino acids: arginine/lysine (+3.0); aspartate/glutamate (+3.0±1); serine (+0.3); asparagine/glutamine (+0.2); glycine (0); threonine (-0.4); proline (-0.5±1); alanine/histidine (-0.5); cysteine (-1.0); methionine (-1.3); valine (-1.5); leucine/isoleucine (-1.8); tyrosine (-2.3); phenylalanine (-2.5); and tryptophan (-3.4). Thus, one amino acid in a peptide, polypeptide, or protein can be substituted by another amino acid having a similar hydrophilicity score and still produce a resultant peptide, etc., having similar biological activity, i.e., still retaining correct biological function. In making such changes, amino acids having hydropathic indices within ±2 are preferably substituted for one another, those within ±1 are more preferred, and those within ±0.5 are most preferred.

As outlined above, amino acid substitutions in the LP polypeptides of the present invention can be based on the relative similarity of the amino acid side-chain substituents, for example, their hydrophobicity, hydrophilicity, charge, size, etc. Exemplary substitutions that take various of the foregoing characteristics into consideration in order to produce conservative amino acid changes resulting in silent changes within the present peptides, etc., can be selected from other members of the class to which the naturally-occurring amino acid belongs. Amino acids can be divided into the following four groups: (1) acidic amino acids; (2) basic amino acids; (3) neutral polar amino acids; and (4) neutral non-polar amino acids. Representative amino acids within these various groups include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, cystine, tyrosine, asparagine, and glutamine; and (4) neutral non-polar amino acids such as alanine, leucine, isoleucine, valine, proline, phenylalanine, tryptophan, and methionine.

It should be noted that changes which are not expected to be advantageous can also be useful if these result in the production of functional sequences. Since small peptides, etc., can be easily produced by conventional solid phase synthetic techniques, the present invention includes peptides, etc., such as those discussed herein, containing the amino acid modifications discussed above, alone or in various combinations. To the extent that such modifications can be made while substantially retaining the activity of the peptide, etc., they are included within the scope of the present invention. The utility of such modified peptides, etc., can be determined without undue experimentation by, for example, the methods described herein.

While biologically functional equivalents of the present LP polypeptides can have any number of conservative or non-conservative amino acid changes that do not significantly affect their activity(ies), or that increase or decrease activity as desired, 40, 30, 20, 10, 5, or 3 changes, such as 1-30 changes or any range or value therein, may be preferred. In particular, 10 or fewer amino acid changes may be preferred. More preferably, seven or fewer amino acid changes may be preferred; most preferably, five or fewer amino acid changes may be preferred. The encoding nucleotide sequences (gene, plasmid DNA, cDNA, synthetic DNA, or mRNA, for example) will, thus, have corresponding base substitutions, permitting them to code on expression for the biologically functional equivalent forms of the LP polypeptides. In any case, the LP peptides, polypeptides, or proteins exhibit the same or similar biological or immunological activity(ies) as that(those) of the LP polypeptides specifically disclosed herein, or increased or reduced activity, if desired. The activity(ies) of the variant LP polypeptides can be determined by the methods described herein. Variant LP polypeptides biologically functionally equivalent to those specifically disclosed herein have activity(ies) differing from those of the presently disclosed molecules by about ±50% or less, preferably by about ±40% or less, more preferably by about ±30% or less, more preferably by about ±20% or less, and even more preferably by about ±10% or less, when assayed by the methods disclosed herein.

Amino acids in an LP molecule of the present invention that are essential for activity can be identified by methods known in the art, such as site-directed mutagenesis or alanine-scanning mutagenesis [Cunningham and Wells, Science 244(4908): 1081-5 (1989)]. The latter procedure introduces single alanine mutations at every residue in the molecule. The resulting mutant molecules are then tested for biological activity. Sites that are critical for ligand-protein binding can also be identified by structural analysis such as crystallization, nuclear magnetic resonance, or photoaffinity labeling [Smith, *et al*., J. Mol. Biol. 224(4): 899-904 (1992), and de Vos, *et al*., Science 255(5042): 306-12 (1992)].

"Stringency" of hybridization reactions is readily determinable by one of ordinary skill in the art, and generally is an empirical calculation dependent upon probe length, washing temperature, and salt concentration. In general, longer probes require higher temperatures for proper annealing, while short probes need lower temperatures. Hybridization generally depends on the ability of denatured DNA to re-anneal when complementary strands are present in an environment below their melting temperature. The higher the degree of desired homology between the probe and hybridizable sequence, the higher the relative temperature that can be used. As a result, it follows that higher relative temperatures would tend to make the reactions more stringent, while lower temperatures less so. For additional details and explanation of stringency of hybridization reactions, see Ausubel, *et al*., Current Protocols in Molecular Biology, Wiley Interscience Publishers (1995).

"Stringent conditions" or "high stringency conditions", as defined herein, may be identified by those that
(1) employ low ionic strength and high temperature for washing, for example, 15 mM sodium chloride/1.5 mM sodium citrate/0.1% sodium dodecyl sulfate at 50°C; (2) employ during hybridization a denaturing agent, such as formamide, for example, 50% (v/v) formamide with 0.1% bovine serum albumin/0.1% ficoll/0.1% polyvinylpyrrolidone/50 mM sodium phosphate buffer at pH 6.5 with 750 mM sodium chloride/75 mM sodium citrate at 42°C; or (3) employ 50% formamide, 5X SSC (750 mM sodium chloride, 75 mM sodium citrate), 50 mM sodium phosphate (pH 6.8), 0.1% sodium pyrophosphate, 5X Denhardt's solution, sonicated salmon sperm DNA (50 µg/mL), 0.1% SDS, and 10% dextran sulfate at 42°C with washes at 42°C in 0.2X SSC (30 mM sodium chloride/3 mM sodium citrate) and 50% formamide at 55°C, followed by a high-stringency wash consisting of 0.1X SSC containing EDTA at 55°C.

"Moderately stringent conditions" may be identified as described by Sambrook, *et al*. [Molecular Cloning: A Laboratory Manual, New York: Cold Spring Harbor Press, (1989)], and include the use of washing solution and hybridization conditions (e.g., temperature, ionic strength and %SDS) less stringent than those described above. An example of moderately stringent conditions is overnight incubation at 37°C in a solution comprising: 20% formamide, 5X SSC (750 mM sodium chloride, 75 mM sodium citrate), 50 mM sodium phosphate at pH 7.6, 5X Denhardt's solution, 10% dextran sulfate, and 20 mg/mL denatured sheared salmon sperm DNA, followed by washing the filters in 1X SSC at about 37-50°C. The skilled artisan will recognize how to adjust the temperature, ionic strength, etc., as necessary to accommodate factors such as probe length and the like.

The term "epitope tagged" where used herein refers to a chimeric polypeptide comprising an LP polypeptide, or domain sequence thereof, fused to a "tag polypeptide." The tag polypeptide has enough residues to provide an epitope against which an antibody may be made, or which can be identified by some other agent, yet is short enough such that it does not interfere with the activity of the LP polypeptide. The tag polypeptide preferably is also fairly unique so that the antibody does not substantially cross-react with other epitopes. Suitable tag polypeptides generally have at least six amino acid residues and usually between about 8 to about 50 amino acid residues (preferably, between about 10 to about 20 residues).

As used herein, the term "immunoadhesin," sometimes referred to as an Fc fusion, designates antibody-like molecules that combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous") and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3 or IgG-4 subtypes, IgA (including IgA-1 and IgA-2), IgE, IgD or IgM.

"Active" or "activity" for the purposes herein refers to form(s) of LP which retain the biologic and/or immunologic activities of native or naturally-occurring LP polypeptide. Elaborating further, "biological" activity refers to a biological function (either inhibitory or stimulatory) caused by a native or naturally-occurring LP polypeptide other than the ability to induce the production of an antibody against an antigenic epitope possessed by a native or naturally-occurring LP polypeptide. An "immunological" activity refers only to the ability to induce the production of an antibody against an antigenic epitope possessed by a native or naturally-occurring LP polypeptide. A preferred biological activity includes, for example, the ability to treat uncontrolled cell proliferation, immune response, or abnormal neurological, hematological, or metabolic activity.

"Medical disorder" describes a host of disorders that are characterized principally by uncontrolled cell proliferation, immune response, or abnormal neurological, hematological, or metabolic activity. Exemplary disorders encompassed within this definition include, but are not limited to, cancer, heart disease, pancreatitis, diabetes, Alzheimer's disease, multiple sclerosis, atherosclerosis, rheumatoid arthritis, asthma, and osteopetrosis.

The term "antagonist" is used in the broadest sense and includes any molecule that partially or fully blocks, inhibits, or neutralizes a biological activity of a native LP polypeptide disclosed herein. In a similar manner, the term "agonist" is used in the broadest sense and includes any molecule that mimics a biological activity of a native LP polypeptide disclosed herein. Suitable agonist or antagonist molecules specifically include agonist or antagonist antibodies or antibody fragments, fragments or amino acid sequence variants of native LP polypeptides, peptides, ribozymes, antisense nucleic acids, small organic molecules, etc. Methods for identifying agonists or antagonists of an LP polypeptide may comprise contacting an LP polypeptide with a candidate agonist or antagonist molecule and measuring a detectable change in one or more biological activities normally associated with the LP polypeptide.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (e.g., bispecific antibodies) formed from at least two intact antibodies, and antibody fragments so long as they exhibit the desired biological activity.

The terms "treating," "treatment," and "therapy" as used herein refer to curative therapy, prophylactic therapy, and preventive therapy. An example of "preventive therapy" is the prevention or lessened targeted pathological condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption but, rather, is cyclic in nature.

Administration "in combination with" one or more further therapeutic agents includes simultaneous (concurrent) and consecutive administration in any order.

A "therapeutically-effective amount" is the minimal amount of active agent (e.g., an LP polypeptide, antagonist or agonist thereof) which is necessary to impart therapeutic benefit to a mammal. For example, a "therapeutically-effective amount" to a mammal suffering or prone to suffering or to prevent it from suffering from a medical disorder is such an amount which induces, ameliorates or otherwise causes an improvement in the pathological symptoms, disease progression, physiological conditions associated with or resistance to succumbing to the aforementioned disorder.

"Carriers" as used herein include pharmaceutically-acceptable carriers, excipients, or stabilizers which are nontoxic to the cell or mammal being exposed thereto at the dosages and concentrations employed. Often the physiologically-acceptable carrier is an aqueous pH buffered solution. Examples of physiologically-acceptable carriers include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONIC™.

"Antibody fragments" comprise a portion of an intact antibody, preferably the antigen binding or variable region of the intact antibody. Examples of antibody fragments include Fab, Fab', F(ab')₂ and Fv fragments; diabodies; linear antibodies [Zapata, *et al*., Protein Engin. 8(10): 1057-62 (1995)]; single-chain antibody molecules; and multispecific antibodies formed from antibody fragments.

"Fv" is the minimum antibody fragment which contains a complete antigen-recognition and binding site. This region consists of a dimer of one heavy- and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDR specific for an antigen) has the ability to recognize and bind antigen, although at a lower affinity than the entire binding site.

"Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domain, which enables the sFv to form the desired structure for antigen binding. For a review of sFv, see Pluckthun, The Pharmacology of Monoclonal Antibodies, vol. 113, Rosenburg and Moore, eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404 097, WO 93/11161; and Hollinger, *et al*., Proc. Natl. Acad. Sci. USA 90: 6444-48 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue, or preferably, silver stain. Isolated antibody includes the antibody *in situ* within recombinant cells since at least one component of the antibody's natural environment will not be present. Ordinarily, however, isolated antibody will be prepared by at least one purification step.

An "LP polypeptide antibody" or "LP antibody" refers to an antibody as defined herein that recognizes and binds at least one epitope of an LP polypeptide of the present invention. The term "LP polypeptide antibody" or "LP antibody" wherein the term "LP" is followed by a numerical designation refers to an antibody that recognizes and binds to at least one epitope of that particular LP polypeptide as disclosed herein.

A "liposome" is a small vesicle composed of various types of lipids, phospholipids and/or surfactant which is useful for delivery of a drug (such as an LP polypeptide or antibody thereto) to a mammal. The components of the liposome are commonly arranged in a bilayer formation, similar to the lipid arrangement of biological membranes.

A "small molecule" is defined herein to have a molecular weight below about 500 daltons.

The term "modulate" means to affect (e.g., either upregulate, downregulate or otherwise control) the level of a signaling pathway. Cellular processes under the control of signal transduction include, but are not limited to, transcription of specific genes, normal cellular functions, such as metabolism, proliferation, differentiation, adhesion, apoptosis and survival, as well as abnormal processes, such as transformation, blocking of differentiation, and metastasis.

An LP polypeptide-encoding polynucleotide or similarly an LP polynucleotide can be composed of any polyribonucleotide or polydeoxyribonucleotide, which may be unmodified RNA or DNA or modified RNA or DNA. For example, LP polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, LP polynucleotides can be composed of triple-stranded regions comprising RNA or DNA or both RNA and DNA. LP polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

LP polypeptides can be composed of amino acids joined to each other by peptide bonds or modified peptide bonds, i.e., peptide isosteres, and may contain amino acids other than the gene-encoded amino acids. The LP polypeptides may be modified by either natural processes, such as post-translational processing, or by chemical modification techniques which are well known in the art. Such modifications are well described in basic texts and in more detailed monographs, as well as in a voluminous research literature. Modifications can occur anywhere in the LP polypeptides, including the peptide backbone, the amino acid side-chains and the amino or carboxyl termini. It will be appreciated that the same type of modification may be present in the same or varying degrees at several sites in a given LP polypeptide. Also, a given LP polypeptide may contain many types of modifications. LP polypeptides may be branched, for example, as a result of ubiquitination, and they may be cyclic, with or without branching. Cyclic, branched, and branched cyclic LP polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. See, for instance, Creighton, Proteins - Structure and Molecular Properties, 2nd Ed., W. H. Freeman and Company, New York (1993); Johnson, Post-transational Covalent Modification of Proteins, Academic Press, New York, pp. 1-12 (1983); Seifter, *et al*., Meth. Enzymol. 182: 626-46 (1990); Rattan, *et al*., Ann. NY Acad. Sci. 663: 48-62 (1992).

Variations in the full-length sequence LP or in various domains of the LP polypeptide described herein can be made, for example, using any of the techniques and guidelines for conservative and non-conservative mutations set forth, for instance, in U.S. Patent No. 5,364,934. Variations may be a substitution, deletion or insertion of one or more codons encoding LP polypeptide that results in a change in the amino acid sequence of the LP polypeptide as compared with the native sequence LP polypeptide or an LP polypeptide as disclosed herein. Optionally, the variation is by substitution of at least one amino acid with any other amino acid in one or more of the domains of the LP polypeptide. Guidance in determining which amino acid residue may be inserted, substituted or deleted without adversely affecting the desired activity may be found by comparing the sequence of the LP polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of 1 to 5 amino acids. The variation allowed may be determined by systematically making insertions, deletions or substitutions of amino acids in the sequence and testing the resulting variants for activity (such as in any of the *in vitro* assays described herein) for activity exhibited by the full-length or mature native polypeptide sequence.

LP polypeptide fragments are also provided herein. Such fragments may be truncated at the N-terminus or C-terminus, or may lack internal residues, for example, when compared with a full-length or native protein. Certain fragments contemplated by the present invention may lack amino acid residues that are not essential for a desired biological activity of the LP polypeptide.

LP polypeptide fragments may be prepared by any of a number of conventional techniques. Desired peptide fragments may be chemically synthesized. An alternative approach involves generating LP fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment by polymerase chain reaction (PCR). Oligonucleotides that define the desired termini of the DNA fragment are employed at the 5' and 3' primers in the PCR. Preferably, LP polypeptide fragments share at least one biological and/or immunological activity with at least one of the LP polypeptides as shown in SEQ ID NO:2, 4, 6, 8, 10, 12, 14, 16, 18 or 20.

Covalent modifications of LP polypeptides are included within the scope of this invention. One type of covalent modification includes reacting targeted amino acid residues of an LP polypeptide with an organic derivatizing agent that is capable of reacting with selected side chains or the N- or C-terminal residues of an LP polypeptide. Derivatization with bifunctional agents is useful, for instance, for crosslinking LP to a water-insoluble support matrix or surface for use in the method for purifying anti-LP polypeptide antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis-(succinimidylpropionate), bifunctional maleimides such as bis-N-maleimido-1,8-octane and agents such as methyl-3-[(p-azidophenyl)dithio]-propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the alpha-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the LP polypeptides included within the scope of this invention comprises altering the native glycosylation pattern of the polypeptide. "Altering the native glycosylation pattern" is intended for purposes herein to mean deleting one or more carbohydrate moieties found in native sequence LP polypeptide and/or adding one or more glycosylation sites that are not present in the native sequence LP polypeptide. Additionally, the phrase includes qualitative changes in the glycosylation of the native proteins, involving a change in the nature and proportions of the various carbohydrate moieties present.

Addition of glycosylation sites to LP polypeptides may be accomplished by altering the amino acid sequence thereof. The alteration may be made, for example, by the addition of, or substitution by, one or more serine or threonine residues to the native sequence LP polypeptide (for O-linked glycosylation sites). The LP amino acid sequences may optionally be altered through changes at the DNA level, particularly by mutating the DNA encoding the LP polypeptides at preselected bases such that codons are generated that will translate into the desired amino acids.

Another means of increasing the number of carbohydrate moieties on the LP polypeptides is by chemical or enzymatic coupling of glycosides to the polypeptide. Such methods are described in the art, e.g., in WO 87/05330, published 11 September 1987, and in Aplin and Wriston, CRC Crit. Rev. Biochem., pp. 259-306 (1981).

Removal of carbohydrate moieties present on the LP polypeptide may be accomplished chemically or enzymatically or by mutational substitution of codons encoding for amino acid residues that serve as targets for glycosylation. Chemical deglycosylation techniques are known in the art and described, for instance, by Sojar, *et al*., Arch. Biochem. Biophys. 259: 52-7 (1987) and by Edge, *et al*., Anal. Biochem. 118: 131-7 (1981). Enzymatic cleavage of carbohydrate moieties on polypeptides can be achieved by the use of a variety of endo- and exo-glycosidases as described by Thotakura, *et al*., Meth. Enzymol. 138: 350-9 (1987).

Another type of covalent modification of LP comprises linking any one of the LP polypeptides to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192; or 4,179,337.

LP polypeptides of the present invention may also be modified in a way to form chimeric molecules comprising an LP polypeptide fused to another heterologous polypeptide or amino acid sequence. In one embodiment, such a chimeric molecule comprises a fusion of an LP polypeptide with a tag polypeptide which provides an epitope to which an anti-tag antibody can selectively bind. The epitope tag is generally placed at the amino- or carboxyl-terminus of the LP polypeptide. The presence of such epitope-tagged forms of an LP polypeptide can be detected using an antibody against the tag polypeptide. Also, provision of the epitope tag enables an LP polypeptide to be readily purified by affinity purification using an anti-tag antibody or another type of affinity matrix that binds to the epitope tag.

In an alternative embodiment, the chimeric molecule may comprise a fusion of an LP polypeptide with an immunoglobulin or a particular region of an immunoglobulin. For a bivalent form of the chimeric molecule, such a fusion could be to the Fc region of an IgG molecule. The Ig fusions preferably include the substitution of a soluble transmembrane domain deleted or inactivated form of an LP polypeptide in place of at least one variable region within an Ig molecule. In a particularly preferred embodiment, the immunoglobulin fusion includes the hinge, CH2, and CH3 or the hinge, CH1, CH2, and CH3 regions of an IgG1 molecule. For the production of immunoglobulin fusions, see also U.S. Patent 5,428,130.

In yet a further embodiment, the LP polypeptides of the present invention may also be modified in a way to form a chimeric molecule comprising an LP polypeptide fused to a leucine zipper. Various leucine zipper polypeptides have been described in the art. See, e.g., Landschulz, *et al*., Science 240(4860): 1759-64 (1988); WO 94/10308; Hoppe, *et al*., FEES Letters 344(2-3): 191-5 (1994); Abel, *et al*., Nature 341(6237): 24-5 (1989). It is believed that use of a leucine zipper fused to an LP polypeptide may be desirable to assist in dimerizing or trimerizing soluble LP polypeptide in solution. Those skilled in the art will appreciate that the zipper may be fused at either the N- or C-terminal end of the LP molecule.

The description below relates primarily to production of LP polypeptides by culturing cells transformed or transfected with a vector containing an LP polypeptide-encoding nucleic acid. It is, of course, contemplated that alternative methods, which are well known in the art, may be employed to prepare LP polypeptides. For instance, the LP polypeptide sequence, or portions thereof, may be produced by direct peptide synthesis using solid-phase techniques [see, e.g., Stewart, *et al*., Solid-Phase Peptide Synthesis, W.H. Freeman Co., San Francisco, CA (1969); Merrifield, J. Am. Chem. Soc. 85: 2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of an LP polypeptide may be chemically synthesized separately and combined using chemical or enzymatic methods to produce a full-length LP polypeptide.

DNA encoding an LP polypeptide may be obtained from a cDNA library prepared from tissue believed to possess the LP polypeptide-encoding mRNA and to express it at a detectable level. Libraries can be screened with probes (such as antibodies to an LP polypeptide or oligonucleotides of at least about 20-80 bases) designed to identify the gene of interest or the protein encoded by it. Screening the cDNA or genomic library with the selected probe may be conducted using standard procedures, such as described in Sambrook, *et al*., Molecular Cloning: A Laboratory Manual, Cold Spring Harbor Laboratory Press, NY (1989). An alternative means to isolate the gene encoding an LP polypeptide is to use PCR methodology [Sambrook, *et al*., *supra*; Dieffenbach, *et al*., PCR Primer: A Laboratory Manual, Cold Spring Harbor Laboratory Press, NY (1995)].

Nucleic acids encoding LP polypeptides may be obtained by screening selected cDNA or genomic libraries using the deduced amino acid sequence disclosed herein for the first time and, if necessary, using conventional primer extension procedures as described in Sambrook, *et al*., *supra,* to detect precursors and processing intermediates of mRNA that may not have been reverse-transcribed into cDNA.

Host cells are transfected or transformed with expression or cloning vectors described herein for LP polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: A Practical Approach, M. Butler, ed. (IRL Press, 1991) and Sambrook, *et al*., *supra.*

Methods of transfection are known to the ordinarily skilled artisan, for example, CaPO₄ and electroporation. General aspects of mammalian cell host system transformations have been described in U.S. Patent No. 4,399,216. Transformations into yeast are typically carried out according to the method of van Solingen, *et al*., J Bact. 130(2): 946-7 (1977) and Hsiao, *et al*., Proc. Natl. Acad. Sci. USA 76(8): 3829-33 (1979). However, other methods for introducing DNA into cells, such as by nuclear microinjection, electroporation, bacterial protoplast fusion with intact cells, or polycations, e.g., polybrene or polyornithine, may also be used. For various techniques for transforming mammalian cells, see Keown, *et al*., Methods in Enzymology 185: 527-37 (1990) and Mansour, *et al*., Nature 336(6197): 348-52 (1988).

Suitable host cells for cloning or expressing the nucleic acid (e.g., DNA) in the vectors herein include prokaryote, yeast, or higher eukaryote cells. Suitable prokaryotes include but are not limited to eubacteria, such as Gram-negative or Gram-positive organisms, for example, Enterobacteriacea such as *E*. *coli.* Various *E*. *coli* strains are publicly available, such as *E*. *coli* K12 strain MM294 (ATCC 31,446); *E. coli* strain X1776 (ATCC 31,537); *E*. *coli* strain W3110 (ATCC 27,325) and K5 772 (ATCC 53,635). Other suitable prokaryotic host cells include Enterobacteriaceae such as Escherichia, e.g., Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41P disclosed in DD 266,710, published 12 April 1989), Pseudomonas such as P. aeruginosa, and Streptomyces. These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3 110 may be modified to effect a genetic mutation in a gene encoding proteins endogenous to the host, with examples of such hosts including *E*. *coli* W3110 strain 1A2, which has the complete genotype tonAD; *E*. *coli* W3110 strain 9E4, which has the complete genotype tonAD ptr3; *E*. *coli* W3110 strain 27C7 (ATCC 55,244), which has the complete genotype tonAD ptr3 phoADE15 D(argF-lac)169 ompTD degP4lkan^{R}'; *E*. *coli* W3110 strain 37D6, which has the complete genotype tonAD ptr3 phoADE15 D(argF-lac)169 ompTD degP41kan^{R} rbs7D ilvG; *E*. *coli* W3110 strain 40B4, which is strain 37D6 with a non-kanamycin resistant degP deletion mutation; and an *E*. *coli* strain having mutant periplasmic protease as disclosed in U.S. Patent No. 4,946,783 issued 7 August 1990. Alternatively, *in vivo* methods of cloning, e.g., PCR or other nucleic acid polymerase reactions, are suitable.

In addition to prokaryotes, eukaryotic microbes such as filamentous fungi or yeast are suitable cloning or expression hosts for LP vectors. Saccharomyces cerevisiae is a commonly used lower eukaryotic host microorganism. Others include Schizosaccharomyces pombe [Beach and Nurse, Nature 290: 140-3 (1981); EP 139,383 published 2 May 1995]; Muyveromyces hosts [U.S. Patent No. 4,943,529; Fleer, *et al.*, Bio/Technology 9(10): 968-75 (1991)] such as, e.g., K. lactis (MW98-8C, CBS683, CBS4574) [de Louvencourt, *et al*., J. Bacteriol. 154(2): 737-42 (1983)]; K. fiagilis (ATCC 12,424), K. bulgaricus (ATCC 16,045), K. wickeramii (ATCC 24,178), K. waltii (ATCC 56,500), K. drosophilarum (ATCC 36,906) [Van den Berg, *et al*., Bio/Technology 8(2): 135-9 (1990)]; K. thermotolerans, and K. marxianus; yarrowia (EP 402,226); Pichia pastoris (EP 183,070) [Sreekrishna, *et al*., J. Basic Microbiol. 28(4): 265-78 (1988)]; Candida; Trichoderma reesia (EP 244,234); Neurospora crassa [Case, *et al*., Proc. Natl. Acad Sci. USA 76(10): 5259-63 (1979)]; Schwanniomyces such as Schwanniomyces occidentulis (EP 394,538 published 31 October 1990); and filamentous fungi such as, e.g., Neurospora, Penicillium, Tolypocladium (WO 91/00357 published 10 January 1991), and Aspergillus hosts such as A. nidulans [Ballance, *et al*., Biochem. Biophys. Res. Comm. 112(1): 284-9 (1983)]; Tilburn, *et al*., Gene 26(2-3): 205-21 (1983); Yelton, *et al.,* Proc. Natl. Acad. Sci. USA 81(5): 1470-4 (1984)] and A. niger [Kelly and Hynes, EMBO J. 4(2): 475-9 (1985)]. Methylotropic yeasts are selected from the genera consisting of Hansenula, Candida, Kloeckera, Pichia, Saccharomyces, Torulopsis, and Rhodotoruia. A list of specific species that are exemplary of this class of yeast may be found in C. Antony, The Biochemistry of Methylotrophs 269 (1982).

Suitable host cells for the expression of glycosylated LP polypeptides are derived from multicellular organisms. Examples of invertebrate cells include insect cells such as Drosophila S2 and Spodoptera Sp, Spodoptera High5 as well as plant cells. Examples of useful mammalian host cell lines include Chinese hamster ovary (CHO) and COS cells. More specific examples include monkey kidney CV-1 line transformed by SV40 (COS-7, ATCC CRL 1651); human embryonic kidney line [293 or 293 cells subcloned for growth in suspension culture, Graham, *et al*., J. Gen Virol., 36(1): 59-74 (1977)]; Chinese hamster ovary cells/-DHFR [CHO, Urlaub and Chasin, Proc. Natl. Acad. Sci. USA, 77(7): 4216-20 (1980)]; mouse sertoli cells [TM4, Mather, Biol. Reprod. 23(1):243-52 (1980)]; human lung cells (W138, ATCC CCL 75); human liver cells (Hep G2, HB 8065); and mouse mammary tumor (MMT 060562, ATCC CCL 51). The selection of the appropriate host cell is deemed to be within the skill in the art.

LP polypeptides may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the LP polypeptide-encoding DNA that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, e.g., the yeast invertase leader, alpha factor leader (including Saccharomyces and Kluyveromyces cc-factor leaders, the latter described in U.S. Patent No. 5,010,182), or acid phosphatase leader, the C. albicans glucoamylase leader (EP 362,179), or the signal described in WO 90/13646. In mammalian cell expression, mammalian signal sequences may be used to direct secretion of the protein, such as signal sequences from secreted polypeptides of the same or related species as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for Bacilli.

An example of suitable selectable markers for mammalian cells are those that enable the identification of cells competent to take up the LP polypeptide-encoding nucleic acid, such as DHFR or thymidine kinase. An appropriate host cell when wild-type DHFR is employed is the CHO cell line deficient in DHFR activity, prepared and propagated as described by Urlaub and Chasin, Proc. Natl. Acad. Sci. USA, 77(7): 4216-20 (1980). A suitable selection gene for use in yeast is the trp 1 gene present in the yeast plasmid YRp7 [Stinchcomb, *et al*., Nature 282(5734): 39-43 (1979); Kingsman, *et al.,* Gene 7(2): 141-52 (1979); Tschumper, *et al*., Gene 10(2): 157-66 (1980)]. The trp 1 gene provides a selection marker for a mutant strain of yeast lacking the ability to grow in tryptophan, for example, ATCC No. 44076 or PEP4-1 [Jones, Genetics 85: 23-33 (1977)].

Expression and cloning vectors usually contain a promoter operably linked to the LP polypeptide-encoding nucleic acid sequence to direct mRNA synthesis. Promoters recognized by a variety of potential host cells are well known. Promoters suitable for use with prokaryotic hosts include the beta-lactamase and lactose promoter systems [Chang, *et al*., Nature 275(5681): 617-24 (1978); Goeddel, *et al*., Nature 281(5732): 544-8 (1979)], alkaline phosphatase, a tryptophan (up) promoter system [Goeddel, Nucleic Acids Res. 8(18): 4057-74 (1980); EP 36,776 published 30 September 1981], and hybrid promoters such as the tat promoter [de Boer, *et al*., Proc. Natl. Acad. Sci. USA 80(1): 21-5 (1983)]. Promoters for use in bacterial systems also will contain a Shine-Dalgarno (S.D.) sequence operably linked to the DNA encoding the LP polypeptide.

Examples of suitable promoting sequences for use with yeast hosts include the promoters for 3-phosphoglycerate kinase [Hitzeman, *et al*., J. Biol. Chem. 255(24): 12073-80 (1980)] or other glycolytic enzymes [Hess, *et al*., J. Adv. Enzyme Reg. 7: 149 (1968); Holland, Biochemistry 17(23): 4900-7 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Transcription of LP polypeptide-encoding mRNA from vectors in mammalian host cells may be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a polynucleotide encoding an LP polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, alpha-ketoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the LP polypeptide coding sequence but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and occasionally 3' untranslated regions of eukaryotic or viral DNAs or cDNAs. These regions contain nucleotide segments transcribed as polyadenylated fragments in the untranslated portion of the mRNA encoding LP polypeptide.

Gene amplification and/or expression may be measured in a sample directly, for example, by conventional Southern blotting, Northern blotting to quantitate the transcription of mRNA [Thomas, Proc. Natl. Acad. Sci. USA 77(9): 5201-5 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal and may be prepared in any mammal. Conveniently, the antibodies may be prepared against a native sequence provided herein or against exogenous sequence fused to an LP-encoding DNA and encoding a specific antibody epitope.

Various forms of an LP polypeptide may be recovered from culture medium or from host cell lysates. If membrane-bound, it can be released from the membrane using a suitable detergent solution (e.g., Triton-X 100) or by enzymatic cleavage. Cells employed in expression of an LP polypeptide can be disrupted by various physical or chemical means, such as freeze-thaw cycling, sonication, mechanical disruption, or cell lysing agents.

It may be desirable to purify LP polypeptides from recombinant cell proteins or polypeptides. The following procedures are exemplary of suitable purification procedures: by fractionation on an ion-exchange column; ethanol precipitation; reversed-phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of an LP polypeptide. Various methods of protein purification may be employed and such methods are known in the art and described, for example, in Deutscher, Methods in Enzymology 182: 83-9 (1990) and Scopes, Protein Purification: Principles and Practice, Springer-Verlag, NY (1982). The purification step(s) selected will depend, for example, on the nature of the production process used and the particular LP polypeptide produced.

Nucleotide sequences (or their complement) encoding LP polypeptides have various applications in the art of molecular biology, including uses as hybridization probes, in chromosome and gene mapping and in the generation of antisense RNA and DNA. LP polypeptide-encoding nucleic acids will also be useful for the preparation of LP polypeptides by the recombinant techniques described herein.

The full-length LP polypeptide-encoding nucleotide sequence (SEQ ID NO:1, 3, 5, 7, 9, 11, 13, 15, 17 or 19), or portions thereof, may be useful as hybridization probes for probing a cDNA or genomic library to isolate the full-length LP polypeptide-encoding cDNA or genomic sequences including promoters, enhancer elements and introns of native sequence LP polypeptide-encoding DNA or to isolate still other genes (for instance, those encoding naturally-occurring variants of LP polypeptides, or the same from other species) which have a desired sequence identity to the LP polypeptide-encoding nucleotide sequence disclosed in SEQ ID NO:1, 3, 5, 7, 9, 11, 13, 15, 17 or 19. Hybridization techniques are well known in the art, some of which are described in further detail in the Examples below.

Other useful fragments of the LP polypeptide-encoding nucleic acids include antisense or sense oligonucleotides comprising a single-stranded nucleic acid sequence (either RNA or DNA) capable of binding to target LP polypeptide-encoding mRNA (sense) of LP polypeptide-encoding DNA (antisense) sequences. Antisense or sense oligonucleotides, according to the present invention, comprise a fragment of the coding region of LP polypeptide-encoding DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen, Cancer Res. 48(10): 2659-68 (1988) and Van der Krol, *et al.,* Bio/Techniques 6(10): 958-76 (1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of LP mRNA and any LP polypeptide encoded thereby. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10448, and other moieties that increase affinity of the oligonucleotide for a target nucleic acid sequence, such poly-L-lysine. Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo*. Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

When the amino acid sequence for an LP polypeptide suggests to one skilled in the art that the polypeptide may bind to another protein (for example, where the LP polypeptide functions as a receptor), the LP polypeptide can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, a receptor LP polypeptide can be used to isolate correlative ligand(s). Screening assays can be designed to find lead compounds that mimic the biological activity of the LP polypeptides disclosed herein or a receptor for such LP polypeptides. Typical screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode an LP polypeptide of the present invention or any of its modified forms can also be used to generate either transgenic animals or "knock out" animals which, in turn, are useful in the development and screening of therapeutically useful reagents. Methods for generating transgenic animals, particularly animals such as mice or rats, have become conventional in the art and are described, for example, in U.S. Patent Nos. 4,736,866 and 4,870,009. Typically, particular cells would be targeted for an LP transgene incorporation with tissue-specific enhancers. Transgenic animals that include a copy of a transgene introduced into the germ line of the animal at an embryonic stage can be used to examine the effect of increased expression of DNA encoding an LP polypeptide. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of LP can be used to construct a "knock out" animal which has a defective or altered gene encoding a particular LP polypeptide as a result of homologous recombination between the endogenous gene encoding the LP polypeptide and the altered genomic DNA introduced into an embryonic cell of the animal. For example, cDNA encoding an LP polypeptide can be used to clone genomic DNA encoding that LP polypeptide in accordance with established techniques. A portion of the genomic DNA encoding an LP polypeptide can be deleted or replaced with another gene, such as a gene encoding a selectable marker which can be used to monitor integration. Typically, several kilobases of unaltered flanking DNA (both at the 5' and 3' ends) are included in the vector [see, e.g., Thomas and Capecchi, Cell 51(3): 503-12 (1987) for a description of homologous recombination vectors]. The vector is introduced into an embryonic stem cell line (e.g., by electroporation), and cells in which the introduced DNA has homologously recombined with the endogenous DNA are selected [see, e.g., Li, *et al*., Cell 69(6): 915-26 (1992)]. The selected cells are then injected into a blastocyst of an animal (e.g., a mouse or rat) to form aggregation chimeras [see, e.g., Bradley, Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized, for instance, for their ability to defend against certain pathological conditions and for their development of pathological conditions due to absence of the native LP polypeptide.

Transgenic non-human mammals are useful as an animal models in both basic research and drug development endeavors. Transgenic animals expressing at least one LP polypeptide or nucleic acid can be used to test compounds or other treatment modalities which may prevent, suppress, or cure a pathology or disease associated with at least one of the above mentioned activities. Such transgenic animals can also serve as a model for the testing of diagnostic methods for those same diseases. Furthermore, tissues derived from such transgenic non-human mammals are useful as a source of cells for cell culture in efforts to develop *in vitro* bioassays to identify compounds that modulate LP polypeptide activity or LP polypeptide dependent signaling.
Accordingly, another aspect of the present invention contemplates a method of identifying compounds efficacious in the treatment of at least one previously described disease or pathology associated with an LP polypeptide associated activity. A non-limiting example of such a method comprises:
a) generating a transgenic non-human animal which expresses an LP polypeptide of the present invention and which is, as compared to a wild-type animal, pathologically distinct in some detectable or measurable manner from wild-type version of said non-human mammal;
b) exposing said transgenic animal to a compound, and;
c) determining the progression of the pathology in the treated transgenic animal, wherein an arrest, delay, or reversal in disease progression in transgenic animal treated with said compound as compared to the progression of the pathology in an untreated control animals is indicative that the compound is useful for the treatment of said pathology.

Another embodiment of the present invention provides a method of identifying compounds capable of inhibiting LP polypeptide activity *in vivo* and/or *in vitro* wherein said method comprises:
a) administering an experimental compound to an LP polypeptide expressing transgenic non-human animal, or tissues derived therefrom, exhibiting one or more physiological or pathological conditions attributable to the expression of an LP transgene; and
b) observing or assaying said animal and/or animal tissues to detect changes in said physiological or pathological condition or conditions.

Another embodiment of the invention provides a method for identifying compounds capable of overcoming deficiencies in LP polypeptide activity *in vivo* or *in vitro* wherein said method comprises:
a) administering an experimental compound to an LP polypeptide expressing transgenic non-human animal, or tissues derived therefrom, exhibiting one or more physiological or pathological conditions attributable to the disruption of the endogenous LP g polypeptide-encoding gene; and
b) observing or assaying said animal and/or animal tissues to detect changes in said physiological or pathological condition or conditions.

Various means for determining a compound's ability to modulate the activity of an LP polypeptide in the body of the transgenic animal are consistent with the invention. Observing the reversal of a pathological condition in the LP polypeptide expressing transgenic animal after administering a compound is one such means. Another more preferred means is to assay for markers of LP activity in the blood of a transgenic animal before and after administering an experimental compound to the animal. The level of skill of an artisan in the relevant arts readily provides the practitioner with numerous methods for assaying physiological changes related to therapeutic modulation of LP activity.

"Gene therapy" includes both conventional gene therapy, where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane [Zamecnik, *et al.,* Proc. Natl. Acad Sci. USA 83(12): 4143-6 (1986)]. The oligonucleotides can be modified to enhance their uptake, e.g., by substituting their negatively charged phosphodiester groups with uncharged groups.

There are a variety of techniques available for introducing nucleic acids into viable cells. The techniques vary depending upon whether the nucleic acid is transferred into cultured cells *in vitro* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically, retroviral) vectors and viral coat protein-liposome mediated transfection [Dzau, *et al*., Trends in Biotechnology 11(5): 205-10 (19.93)]. In some situations it is desirable to provide the nucleic acid source with an agent that targets the target cells, such as an antibody specific for a cell surface membrane protein or the target cell, a ligand for a receptor on the target cells, etc. Where liposomes are employed, proteins which bind to a cell surface membrane protein associated with endocytosis may by used for targeting and/or to facilitate uptake, e.g., capsid proteins or fragments thereof tropic for a particular cell type, antibodies for proteins which undergo internalization in cycling, proteins that target intracellular localization and enhance intracellular half-life. The technique of receptor-mediated endocytosis is described, for example by Wu, *et al*., J. Biol. Chem. 262(10): 4429-32 (1987); and Wagner, *et al*., Proc. Natl. Acad. Sci. USA 87(9): 3410-4 (1990). For a review of gene marking and gene therapy protocols, see Anderson, Science 256(5058): 808-13 (1992).

The nucleic acid molecules encoding LP polypeptides or fragments thereof described herein are useful for chromosome identification. In this regard, there exists an ongoing need to identify new chromosome markers, since relatively few chromosome marking reagents, based upon actual sequence data, are presently available. Each LP polypeptide-encoding nucleic acid molecule of the present invention can be used as a chromosome marker. An LP polypeptide-encoding nucleic acid or fragments thereof can also be used for chromosomal localization of the gene encoding that LP polypeptide.

The present invention further provides anti-LP polypeptide antibodies. Exemplary antibodies include polyclonal, monoclonal, humanized, bispecific, and heteroconjugate antibodies.

The anti-LP polypeptide antibodies of the present invention may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the LP polypeptide or a fusion protein thereof. It may be useful to conjugate the immunizing agent to a protein known to be immunogenic in the mammal being immunized. Examples of such immunogenic proteins include, but are not limited to, keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

Anti-LP polypeptide antibodies may, alternatively, be monoclonal antibodies. Monoclonal antibodies may be prepared using hybridoma methods, such as those described by Kohler and Milstein, Nature 256(5517): 495-7 (1975). In a hybridoma method, a mouse, hamster, or other appropriate host animal is typically immunized with an immunizing agent to elicit lymphocytes that produce or are capable of producing antibodies that will specifically bind to the immunizing agent. Alternatively, the lymphocytes may be immunized *in vitro*.

The immunizing agent will typically include an LP polypeptide or a fusion protein thereof. Generally, either peripheral blood lymphocytes ("PBLs") are used, if cells of human origin are desired, or spleen cells or lymph node cells are used, if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California, and the American Type Culture Collection, Rockville, Maryland. Human myeloma and mouse-human heteromyeloma cell lines also have been described for the production of human monoclonal antibodies [Kozbor, J. Immunol. 133(6): 3001-5 (1984); Brodeur, *et al.,* Monoclonal Antibody Production Techniques and Applications, Marcel Dekker, Inc., NY (1987) pp. 51-63].

The culture medium in which the hybridoma cells are cultured can then be assayed for the presence of monoclonal antibodies directed against an LP polypeptide. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Rodbard, Anal. Biochem. 107(1): 220-39 (1980).

After the desired hybridoma cells are identified, the clones may be subcloned by limiting dilution procedures and grown by standard methods [Goding, Monoclonal Antibodies: Principles and Practice, Academic Press, (1986) pp. 59-103].. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison, *et al*., Proc. Natl. Acad. Sci. USA 81(21): 6851-5 (1984)] or by covalently joining to the immunoglobulin coding sequence all or part of the coding sequence for a non-immunoglobulin polypeptide. Such a non-immunoglobulin polypeptide can be substituted for the constant domains of an antibody of the invention or can be substituted for the variable domains of one antigen-combining site of an antibody of the invention to create a chimeric bivalent antibody.

Anti-LP polypeptide antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using routine techniques known in the art.

The anti-LP polypeptide antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary-determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin, and all or substantially all of the FR regions are those of a human immunoglobulin consensus sequence. The humanized antibody optimally also will comprise at least a portion of an immunoglobulin constant region (Fc), typically that of a human immunoglobulin [Jones, *et al*., Nature 321(6069): 522-5 (1986); Riechmann, *et al*., Nature 332(6162): 323-7 (1988); and Presta, Curr. Op. Struct. Biol. 2: 593-6 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones, *et al*., Nature 321(6069): 522-5 (1986); Riechmann, *et al.,* Nature 332(6162): 323-7 (1988); Verhoeyen, *et al*., Science 239(4847): 1534-6 (1988)], by substituting rodent CDRs or CDR sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human anti-LP polypeptide antibodies can also be produced using various techniques known in the art, including phage display libraries [Hoogenboom and Winter, J. Mol. Biol. 227(2): 381-8 (1992); Marks, *et al*., J. Mol. Biol. 222(3): 581-97 (1991)]. The techniques of Cole *et al*. and Boerner, *et al.,* are also available for the preparation of human monoclonal antibodies (Cole, *et al*., Monoclonal Antibodies and Cancer Therapy, Alan R. Liss, p. 77 (1985) and Boerner, *et al*., J. Immunol. 147(1): 86-95 (1991)]. Similarly, human anti-LP polypeptide antibodies can be made by introducing human immunoglobulin loci into transgenic animals, e.g., mice in which the endogenous immunoglobulin genes have been partially or complete inactivated. Upon challenge, human LP polypeptide antibody production is observed, which closely resembles that seen in humans in all respects, including gene rearrangement, assembly and antibody repertoire. This approach is described, for example, in U.S. Patent Nos. 5,545,807; 5,545,806; 5,569,825; 5,625,126; 5,633,425; 5,661,016, and in the following scientific publications: Marks, *et al*., Biotechnology 10(7): 779-83 (1992); Lonberg, *et al.,* Nature 368(6474): 856-9 (1994); Morrison, Nature 368(6474): 812-3 (1994); Fishwild, *et al.,* Nature Biotechnology 14(7): 845-51 (1996); Neuberger, Nature Biotechnology 14(7): 826 (1996); Lonberg and Huszar, Int. Rev. Immunol. 13(1): 65-93 (1995).

Bispecific antibodies are monoclonal, preferably human or humanized, antibodies that have binding specificities for at least two different antigens. In the present case, one of the binding specificities is for an LP polypeptide, the other one is for any other antigen, and preferably for a cell-surface protein or receptor or receptor subunit. Methods for making bispecific antibodies are known in the art. Antibodies with more than two valencies are contemplated. For example, trispecific antibodies can be prepared [Tutt, *et al*., J. Immunol. 147(1): 60-9 (1991)].

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/20373]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

The invention also pertains to immunoconjugates comprising an antibody conjugated to a cytotoxic agent such as a chemotherapeutic agent, toxin (e.g., an enzymatically active toxin of bacterial, fungal, plant or animal origin, or fragments thereof, or a small molecule toxin), or a radioactive isotope (i.e., a radioconjugate).

Conjugates of the antibody and cytotoxic agent are made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds [such as bis-(p-azidobenzoyl) hexanediamine], bis-diazonium derivatives [such as bis-(p-diazoniumbenzoyl)-ethylenediamine], diisocyanates (such as tolylene 2,6-diisocyanate), and bioactive fluorine compounds (such as 1,5-difluoro-2,4-dinitrobenzene). For example, a ricin immunotoxin can be prepared as described in Vitetta, *et al*., Science 238(4830): 1098-104 (1987). Carbon-14-labeled 1-isothiocyanatobenzyl-3-methyldiethylene triaminepentaacetic acid (MX-DTPA) is an exemplary chelating agent for conjugation of radionucleotide to the antibody.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent, and then administration of a "ligand" (e.g., avidin) which is conjugated to a cytotoxic agent (e.g., a radionuclide).

The antibodies disclosed herein may also be formulated as immunoliposomes. Liposomes containing the antibody are prepared by methods known in the art, such as described in Eppstein, *et al*., Proc. Natl. Acad. Sci. USA 82: 3688-92 (1985); Hwang, *et al*., Proc. Natl. Acad. Sci. USA 77(7): 4030-4 (1980); and U.S. Pat. Nos. 4,485,045 and 4,544,545. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin, *et al*., J. Biol. Chem. 257(1): 286-8 (1982) via a disulfide interchange reaction. A chemotherapeutic agent (such as Doxorubicin) is optionally contained within the liposome. See Gabizon, *et al*., J. National Cancer Inst. 81(19): 484-8 ( 1989).

Antibodies specifically binding an LP polypeptide identified herein, as well as other molecules identified by the screening assays disclosed hereinbefore, can be administered for the treatment of various disorders in the form of pharmaceutical compositions.

If an LP polypeptide is intracellular and whole antibodies are used as inhibitors, internalizing antibodies are preferred. However, lipofections or liposomes can also be used to deliver the antibody or an antibody fragment into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, e.g., Marasco, *et al.,* Proc. Natl. Acad. Sci. USA 90(16): 7889-93 (1993).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokines, chemotherapeutic agent, or growth-inhibitory agent. Such molecules are suitably present in combination in amounts that are effective for the purpose intended. The active ingredients may also be entrapped in microcapsules prepared, for example, by coascervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g., films or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and ethyl-L-glutamate, non-degradable ethylene-vinylacetate, degradable lactic acid-glycolic acid copolymers such as the LUPRON DEPOT™ (injectable microspheres composed of lactic acid-glycolic acid copolymer and leuprolide acetate), and poly-D-(-)3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanisms involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

The anti-LP polypeptide antibodies of the present invention have various utilities. For example, such antibodies may be used in diagnostic assays for LP polypeptide expression, e.g., detecting expression in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter, *et al*., Nature 144: 945 (1962); David, *et al*., Biochemistry 13(5): 1014-21 (1974); Pain, *et al.,* J. Immunol. Meth., 40(2): 219-30 (1981); and Nygren, J. Histochem. Cytochem. 30(5): 407-12 (1982).

Anti-LP polypeptide antibodies also are useful for affinity purification from recombinant cell culture or natural sources. In this process, the antibodies against an LP polypeptide are immobilized on a suitable support, such a Sephadex resin or filter paper, using methods well known in the art. The immobilized antibody is then contacted with a sample containing the LP polypeptide to be purified, and thereafter the support is washed with a suitable solvent that will remove substantially all the material in the sample except the LP polypeptide, which is bound to the immobilized antibody. Finally, the support is washed with another suitable solvent that will release the desired polypeptide from the antibody.

This invention encompasses methods of screening compounds to identify those that mimic the activity of the LP polypeptide (agonists) disclosed herein or prevent the effects of the LP polypeptide (antagonists). Screening assays for antagonist drug candidates are designed to identify compounds that bind or complex with an LP polypeptide encoded by the genes identified herein or otherwise interfere with the interaction of LP polypeptides with other cellular proteins. Such screening assays will include assays amenable to high-throughput screening of chemical libraries, making them particularly suitable for identifying small molecule drug candidates.

The assays can be performed in a variety of formats. In binding assays, the interaction is binding, and the complex formed can be isolated or detected in the reaction mixture. In a particular embodiment, an LP polypeptide encoded by a gene identified herein or the drug candidate is immobilized on a solid phase, e.g., on a microtiter plate, by covalent or non-covalent attachments. Non-covalent attachment generally is accomplished by coating the solid surface with a solution comprising LP polypeptide and drying. Alternatively, an immobilized antibody, e.g., a monoclonal antibody specific for the polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to an LP polypeptide, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored through gradients or chromatographic columns. In addition, protein-protein interactions can be monitored by using a yeast-based genetic system described by Fields and co-workers [Fields and Song, Nature 340(6230): 245-6 (1989); Chien, *et al*., Proc. Natl. Acad. Sci. USA 88(21): 9578-82 (1991); Chevray and Nathans, Proc. Natl. Acad. Sci. USA 89(13): 5789-93 (1992)]. Many transcriptional activators, such as yeast GAL4, consist of two physically discrete modular domains, one acting as the DNA-binding domain, while the other functions as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another in which candidate activating proteins are fused to the activation domain. The expression of GAL1-lacZ reporter gene under control of a GAL4-activated promoter depends on reconstitution of GAL4 activity via protein-protein interaction. Colonies containing interacting polypeptides are detected with a chromogenic substrate for beta-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of an LP polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture to serve as a positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

Antagonists may be detected by combining at least one LP polypeptide and a potential antagonist with a membrane-bound or recombinant receptor for that LP polypeptide under appropriate conditions for a competitive inhibition assay. The LP polypeptide can be labeled, such as by radioactivity, such that the number of LP polypeptide molecules bound to the receptor can be used to determine the effectiveness of the potential antagonist. The gene encoding the receptor for an LP polypeptide can be identified by numerous methods known to those of skill in the art, for example, ligand panning and FACS sorting. See Coligan, *et al.,* Current Protocols in Immunology 1(2): Chap. 5 (1991). Preferably, expression cloning is employed such that polyadenylated mRNA is prepared from a cell responsive to the secreted form of a particular LP polypeptide, and a cDNA library created from this mRNA is divided into pools and used to transfect COS cells or other cells that are not responsive to the secreted LP polypeptide. Transfected cells that are grown on glass slides are exposed to the labeled LP polypeptide. The LP polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, a labeled LP polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro-sequencing would be used to design a set of degenerate oligonucleotide probes to screen a cDNA library to identify the gene encoding the putative receptor.

In another assay for antagonists, mammalian cells or a membrane preparation expressing the receptor would be incubated with a labeled LP polypeptide in the presence of the candidate compound. The ability of the compound to enhance or block this interaction could then be removed.

Alternatively, a potential antagonist may be a closely related protein, for example, a mutated form of the LP polypeptide that recognizes the receptor but imparts no effect, thereby competitively inhibiting the action of the polypeptide.

Another potential LP antagonist is an antisense RNA or DNA construct prepared using antisense technology, where, e.g., an antisense RNA or DNA molecule acts to block directly the translation of mRNA by hybridizing to targeted mRNA and prevent its translation into protein. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature form of an LP polypeptide can be used to design an antisense RNA oligonucleotide sequence of about 10 to 40 base pairs in length. A DNA oligonucleotide is designed to be complementary to a region of the gene involved in transcription [triple helix; see Lee, *et al*., Nucl. Acids Res 6(9): 3073-91 (1979); Cooney, *et al.,* Science 241(4864): 456-9 (1988); Beal and Dervan, Science 251(4999): 1360-3 (1991)], thereby preventing transcription and production of the LP polypeptide. The antisense RNA oligonucleotide hybridizes to the mRNA *in vivo* and blocks translation of the mRNA molecules [antisense; see Okano, J. Neurochem. 56(2): 560-7 (1991); Oligodeoxynucleotides as Antisense Inhibitors of Gene Expression, CRC Press: Boca Raton, FL (1988)]. The oligonucleotides described above can also be delivered to cells such that the antisense RNA or DNA may be expressed *in vivo* to inhibit production of the LP polypeptide. When antisense DNA is used, oligodeoxy-ribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the LP polypeptide, thereby blocking the normal biological activity of the LP polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details, see, e.g., Rossi, Current Biology 4(5): 469-71 (1994) and PCT publication No. WO 97/33551.

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra*.

Another use of the compounds of the invention (e.g., LP polypeptides, fragments and variants and LP antibodies directed thereto) described herein is to help diagnose whether a disorder is driven, to some extent, by the modulation of signaling by an LP polypeptide.

A diagnostic assay to determine whether a particular disorder is driven by LP polypeptide dependent signaling can be carried out using the following steps:
a) culturing test cells or tissues expressing an LP polypeptide;
b) administering a compound which can inhibit LP polypeptide dependent signaling; and
c) measuring LP polypeptide mediated phenotypic effects in the test cells.

The steps can be carried out using standard techniques in light of the present disclosure. Appropriate controls take into account the possible cytotoxic effect of a compound, such as treating cells not associated with a cell proliferative disorder (e.g., control cells) with a test compound and can also be used as part of the diagnostic assay. The diagnostic methods of the invention involve the screening for agents that modulate the effects of LP polypeptide associated disorders.

The LP polypeptides or antibodies thereto as well as LP polypeptide antagonists or agonists can be employed as therapeutic agents. Such therapeutic agents are formulated according to known methods to prepare pharmaceutically useful compositions, whereby the LP polypeptide or antagonist or agonist thereof is combined in a mixture with a pharmaceutically acceptable carrier.

In the case of LP polypeptide antagonistic or agonistic antibodies, if the LP polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology [see, e.g., Marasco, *et al*., Proc. Natl. Acad. Sci. USA 90(16): 7889-93 (1993)].

Therapeutic formulations are prepared for storage by mixing the active ingredient having the desired degree of purity with optional pharmaceutically acceptable carriers, excipients or stabilizers [Remington's Pharmaceutical Sciences 16th edition (1980)], in the form of lyophilized formulations or aqueous solutions.

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly(methylmethacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition (1980).

The formulations to be used for *in vivo* administration must be sterile. This is readily accomplished by filtration through sterile filtration membranes.

Therapeutic compositions herein generally are placed into a container having a sterile access port, for example, an intravenous solution bag or vial having a stopper pierceable by a hypodermic injection needle.

Sustained-release preparations may be prepared. Suitable examples of sustained-release preparations include semipermeable matrices of solid hydrophobic polymers containing the therapeutic agent(s), which matrices are in the form of shaped articles, e.g., films, or microcapsules. Examples of sustained-release matrices include polyesters, hydrogels [for example, poly(2-hydroxyethylmethacrylate), or poly(vinylalcohol)], polylactides, copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. Microencapsulation of recombinant proteins for sustained release has been successfully performed with human growth hormone (rhGH), interferon, and interleukin-2. Johnson, *et al*., Nat. Med. 2(7): 795-9 (1996); Yasuda, *et al*., Biomed. Ther. 27: 1221-3 (1993); Hora, *et al*., Bio/Technology 8(8): 755-8 (1990); Cleland, "Design and Production of Single Immunization Vaccines Using Polylactide Polyglycolide Microsphere Systems" in Vaccine Design: The Subunit and Adjuvant Approach, Powell and Newman, Eds., Plenum Press, NY, 1995, pp. 439-62; WO 97/03692; WO 96/40072; WO 96/07399; and U.S. Pat. No. 5,654,010.

The sustained-release formulations of these proteins may be developed using polylactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. See Lewis, "Controlled release of bioactive agents from lactide/glycolide polymer" in Biodegradable Polymers as Drug Delivery Systems [Marcel Dekker; New York (1990), M. Chasin and R. Langer (Eds.) pp. 1-41.]

While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity.

It is contemplated that the compounds, including, but not limited to, antibodies, small organic and inorganic molecules, peptides, antisense molecules, ribozymes, etc., of the present invention may be used to treat various conditions including those characterized by overexpression and/or activation of the disease-associated genes identified herein.

The active agents of the present invention (e.g., antibodies, polypeptides, nucleic acids, ribozymes, small organic or inorganic molecules) are administered to a mammal, preferably a human, in accord with known methods, such as intravenous administration as a bolus or by continuous infusion over a period of time, by intramuscular, intraperitoneal, intracerebral, intracerebrospinal, subcutaneous, intra-articular, intrasynovial, intrathecal, intraoccular, intranasal, intralesional, oral, topical, inhalation, pulmonary, and/or through sustained release.

Other therapeutic regimens may be combined with the administration of LP polypeptide agonists or antagonists,anti-cancer agents, or antibodies of the instant invention.

For the prevention or treatment of disease, the appropriate dosage of an active agent, (e.g., an antibody, polypeptide, nucleic acid, ribozyme, or small organic or inorganic molecule) will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments.

Dosages and desired drug concentration of pharmaceutical compositions of the present invention may vary depending on the particular use envisioned. The determination of the appropriate dosage or route of administration is well within the skill of an ordinary artisan. Animal experiments provide reliable guidance for the determination of effective does for human therapy. Interspecies scaling of effective doses can be performed following the principles laid down by Mordenti and Chappell, "The Use of Interspecies Scaling in Toxicokinetics," in Toxicokinetics and New Drug Development, Yacobi, *et al*., Eds., Pergamon Press, NY (1989), p. 4246.

When *in vivo* administration of a composition comprising an LP polypeptide, LP polypeptide epitope-recognizing antibody, nucleic acid, ribozyme, or small organic and inorganic molecule is employed, normal dosage amounts may vary from about 1 ng/kg up to 100 mg/kg of mammal body weight or more per day, preferably about 1 pg/kg/day up to 100 mg/kg of mammal body weight or more per day, depending upon the route of administration. Guidance as to particular dosages and methods of delivery is provided in the literature; see, for example, U.S. Pat. Nos. 4,657,760; 5,206,344; or 5,225,212. It is within the scope of the invention that different formulations will be effective for different treatment compounds and different disorders, that administration targeting one organ or tissue, for example, may necessitate delivery in a manner different from that to another organ or tissue. Moreover, dosages may be administered by one or more separate administrations or by continuous infusion. For repeated administrations over several days or longer, depending on the condition, the treatment is sustained until a desired suppression of disease symptoms occurs. However, other dosage regimens may be useful. The progress of this therapy is easily monitored by conventional techniques and assays.

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example, the container may be an intravenous solution bag or a vial having a stopper pierceable by a hypodermic injection needle). The active agent in the composition is typically an LP polypeptide, antagonist or agonist thereof. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial end user standpoint, including other buffers, diluents, filters, needles, syringes, and package inserts with instructions for use.

Having generally described the invention, the same will be more readily understood by reference to the following examples, which are provided by way of illustration and are not intended as limiting.

### EXAMPLES

### Example 1

### Expression and Purification of LP Polypeptides in E. coli

The bacterial expression vector pQE60 is used for bacterial expression in this example. (QIAGEN, Inc., Chatsworth, CA). pQE60 encodes ampicillin antibiotic resistance ("Ampr") and contains a bacterial origin of replication ("ori"), an IPTG inducible promoter, a ribosome binding site ("RBS"), six codons encoding histidine residues that allow affinity purification using nickel-nitrilo-tri-acetic acid ("Ni-NTA") affinity resin sold by QIAGEN, Inc., and suitable single restriction enzyme cleavage sites. These elements are arranged such that a DNA fragment encoding a polypeptide can be inserted in such a way as to produce that polypeptide with the six His residues (i.e., a "6 X His tag") covalently linked to the carboxyl terminus of that polypeptide. However, a polypeptide coding sequence can optionally be inserted such that translation of the six His codons is prevented and, therefore, a polypeptide is produced with no 6 X His tag.

The nucleic acid sequence encoding the desired portion of an LP polypeptide lacking the hydrophobic leader sequence is amplified from a cDNA clone using PCR oligonucleotide primers (based on the sequences presented, e.g., as in SEQ ID NO:1, 3, 5, 7, 9, 11, 13, 15, 17 or 19), which anneal to the amino terminal encoding DNA sequences of the desired portion of the LP polypeptide-encoding nucleic acid and to sequences in the construct 3' to the cDNA coding sequence. Additional nucleotides containing restriction sites to facilitate cloning in the pQE60 vector are added to the 5' and 3' sequences, respectively.

For cloning, the 5' and 3' primers have nucleotides corresponding or complementary to a portion of the coding sequence of the LP polypeptide-encoding nucleic acid, e.g., as presented in SEQ ID NO:1, 3, 5, 7, 9, 11, 13, 15, 17 or 19, according to known method steps. One of ordinary skill in the art would appreciate, of course, that the point in a polynucleotide coding sequence where the 5' primer begins can be varied to amplify a desired portion of the complete polypeptide-encoding polynucleotide shorter or longer than the polynucleotide which encodes the mature form of the polypeptide.

The amplified nucleic acid fragments and the vector pQE60 are digested with appropriate restriction enzymes and the digested DNAs are then ligated together. Insertion of the LP polypeptide-encoding DNA into the restricted pQE60 vector places the LP polypeptide coding region, including its associated stop codon, downstream from the IPTG-inducible promoter and in-frame with an initiating AUG codon. The associated stop codon prevents translation of the six histidine codons downstream of the insertion point.

The ligation mixture is transformed into competent *E*. *coli* cells using standard procedures such as those described in Sambrook, *et al.,* 1989; Ausubel, 1987-1998. *E. coli* strain M15/rep4, containing multiple copies of the plasmid pREP4, which expresses the lac repressor and confers kanamycin resistance ("Kanr"), is used in carrying out the illustrative example described herein. This strain, which is only one of many that are suitable for expressing LP polypeptides, is available commercially from QIAGEN, Inc. Transformants are identified by their ability to grow on LB plates in the presence of ampicillin and kanamycin. Plasmid DNA is isolated from resistant colonies and the identity of the cloned DNA confirmed by restriction analysis, PCR and DNA sequencing.

Clones containing the desired constructs are grown overnight ("O/N") in liquid culture in LB media supplemented with both ampicillin (100 µg/mL) and kanamycin (25 µg/mL). The O/N culture is used to inoculate a large culture, at a dilution of approximately 1:25 to 1:250. The cells are grown to an optical density at 600 nm ("OD600") of between 0.4 and 0.6. Isopropyl-b-D-thiogalactopyranoside ("IPTG") is then added to a final concentration of 1 mM to induce transcription from the lac repressor sensitive promoter, by inactivating the lacI repressor. Cells subsequently are incubated further for 3 to 4 hours. Cells then are harvested by centrifugation.

The cells are then stirred for 3-4 hours at 4°C in 6M guanidine-HCl, pH 8. The cell debris is removed by centrifugation, and the supernatant containing the LP polypeptide is dialyzed against 50 mM Na-acetate buffer, pH 6, supplemented with 200 mM NaCl. Alternatively, an LP polypeptide can be successfully refolded by dialyzing it against 500 mM NaCl, 20% glycerol, 25 mM Tris/HCl pH 7.4, containing protease inhibitors.

If insoluble protein is generated, the protein is made soluble according to known method steps. After renaturation, the LP polypeptide is purified by ion exchange, hydrophobic interaction, and/or size exclusion chromatography. Alternatively, an affinity chromatography step such as an antibody column is used to obtain a purified form of the LP polypeptide. The purified polypeptide is stored at 4°C or frozen at -40°C to -120°C.

### Example 2

### Cloning and Expression of LP Polypeptides in a Baculovirus Expression System

In this example, the plasmid shuttle vector pA2 GP is used to insert the cloned DNA encoding the mature LP polypeptide into a baculovirus using a baculovirus leader and standard methods as described in Summers, *et al*., A Manual of Methods for Baculovirus Vectors and Insect Cell Culture Procedures, Texas Agricultural Experimental Station Bulletin No. 1555 (1987). This expression vector contains the strong polyhedrin promoter of the Autographa californica nuclear polyhedrosis virus (AcMNPV) followed by the secretory signal peptide (leader) of the baculovirus gp67 polypeptide and convenient restriction sites such as BamHI, XbaI, and Asp718. The polyadenylation site of the simian virus 40 ("SV40") is used for efficient polyadenylation. For easy selection of recombinant virus, the plasmid contains the beta-galactosidase gene from *E*. *coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate viable virus that expresses the cloned polynucleotide.

Other baculovirus vectors are used in place of the vector above, such as pAc373, pVL941 and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow, *et al*., Virology 170: 31-9 (1989).

The cDNA sequence lacking the AUG initiation codon and the naturally associated nucleotide binding site but encoding a mature LP polypeptide, is amplified using PCR oligonucleotide primers corresponding to the 5' and 3' sequences of the gene. Non-limiting examples include 5' and 3' primers having nucleotides corresponding or complementary to a portion of the coding sequence of an LP polypeptide-encoding polynucleotide, e.g., as presented in SEQ ID NO:1, 3, 5, 7, 9, 11, 13, 15, 17 or 19, according to known method steps.

The amplified fragment is isolated from a 1% agarose gel using a commercially available kit (e.g., "Geneclean," BIO 101 Inc., La Jolla, CA). The fragment then is then digested with the appropriate restriction enzyme and again is purified on a 1% agarose gel. This fragment is designated herein "F1."

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean," BIO 101 Inc., La Jolla, CA). This vector DNA is designated herein "V1."

Fragment F1 and the dephosphorylated plasmid V1 are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria are identified that contain the plasmid bearing a human LP polypeptide-encoding polynucleotide using the PCR method, in which one of the primers that is used to amplify the gene and the second primer is from well within the vector so that only those bacterial colonies containing an LP polypeptide-encoding polynucleotide fragment will show amplification of the DNA. The sequence of the cloned fragment is confirmed by DNA sequencing. The resulting plasmid is designated herein as pBacLP.

Five µg of a pBacLP construct is co-transfected with 1.0 µg of a commercially available linearized baculovirus DNA ("BaculoGold™ baculovirus DNA", Pharmingen, San Diego, CA), using the lipofection method described by Felgner, *et* *al*., Proc. Natl. Acad. Sci. USA 84: 7413-7 (1987). 1 µg of BaculoGold™ virus DNA and 5 µg of the plasmid pBacLP are mixed in a sterile well of a microtiter plate containing 50 µL of serum-free Grace's medium (Life Technologies, Inc., Rockville, MD). Afterwards, 10 µL Lipofectin plus 90 µL Grace's medium are added, mixed and incubated for 15 minutes at room temperature. Then the transfection mixture is added drop-wise to Sf9 insect cells (ATCC CRL 1711) seeded in a 35mm tissue culture plate with 1 mL Grace's medium without serum. The plate is rocked back and forth to mix the newly added solution. The plate is then incubated for 5 hours at 27°C. After 5 hours the transfection solution is removed from the plate and 1 mL of Grace's insect medium supplemented with 10% fetal calf serum is added. The plate is put back into an incubator and cultivation is continued at 27°C for four days.

After four days the supernatant is collected, and a plaque assay is performed. An agarose gel with "Blue Gal" (Life Technologies, Inc., Rockville, MD) is used to allow easy identification and isolation of gal-expressing clones, which produce blue-stained plaques. (A detailed description of a "plaque assay" of this type can also be found in the user's guide for insect cell culture and baculovirology distributed by Life Technologies, Inc., Rockville, MD, pages 9-10). After appropriate incubation, blue stained plaques are picked with a micropipettor tip (e.g., Eppendorf). The agar containing the recombinant viruses is then resuspended in a microcentrifuge tube containing 200 µL of Grace's medium, and the suspension containing the recombinant baculovirus is used to infect Sf9 cells seeded in 35mm dishes. Four days later the supernatants of these culture dishes are harvested, and then they are stored at 4°C.

To verify the expression of the LP polypeptide, Sf9 cells are grown in Grace's medium supplemented with 10% heat-inactivated FBS. The cells are infected with the recombinant baculovirus at a multiplicity of infection ("MOI") of about 2. Six hours later the medium is removed and is replaced with SF900 II medium minus methionine and cysteine (available, e.g., from Life Technologies, Inc., Rockville, MD). If radiolabeled polypeptides are desired, 42 hours later, 5 mCi of ³⁵S-methionine and 5 mCi ³⁵S-cysteine (available from Amersham) are added. The cells are further incubated for 16 hours and then they are harvested by centrifugation. The polypeptides in the supernatant as well as the intracellular polypeptides are analyzed by SDS-PAGE followed by autoradiography (if radiolabeled). Microsequencing of the amino acid sequence of the amino terminus of purified polypeptide can be used to determine the amino terminal sequence of the mature polypeptide and thus the cleavage point and length of the secretory signal peptide.

### Example 3

### Cloning and Expression of LP Polypeptides in Mammalian Cells

A typical mammalian expression vector contains at least one promoter element, which mediates the initiation of transcription of mRNA, the polypeptide coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as pIRES1neo, pRetro-Off, pRetro-On, PLXSN, or pLNCX (Clonetech Labs, Palo Alto, CA), pcDNA3.1 (+/-), pcDNA/Zeo (+/-) or pcDNA3.1/Hygro (+/-) (Invitrogen), PSVL and PMSG (Pharmacia, Uppsala, Sweden), pRSVcat (ATCC 37152), pSV2dhfr (ATCC 37146) and pBC12MI (ATCC 67109). Other suitable mammalian host cells include human Hela 293, H9, Jurkat cells, mouse NIH3T3, C127 cells, Cos 1, Cos 7 and CV 1, quail QC1-3 cells, mouse L cells and Chinese hamster ovary (CHO) cells.

Alternatively, the gene is expressed in stable cell lines that contain the gene integrated into a chromosome. The co-transfection with a selectable marker such as DHRF (dihydrofolate reductase), GPT neomycin, or hygromycin allows the identification and isolation of the transfected cells.

The transfected gene can also be amplified to express large amounts of the encoded polypeptide. The DHFR marker is useful to develop cell lines that carry several hundred or even several thousand copies of the gene of interest. Another useful selection marker is the enzyme glutamine synthase (GS) [Murphy, *et al.,* Biochem. J. 277(Part 1): 277-9 (1991); Bebbington, *et al*., Bio/Technology 10(2): 169-175 (1992)]. Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of polypeptides.

The expression vectors pC1 and pC4 contain the strong promoter (LTR) of the Rous Sarcoma Virus [Cullen, *et al*., Mol. Cell. Biol. 5(3): 438-47 (1985)] plus a fragment of the CMV-enhancer [Boshart, *et al*., Cell 41(2): 521-30 (1985)]. Multiple cloning sites, e.g., with the restriction enzyme cleavage sites BamHI, XbaI and Asp718, facilitate the cloning of the gene of interest. The vectors contain in addition the 3' intron, the polyadenylation and termination signal of the rat preproinsulin gene.

### Example 3(a)

### Cloning and Expression in COS Cells

The expression plasmid, pLP HA, is made by cloning a cDNA encoding LP polypeptide into the expression vector pcDNAI/Amp or pcDNAIII (which can be obtained from Invitrogen, Inc.).

The expression vector pcDNAI/amp contains: (1) an *E. coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker, an SV40 intron; (5) several codons encoding a hemagglutinin fragment (i.e., an "HA" tag to facilitate purification) or HIS tag (see, e.g, Ausubel, supra) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin polypeptide described by Wilson, *et al.,* Cell 37(3): 767-78 (1984). The fusion of the HA tag to the target polypeptide allows easy detection and recovery of the recombinant polypeptide with an antibody that recognizes the HA epitope. pcDNAIII contains, in addition, the selectable neomycin marker.

A DNA fragment encoding the LP polypeptide is cloned into the polylinker region of the vector so that recombinant polypeptide expression is directed by the CMV promoter. The plasmid construction strategy is as follows. The LP polypeptide-encoding cDNA of a clone is amplified using primers that contain convenient restriction sites, much as described above for construction of vectors for expression of LP polypeptides in *E*. *coli*. Non-limiting examples of suitable primers include those based on the coding sequences presented in SEQ ID NO:1, 3, 5, 7, 9, 11, 13, 15, 17 or 19.

The PCR amplified DNA fragment and the vector, pcDNAI/Amp, are digested with suitable restriction enzyme(s) and then ligated. The ligation mixture is transformed into *E*. *coli* strain SURE (available from Stratagene Cloning Systems, 11099 North Torrey Pines Road, La Jolla, CA 92037), and the transformed culture is plated on ampicillin media plates which then are incubated to allow growth of ampicillin resistant colonies. Plasmid DNA is isolated from resistant colonies and examined by restriction analysis or other means for the presence of the LP polypeptide-encoding fragment.

For expression of a recombinant LP polypeptide, COS cells are transfected with an expression vector, as described above, using DEAE-DEXTRAN, as described, for instance, in Sambrook, *et al*., Molecular Cloning: a Laboratory Manual, Cold Spring Laboratory Press, Cold Spring Harbor, New York (1989). Cells are incubated under conditions suitable for expression of the LP polypeptide-encoding polynucleotide by the vector.

Expression of the LP polypeptide-HA fusion polypeptide is detected by radiolabeling and immunoprecipitation, using methods described in, for example Harlow, *et al*., Antibodies: A Laboratory Manual, 2nd Ed., Cold Spring Harbor Laboratory Press, Cold Spring Harbor, New York (1988). To this end, two days after transfection, the cells are labeled by incubation in media containing 35S-cysteine for 8 hours. The cells and the media are collected, and the cells are washed and lysed with detergent-containing RIPA buffer: 150 mM NaCl, 1% NP-40, 0.1% SDS, 0.5% DOC, 50 mM TRIS, pH 7.5, as described by Wilson, *et al*., cited above. Proteins are precipitated from the cell lysate and from the culture media using an HA-specific monoclonal antibody. The precipitated polypeptides are then analyzed by SDS-PAGE and autoradiography. An expression product of the expected size is seen in the cell lysate, which is not seen in negative controls.

### Example 3(b)

### Cloning and Expression in CHO Cells

The vector pC4 is used for the expression of the LP polypeptide. Plasmid pC4 is a derivative of the plasmid pSV2-dhfr (ATCC Accession No. 37146). The plasmid contains the mouse DHFR gene under control of the SV40 early promoter. Chinese hamster ovary cells or other cells lacking dihydrofolate activity that are transfected with these plasmids can be selected by growing the cells in a selective medium (alpha minus MEM, Life Technologies) supplemented with methotrexate. The amplification of the DHFR genes in cells resistant to methotrexate (MTX) has been well documented [see, e.g., Alt, *et al*., J. Biol. Chem. 253(5): 1357-70 (1978); Hamlin and Ma, Biochem. et Biophys. Acta 1087(2): 107-25 (1990); and Page and Sydenham, Biotechnology 9(1): 64-8 (1991)]. Cells grown in increasing concentrations of MTX develop resistance to the drug by overproducing the target enzyme, DHFR, as a result of amplification of the DHFR gene. If a second gene is linked to the DHFR gene, it is usually co-amplified and over-expressed. It is known in the art that this approach can be used to develop cell lines carrying more than 1,000 copies of the amplified gene(s). Subsequently, when the methotrexate is withdrawn, cell lines are obtained which contain the amplified gene integrated into one or more chromosome(s) of the host cell.

Plasmid pC4 contains for expressing the gene of interest the strong promoter of the long terminal repeat (LTR) of the Rous Sarcoma Virus [Cullen, *et al*., Mol. Cell. Biol. 5(3): 438-47 (1985)] plus a fragment isolated from the enhancer of the immediate early gene of human cytomegalovirus (CMV) [Boshart, *et al*., Cell 41(2): 521-30 (1985)]. Downstream of the promoter are BamHI, XbaI, and Asp718 restriction enzyme cleavage sites that allow integration of the genes. Behind these cloning sites the plasmid contains the 3' intron and polyadenylation site of the rat preproinsulin gene. Other high efficiency promoters can also be used for the expression, e.g., the human b-actin promoter, the SV40 early or late promoters or the long terminal repeats from other retroviruses, e.g., HIV and HTLVI. Clontech's Tet-Off and Tet-On gene expression systems and similar systems can be used to express the LP polypeptide in a regulated way in mammalian cells [Gossen and Bujard, Proc. Natl. Acad. Sci. USA 89(12): 5547-51 (1992)]. For the polyadenylation of the mRNA other signals, e.g., from the human growth hormone or globin genes can be used as well. Stable cell lines carrying a gene of interest integrated into the chromosomes can also be selected upon co-transfection with a selectable marker such as gpt, G418 or hygromycin. It is advantageous to use more than one selectable marker in the beginning, e.g., G418 plus methotrexate.

The plasmid pC4 is digested with restriction enzymes and then dephosphorylated using calf intestinal phosphatase by procedures known in the art. The vector is then isolated from a 1% agarose gel.

The DNA sequence encoding the complete LP polypeptide is amplified using PCR oligonucleotide primers corresponding to the 5' and 3' sequences of the gene. Non-limiting examples include 5' and 3' primers having nucleotides corresponding or complementary to a portion of the coding sequences of an LP polypeptide-encoding polynucleotide, e.g., as presented in SEQ ID NO:1, 3, 5, 7, 9, 11, 13, 15, 17 or 19 according to known method steps.

The amplified fragment is digested with suitable endonucleases and then purified again on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC4 using, for instance, restriction enzyme analysis.

Chinese hamster ovary (CHO) cells lacking an active DHFR gene are used for transfection. 5 µg of the expression plasmid pC4 is cotransfected with 0.5 µg of the plasmid pSV2-neo using lipofectin. The plasmid pSV2-neo contains a dominant selectable marker, the neo gene from Tn5 encoding an enzyme that confers resistance to a group of antibiotics including G418. The cells are seeded in alpha minus MEM supplemented with 1 µg/mL G418. After 2 days, the cells are trypsinized and seeded in hybridoma cloning plates (Greiner, Germany) in alpha minus MEM supplemented with 10, 25, or 50 ng/mL of methotrexate plus 1 µg/mL G418. After about 10-14 days single clones are trypsinized and then seeded in 6-well petri dishes or 10 mL flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 mM, 2 mM, 5 mM, 10 mM, 20 mM). The same procedure is repeated until clones are obtained which grow at a concentration of 100-200 mM. Expression of the desired gene product is analyzed, for instance, by SDS-PAGE and Western blot or by reversed-phase HPLC analysis.

### Example 4

### Tissue Distribution of LP Polypeptide-encoding mRNA

Northern blot analysis is carried out to examine expression of LP polypeptide-encoding mRNA in human tissues, using methods described by, among others, Sambrook, *et al*., cited above. A cDNA probe preferably encoding the entire LP polypeptide is labeled with ³²P using the Rediprime™ DNA labeling system (Amersham Life Science), according to the manufacturer's instructions. After labeling, the probe is purified using a CHROMA SPIN-100™ column (Clontech Laboratories, Inc.), according to the manufacturer's protocol number PT1200-1. The purified and labeled probe is used to examine various human tissues for LP polypeptide mRNA.

Multiple Tissue Northern (MTN) blots containing various human tissues (H) or human immune system tissues (IM) are obtained from Clontech and are examined with the labeled probe using ExpressHyb hybridization solution (Clontech) according to manufacturer's protocol number PT1190-1. Following hybridization and washing, the blots are mounted and exposed to film at -70°C overnight, and developed according to standard procedures.

## Claims

1. Isolated nucleic acid comprising DNA having at least 75% sequence identity to a polynucleotide selected from:
a) a polynucleotide having a nucleotide sequence as shown in SEQ ID NO:3;
b) a polynucleotide encoding a polypeptide having the amino acid sequence as shown in SEQ ID NO:4;
c) a polynucleotide encoding the mature form of a polypeptide having the amino acid sequence as shown in SEQ ID NO:4;
d) a polynucleotide fragment of a polynucleotide as in (a), (b), or (c); and
e) a polynucleotide having a nucleotide sequence which is complementary to the nucleotide sequence of a polynucleotide as in (a), (b), (c), or (d).

2. An isolated nucleic acid molecule encoding a polypeptide comprising DNA that hybridizes to the complement of the nucleic acid sequence that encodes an LP polypeptide LP191, any fragment or any variant thereof.

3. The isolated nucleic acid molecule of claim 2, wherein hybridization occurs under stringent hybridization and wash conditions.

4. A vector comprising the nucleic acid molecule of any of claims 1 to 3.

5. The vector of claim 4, wherein said nucleic acid molecule is operably linked to control sequences recognized by a host cell transformed with the vector.

6. A host cell comprising the vector of claim 5.

7. A process for producing an LP polypeptide comprising culturing the host cell of claim 6 under conditions suitable for expression of said LP polypeptide and recovering said LP polypeptide from the cell culture.

8. An isolated polypeptide comprising an amino acid sequence comprising at least about 90% sequence identity to a sequence of amino acid residues selected from LP191, as shown in SEQ ID NO:4.

9. An isolated polypeptide comprising at least 20 contiguous amino acid residues of sequence of:
a) SEQ ID NO:4;
b) immunogenic fragments of (a) at 12 contiguous amino acids in length; or
c) variants of (a) and (b).

10. An isolated polypeptide produced by the method of claim 7.

11. A chimeric molecule comprising an LP polypeptide fused to a heterologous amino acid sequence.

12. The chimeric molecule of claim 11, wherein said heterologous amino acid sequence is an epitope tag sequence.

13. The chimeric molecule of claim 12, wherein said heterologous amino acid sequence is an Fc region of an immunoglobulin.

14. An antibody which specifically binds to an LP polypeptide of:
a) SEQ ID NO:4.

15. The antibody of claim 14, wherein said antibody is a monoclonal antibody.

16. The antibody of claim 15, wherein said antibody is selected from a humanized antibody and a human antibody.

17. A composition comprising a therapeutically effective amount of an active agent selected from: (a) an LP polypeptide; (b) an agonist to an LP polypeptide; (c) an antagonist to an LP polypeptide; (d) an LP polypeptide antibody; (e) an anti-LP polypeptide-encoding mRNA specific ribozyme; and (f) a polynucleotide as in any one of claims 8 to 10; in combination with a pharmaceutically-acceptable carrier.

18. Use of an LP polypeptide or LP polypeptide agonist in the manufacture of a medicament in the treatment of a mammal suffering from a disease, condition, or disorder associated with aberrant levels of an LP polypeptide.

19. A method of diagnosing a disease, condition, or disorder by: (1) culturing test cells or tissues expressing an LP polypeptide; (2) administering a compound which can inhibit LP-modulated signaling; and (3) measuring the LP-mediated phenotypic effects in the test cells or tissues.

20. An article of manufacture comprising a container, label, and therapeutically effective amount of the composition in claim 17.

21. An LP polypeptide according to any one of claims 8 to 10 or an agonist thereto for use in the treatment of a mammal suffering from a disease, condition, or disorder associated with aberrant levels of an LP polypeptide.

22. Use of an LP polypeptide according to any one of claims 8 to 10 or an agonist thereto in the manufacture of a medicament in the treatment of a mammal suffering from a disease, condition, or disorder associated with aberrant levels of an LP polypeptide.
